(19) Europäisches Patentamt / European Patent Office / Office européen des brevets

(11) **EP 4 477 654 A1**

(12) **EUROPEAN PATENT APPLICATION**
published in accordance with Art. 153(4) EPC


(43) Date of publication:
**18.12.2024 Bulletin 2024/51**

(21) Application number: **23752474.9**

(22) Date of filing: **14.02.2023**

(51) International Patent Classification (IPC):
***C07D 487/04*** (2006.01) ***C07D 495/04*** (2006.01)
***C07D 403/12*** (2006.01) ***C07D 417/12*** (2006.01)
***A61K 31/519*** (2006.01) ***A61P 35/02*** (2006.01)
***A61P 35/00*** (2006.01)

(52) Cooperative Patent Classification (CPC):
**A61K 31/519; A61P 35/00; A61P 35/02; C07D 403/12; C07D 417/12; C07D 487/04; C07D 495/04**

(86) International application number:
**PCT/CN2023/076002**

(87) International publication number:
**WO 2023/151697 (17.08.2023 Gazette 2023/33)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority:
**11.02.2022 CN 202210123913**
**25.02.2022 CN 202210175256**
**22.03.2022 CN 202210274432**
**18.04.2022 CN 202210395574**
**12.05.2022 CN 202210512846**
**02.06.2022 CN 202210619691**
**13.07.2022 CN 202210817732**



(71) Applicant: **Xizang Haisco Pharmaceutical Co., Ltd.**
**Lhoka, Tibet 856099 (CN)**

(72) Inventors:
- **ZHANG, Chen**
  **Chengdu, Sichuan 611130 (CN)**
- **LEI, Ming**
  **Chengdu, Sichuan 611130 (CN)**
- **MOU, Tao**
  **Chengdu, Sichuan 611130 (CN)**
- **YU, Yan**
  **Chengdu, Sichuan 611130 (CN)**
- **TANG, Pingming**
  **Chengdu, Sichuan 611130 (CN)**
- **MENG, Yifei**
  **Chengdu, Sichuan 611130 (CN)**
- **WENG, Guanglin**
  **Chengdu, Sichuan 611130 (CN)**
- **LI, Yao**
  **Chengdu, Sichuan 611130 (CN)**
- **YAN, Pangke**
  **Chengdu, Sichuan 611130 (CN)**

(74) Representative: **Sagittarius IP**
**Marlow International**
**Parkway**
**Marlow SL7 1YL (GB)**


(54) **METTL3 INHIBITOR AND COMPOSITION, AND APPLICATION OF SAME IN MEDICINE**


(57) The present invention relates to a compound represented by general formula (I) or a stereoisomer, a tautomer, a deuterated substance, a solvate, a prodrug, a metabolite and a pharmaceutically acceptable salt or eutectic crystal thereof, an intermediate thereof, and a use of the compound in METTL3-related diseases such as cancer.

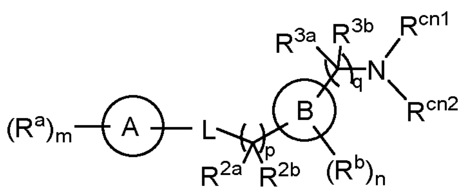

(I)


EP 4 477 654 A1

## Description

### Technical Field

The present invention relates to a compound of general formula (I) or a stereoisomer, a tautomer, a deuterate, a solvate, a prodrug, a metabolite, a pharmaceutically acceptable salt, or co-crystal thereof, an intermediate thereof, and a use thereof in METTL3 enzyme-related diseases.

### Background Art

m6A methylation of mRNA affects almost every aspect of RNA metabolism, including RNA expression, splicing, nuclear export, translation, decay and RNA-protein interactions, and has an important function in the occurrence and development of tumours, affecting the function of different proteins and having different effects in different cancer types.

METTL3 is one of the methyltransferases of M6A, and together with METTL14 and WTAP, among others, form a complex for mRNA methylation modification, affecting protein translation, and promote the occurrence and development of a variety of cancers, including hematopoietic malignancies and solid tumours, by depositing m6A modifications on key transcripts. METTL3 can promote tumour cell growth in AML, breast cancer and HCC. The inhibition of METTL3 is expected to achieve tumour suppression by regulation of mRNA methylation modifications.

AML is characterised by the accumulation of immature myeloid cells in the bone marrow and by the suppression of bone marrow hematopoiesis. CMPD, chronic myeloproliferative disease, often accompanies by an increase in the number of undifferentiated myeloid cells, with hyperproliferative myelogram, and an increase in peripheral blood cells. In the United States, AML's age-adjusted incidence is 3.4/100,000 people, causing about 10,000 deaths per year. AML accounts for approximately 15% to 20% of acute leukemia in children and adolescents and 85% in adults. The incidence of AML increases with age, and most patients develop the disease over 60 years of age, with a median age of 67 years at the time of diagnosis. Developing a METTL3 inhibitor that can be used to treat AML is of a clinical importance.

### Summary of the Invention

An object of the present invention is to provide a compound of general formula (I) or a stereoisomer, tautomer, deuterate, solvate, prodrug, metabolite, pharmaceutically acceptable salt or co-crystal thereof, an intermediate thereof, a cpreparation method therefor, and the use thereof in the preparation of a drug for treating a disease related to the activity or expression quantity of METTL3 enzyme.

The compound of the present invention has good pharmacokinetic performance and bioavailability, oral performance and good safety.

The present invention provides a compound or a stereoisomer, tautomer, deuterate, solvate, prodrug, metabolite, pharmaceutically acceptable salt or co-crystal thereof, wherein the compound is selected from a compound represented by general formula (I),

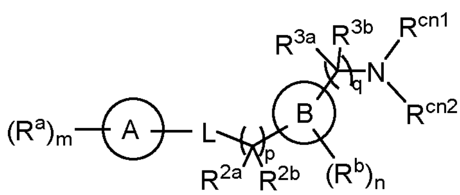

(I)

in some embodiments, the compound of general formula (I) is selected from a compound of general formula (II),

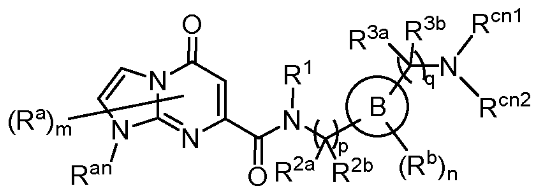

(II);

in some embodiments, the compound of general formula (I) is selected from a compound of general formula (III),

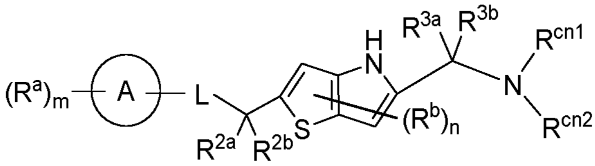
(III);

in some embodiments, the compound of general formula (II) is selected from a compound of general formula (II-1),

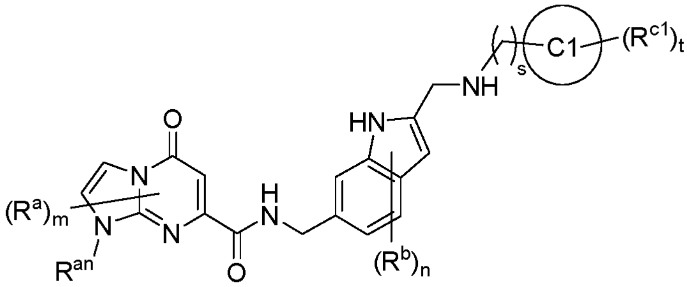
(II-1);

in some embodiments, the compound of general formula (II) is selected from a compound of general formula (II-2),

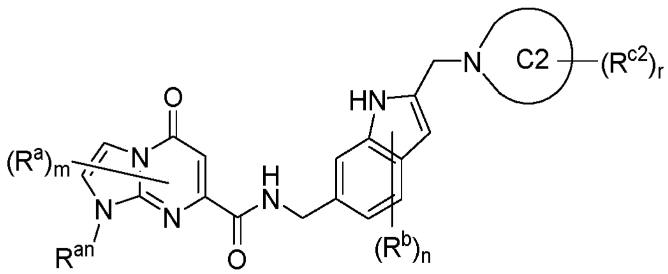
(II-2);

in some embodiments, ring A is selected from

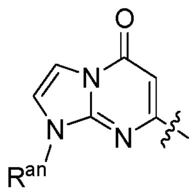
,
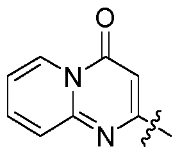
,

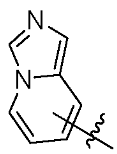
,
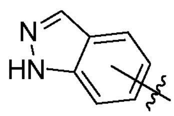
, or
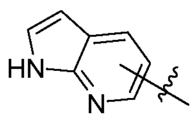
;

in some embodiments, ring A is selected from

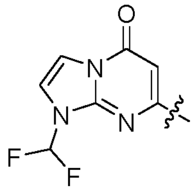
,
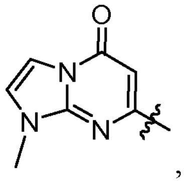
,

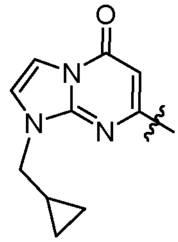, 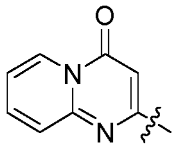, 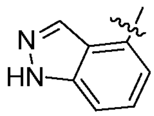, 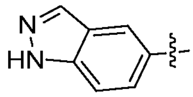, 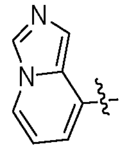, 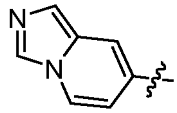

, 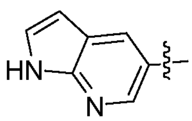, or 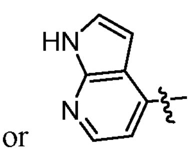;

in some embodiments, $R^{an}$ is selected from H, $C_{1-6}$ alkyl, $C_{3-12}$ carbocyclyl or 3- to 12-membered heterocyclyl, the alkyl, carbocyclyl or heterocyclyl is optionally further substituted with 0 to 4 substituents selected from H, halogen, OH, =O, cyano, $NH_2$, $C_{1-6}$ alkyl, $C_{2-4}$ alkenyl, $C_{2-4}$ alkynyl, -$C_{2-4}$ alkynyl-$C_{3-6}$ cycloalkyl, halogen-substituted-$C_{1-6}$ alkyl, hydroxyl-substituted-$C_{1-6}$ alkyl, cyano-substituted-$C_{1-6}$ alkyl, $C_{1-6}$ alkoxy, $C_{3-12}$ cycloalkyl, phenyl, 3- to 12-membered heterocycloalkyl or 5- to 12-membered heteroaryl, the heterocyclyl, heterocycloalkyl or heteroaryl contains 1 to 3 heteroatoms selected from O, S or N;

in some embodiments, $R^{an}$ is selected from H, $C_{1-4}$ alkyl, $C_{3-6}$ cycloalkyl, or 3-to 6-membered heterocycloalkyl, the alkyl, cycloalkyl or heterocycloalkyl is optionally further substituted with 0 to 4 substituents selected from H, halogen, OH, =O, cyano, $NH_2$, $C_{1-4}$ alkyl, $C_{2-4}$ alkenyl, $C_{2-4}$ alkynyl, -$C_{2-4}$ alkynyl-$C_{3-6}$ cycloalkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy, $C_{3-6}$ cycloalkyl, phenyl, 3- to 6-membered heterocycloalkyl or 5- to 6-membered heteroaryl, and the heterocycloalkyl or heteroaryl contains 1 to 3 heteroatoms selected from O, S or N;

in some embodiments, $R^{an}$ is selected from H, methyl, ethyl, propyl, isopropyl, cyclopropyl, cyclohexyl, cyclopentyl, cyclobutyl, oxetanyl, oxacyclopentyl, oxacyclohexyl, azetidinyl, azacyclopentyl, piperidyl or piperazinyl, the methyl, ethyl, propyl, isopropyl, cyclopropyl, cyclohexyl, cyclopentyl, cyclobutyl, oxetanyl, oxacyclopentyl, oxacyclohexyl, azetidinyl, azacyclopentyl, piperidyl or piperazinyl is optionally further substituted with 0 to 4 substituents selected from H, F, Cl, Br, I, OH, =O, cyano, $NH_2$, $C_{1-4}$ alkyl, $C_{2-4}$ alkenyl, $C_{2-4}$ alkynyl, -$C_{2-4}$ alkynyl-$C_{3-6}$ cycloalkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy, $C_{3-6}$ cycloalkyl, phenyl, 3- to 6-membered heterocycloalkyl or 5- to 6-membered heteroaryl, and the heterocycloalkyl or heteroaryl contains 1 to 3 heteroatoms selected from O, S or N;

in some embodiments, $R^{an}$ is selected from H, methyl, ethyl, propyl, isopropyl, cyclopropyl, cyclobutyl, cyclopentyl, oxetanyl, oxacyclopentyl or oxacyclohexyl, and the methyl, ethyl, propyl, isopropyl, cyclopropyl, oxetanyl, oxacyclopentyl or oxacyclohexyl is optionally further substituted with 0 to 4 substituents selected from H, F, Cl, Br, I, OH, =O, cyano, $NH_2$, $C_{1-4}$ alkyl, halogen-substituted-$C_{1-4}$ alkyl, $C_{2-4}$ alkenyl, $C_{2-4}$ alkynyl, -$C_{2-4}$ alkynyl-$C_{3-6}$ cycloalkyl, hydroxyl-substituted-$C_{1-4}$ alkyl, cyano-substituted-$C_{1-4}$ alkyl, $C_{1-4}$ alkoxy, $C_{3-6}$ cycloalkyl, phenyl, 3- to 6-membered heterocycloalkyl or 5- to 6-membered heteroaryl, the heterocycloalkyl or heteroaryl contains 1 to 3 heteroatoms selected from O, S or N;

in some embodiments, $R^{an}$ is selected from H, methyl, ethyl, propyl, isopropyl, cyclopropyl, oxetanyl, oxacyclopentyl or oxacyclohexyl, and the methyl, ethyl, propyl, isopropyl, cyclopropyl, oxetanyl, oxacyclopentyl or oxacyclohexyl is optionally further substituted with 0 to 4 substituents selected from H, F, Cl, Br, I, OH, =O, cyano, $NH_2$, methyl, ethyl, propyl, isopropyl, ethenyl, 1-propenyl, 2-propenyl, ethynyl, 1-propynyl, 2-propynyl,

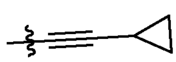,

cyclopropyl, methoxy, ethoxy, or phenyl;

in some embodiments, $R^{an}$ is selected from H, methyl, ethyl, propyl, isopropyl, cyclopropyl,

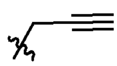, 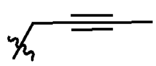, 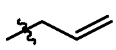, 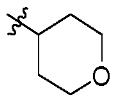, 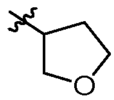, 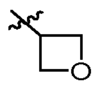, 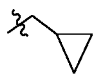

, 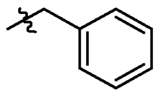, or 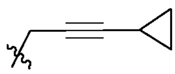;

in some embodiments, $R^{an}$ is selected from H, methyl, ethyl, propyl, isopropyl, cyclopropyl or

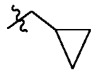;

in some embodiments, ring B is selected from 5- to 10-membered heterocyclyl, wherein the heterocyclyl contains 1 to 5 heteroatoms selected from O, S or N;
in some embodiments, ring B is selected from 5-fused-5-membered bisheteroaryl, 5-fused-6-membered bisheteroaryl or 6-fused-6-membered bisheteroaryl, and the heteroaryl contains 1 to 5 heteroatoms selected from O, S or N;
in some embodiments, ring B is selected from pyrrolopyrrolyl, pyrrolopyrazolyl, pyrroloimidazolyl, pyrazolopyrazolyl, pyrazoloimidazolyl, imidazoimidazolyl, benzopyrrolyl, benzopyrazolyl, benzoimidazolyl, pyridopyrrolyl, pyridopyrazolyl, pyridoimidazolyl, pyrimidopyrrolyl, pyrimidopyrazolyl, pyrimidoimidazolyl, pyrazinopyrrolyl, pyrazinopyrazolyl, pyrazinoimidazolyl, pyridazinopyrrolyl, pyridazinopyrazolyl, pyridazinoimidazolyl, benzopyridyl, benzopyrimidyl, benzopyrazinyl, benzopyridazinyl, pyridopyridyl, pyridopyrimidinyl, pyridopyrazinyl or pyridopyridazinyl;
in some embodiments, ring B is selected from benzopyrrolyl, benzopyrazolyl, benzoimidazolyl, pyridopyrrolyl, pyridopyrazolyl, pyridoimidazolyl, pyrimidopyrrolyl, pyrimidopyrazolyl, pyrimidoimidazolyl, pyrazinopyrrolyl, pyrazinopyrazolyl, pyrazinoimidazolyl, pyridazinopyrrolyl, pyridazinopyrazolyl, or pyridazinoimidazolyl;
in some embodiments, ring B is selected from benzopyrrolyl;
in some embodiments, ring B is selected from thienopyrrolyl;
in some embodiments, L is selected from

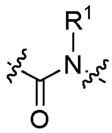

or 5- to 6-membered heteroaryl, L is connected with ring A at the left side, the heteroaryl is optionally further substituted with 0 to 3 substituents selected from H, halogen, OH, cyano, $NH_2$, $C_{1-6}$ alkyl, halogen-substituted-$C_{1-6}$ alkyl, hydroxyl-substituted-$C_{1-6}$ alkyl, cyano-substituted-$C_{1-6}$ alkyl, or $C_{1-6}$ alkoxy, the heteroaryl contains 1 to 3 heteroatoms selected from O, S or N;
in some embodiments, L is selected from

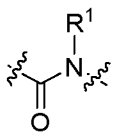

or 5-membered heteroaryl, L is connected with ring A at the left side, the heteroaryl is optionally further substituted with 0 to 2 substituents selected from H, halogen, OH, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, or $C_{1-4}$ alkoxy, the heteroaryl contains 1 to 3 heteroatoms selected from O, S or N;
in some embodiments, L is selected from

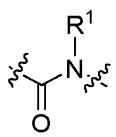,

oxazolyl, thiazolyl, thienyl, furyl, pyrrolyl, pyrazolyl, triazolyl or imidazolyl, L is connected with ring A at the left side, and the oxazolyl, thiazolyl, thienyl, furyl, pyrrolyl, pyrazolyl, triazolyl or imidazolyl is optionally further substituted with 0 to 2 substituents selected from H, F, Cl, Br, I, OH, cyano, $NH_2$, methyl, ethyl, propyl, isopropyl, methoxy, or ethoxy;

in some embodiments, L is selected from

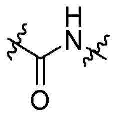, 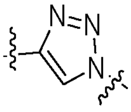, 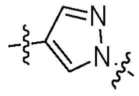,

or

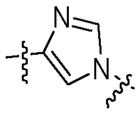,

and is connected with ring A at the left side;

in some embodiments, $R^1$ is selected from H, $C_{1-6}$ alkyl or $C_{3-6}$ cycloalkyl, the alkyl or cycloalkyl is optionally further substituted with 0 to 4 substituents selected from H, halogen, OH, =O, cyano, $NH_2$, $C_{1-6}$ alkyl, halogen-substituted-$C_{1-6}$ alkyl, hydroxyl-substituted-$C_{1-6}$ alkyl, cyano-substituted-$C_{1-6}$ alkyl, $C_{1-6}$ alkoxy or $C_{3-6}$ cycloalkyl;

in some embodiments, $R^1$ is selected from H, $C_{1-4}$ alkyl or $C_{3-6}$ cycloalkyl, the alkyl or cycloalkyl is optionally further substituted with 0 to 4 substituents selected from H, halogen, OH, =O, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy or $C_{3-6}$ cycloalkyl;

in some embodiments, $R^1$ is selected from H, methyl, ethyl, propyl, isopropyl or cyclopropyl, and the methyl, ethyl, propyl, isopropyl or cyclopropyl is optionally further substituted with 0 to 4 substituents selected from H, F, Cl, Br, I, OH, =O, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy or $C_{3-6}$ cycloalkyl;

in some embodiments, $R^1$ is selected from H, methyl, ethyl, propyl, isopropyl or cyclopropyl, and the methyl, ethyl, propyl, isopropyl or cyclopropyl is optionally further substituted with 0 to 4 substituents selected from H, F, Cl, Br, I, OH, =O, cyano, $NH_2$, methyl, ethyl, propyl, isopropyl, methoxy, ethoxy, or cyclopropyl;

in some embodiments, $R^1$ is selected from H, methyl, ethyl, propyl or isopropyl;

in some embodiments, $R^1$ is selected from H;

in some embodiments, $R^a$ or $R^b$ is each independently selected from H, halogen, cyano, OH, =O, $C_{1-6}$ alkyl, $C_{1-6}$ alkoxy, or $C_{3-6}$ cycloalkyl, the alkyl, alkoxy or cycloalkyl is optionally further substituted with 0 to 4 substituents selected from H, halogen, OH, =O, cyano, $NH_2$, $C_{1-6}$ alkyl, halogen-substituted-$C_{1-6}$ alkyl, hydroxyl-substituted-$C_{1-6}$ alkyl, cyano-substituted-$C_{1-6}$ alkyl, $C_{1-6}$ alkoxy or $C_{3-6}$ cycloalkyl;

in some embodiments, $R^a$ or $R^b$ is each independently selected from H, halogen, cyano, OH, =O, $C_{1-4}$ alkyl, $C_{1-4}$ alkoxy, or $C_{3-6}$ cycloalkyl, and the alkyl, alkoxy or cycloalkyl is optionally further substituted with 0 to 4 substituents selected from H, halogen, OH, =O, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy or $C_{3-6}$ cycloalkyl;

in some embodiments, $R^a$ or $R^b$ is each independently selected from H, F, Cl, Br, I, cyano, OH, =O, methyl, ethyl, propyl, isopropyl, cyclopropyl, cyclohexyl, cyclopentyl, cyclobutyl, azetidinyl, azacyclopentyl, azacyclohexyl, piperidyl, methoxy or ethoxy, and the methyl, ethyl, propyl, isopropyl, cyclopropyl, cyclohexyl, cyclopentyl, cyclobutyl, azetidinyl, azacyclopentyl, azacyclohexyl, piperidyl, methoxy or ethoxy is optionally further substituted with 0 to 4 substituents selected from H, F, Cl, Br, I, OH, =O, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy or $C_{3-6}$ cycloalkyl;

in some embodiments, $R^a$ or $R^b$ is each independently selected from H, F, Cl, Br, I, cyano, OH, =O, methyl, ethyl, propyl, isopropyl, or cyclopropyl, and the methyl, ethyl, propyl, isopropyl or cyclopropyl is optionally further substituted with 0 to 4 substituents selected from H, F, Cl, Br, I, OH, =O, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy or $C_{3-6}$ cycloalkyl;

in some embodiments, $R^a$ or $R^b$ is each independently selected from H, F, Cl, Br, I, cyano, OH, =O, $CF_3$, methyl, ethyl, propyl, isopropyl, or cyclopropyl;

in some embodiments, $R^a$ or $R^b$ is each independently selected from H, F, Cl, Br, I, cyano, OH, =O, methyl, ethyl, propyl, isopropyl, or cyclopropyl;

in some embodiments, $R^{an}$ and $R^a$ together with the atom to which they are attached form a 5- to 6-membered heterocycle, the heterocycle is optionally further substituted with 0 to 4 substituents selected from H, halogen, OH, =O, cyano, $NH_2$, $C_{1-6}$ alkyl, halogen-substituted-$C_{1-6}$ alkyl, hydroxyl-substituted-$C_{1-6}$ alkyl, cyano-substituted-$C_{1-6}$ alkyl, $C_{1-6}$ alkoxy or $C_{3-6}$ cycloalkyl, the heterocycle contains 1 to 3 heteroatoms selected from O, S or N;

in some embodiments, $R^{an}$ and $R^a$ together with the atom to which they are attached form a 5- to 6-membered heteroaromatic ring, the heteroaromatic ring is optionally further substituted with 0 to 4 substituents selected from H,

halogen, OH, =O, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy or $C_{3-6}$ cycloalkyl, and the heteroaryl contains 1 to 3 heteroatoms selected from O, S or N;

in some embodiments, $R^{an}$ and $R^a$ together with the atom to which they are attached form a pyridine ring, pyrimidine ring, pyridazine ring or pyrazine ring, and the pyridine ring, pyrimidine ring or pyrazine ring is optionally further substituted with 0 to 4 substituents selected from H, F, Cl, Br, I, OH, =O, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy or $C_{3-6}$ cycloalkyl;

in some embodiments, $R^{an}$ and $R^a$ together with the atom to which they are attached form a pyridine ring, and the pyridine ring is optionally further substituted with 0 to 4 substituents selected from H, F, Cl, Br, I, OH, =O, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy or $C_{3-6}$ cycloalkyl;

in some embodiments, $R^{an}$ and $R^a$ together with the atom to which they are attached form a pyridine ring;

in some embodiments,

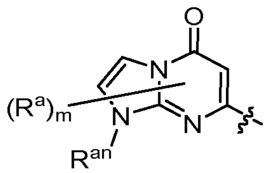

is selected from

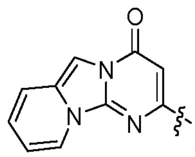

;

in some embodiments, $R^{2a}$, $R^{2b}$, $R^{3a}$ and $R^{3b}$ are each independently selected from H, halogen, cyano, OH, =O, $C_{1-6}$ alkyl, $C_{1-6}$ alkoxy or $C_{3-6}$ cycloalkyl, the alkyl, alkoxy or cycloalkyl is optionally further substituted with 0 to 4 substituents selected from H, halogen, OH, =O, cyano, $NH_2$, $C_{1-6}$ alkyl, halogen-substituted-$C_{1-6}$ alkyl, hydroxyl-substituted-$C_{1-6}$ alkyl, cyano-substituted-$C_{1-6}$ alkyl, $C_{1-6}$ alkoxy or $C_{3-6}$ cycloalkyl;

in some embodiments, $R^{2a}$, $R^{2b}$, $R^{3a}$ and $R^{3b}$ are each independently selected from H, halogen, cyano, OH, =O, $C_{1-4}$ alkyl, $C_{1-4}$ alkoxy or $C_{3-6}$ cycloalkyl, and the alkyl, alkoxy or cycloalkyl is optionally further substituted with 0 to 4 substituents selected from H, halogen, OH, =O, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy or $C_{3-6}$ cycloalkyl;

in some embodiments, $R^{2a}$, $R^{2b}$, $R^{3a}$ and $R^{3b}$ are each independently selected from H, F, Cl, Br, I, cyano, OH, =O, methyl, ethyl, propyl, isopropyl, methoxy, ethoxy or cyclopropyl, and the methyl, ethyl, propyl, isopropyl, methoxy, ethoxy or cyclopropyl is optionally further substituted with 0 to 4 substituents selected from H, F, Cl, Br, I, OH, =O, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy or $C_{3-6}$ cycloalkyl;

in some embodiments, $R^{2a}$, $R^{2b}$, $R^{3a}$ and $R^{3b}$ are each independently selected from H, F, Cl, Br, I, cyano, OH, =O, methyl, ethyl, propyl, isopropyl, methoxy, ethoxy or cyclopropyl, and the methyl, ethyl, propyl, isopropyl, methoxy, ethoxy or cyclopropyl is optionally further substituted with 0 to 4 substituents selected from H, F, Cl, Br, I, OH, =O, cyano, $NH_2$, methyl, ethyl, propyl, isopropyl, methoxy, ethoxy or cyclopropyl;

in some embodiments, $R^{2a}$, $R^{2b}$, $R^{3a}$ and $R^{3b}$ are each independently selected from H, F, Cl, Br, I, cyano, OH, =O, methyl, ethyl, propyl, isopropyl, methoxy, ethoxy or cyclopropyl;

in some embodiments, $R^{2a}$, $R^{2b}$, $R^{3a}$ and $R^{3b}$ are each independently selected from H;

in some embodiments, $R^{2a}$ and $R^{2b}$, $R^{3a}$ and $R^{3b}$ together with the atom to which they are attached form $C_{3-6}$ carbocyclyl or 3- to 6-membered heterocyclyl, respectively, the carbocyclyl or heterocyclyl is optionally further substituted with 0 to 4 substituents selected from H, halogen, OH, =O, cyano, $NH_2$, $C_{1-6}$ alkyl, halogen-substituted-$C_{1-6}$ alkyl, hydroxyl-substituted-$C_{1-6}$ alkyl, cyano-substituted-$C_{1-6}$ alkyl, $C_{1-6}$ alkoxy or $C_{3-6}$ cycloalkyl, the heterocyclyl contains 1 to 3 heteroatoms selected from O, S or N;

in some embodiments, $R^{2a}$ and $R^{2b}$, $R^{3a}$ and $R^{3b}$ are each together with the atom to which they are attached form cyclopropyl or cyclohexyl, respectively;

in some embodiments, $R^{cn1}$ and $R^{cn2}$ are each independently selected from H, halogen, cyano, OH, =O, $C_{1-6}$ alkyl, $C_{1-6}$ alkoxy, $C_{3-10}$ carbocyclyl, 3- to 10-membered heterocyclyl or

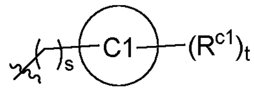

,

the alkyl, alkoxy, carbocyclyl or heterocyclyl is optionally further substituted with 0 to 4 substituents selected from H, halogen, OH, =O, cyano, $NH_2$, $C_{1-6}$ alkyl, halogen-substituted-$C_{1-6}$ alkyl, hydroxyl-substituted-$C_{1-6}$ alkyl, cyano-substituted-$C_{1-6}$ alkyl, $C_{1-6}$ alkoxy or $C_{3-6}$ cycloalkyl, the heterocyclyl contains 1 to 3 heteroatoms selected from O, S or N;

in some embodiments, $R^{cn1}$ and $R^{cn2}$ are each independently selected from H, halogen, cyano, OH, =O, $C_{1-4}$ alkyl, $C_{1-4}$ alkoxy, $C_{3-10}$ cycloalkyl, 3- to 10-membered heterocycloalkyl or

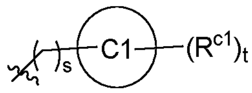

,

the alkyl, alkoxy, cycloalkyl or heterocycloalkyl is optionally further substituted with 0 to 4 substituents selected from H, halogen, OH, =O, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy or $C_{3-6}$ cycloalkyl, and the heterocycloalkyl contains 1 to 3 heteroatoms selected from O, S or N;

in some embodiments, $R^{cn1}$ and $R^{cn2}$ are each independently selected from H, F, Cl, Br, I, cyano, OH, =O, methyl, ethyl, propyl, isopropyl, cyclopropyl, cyclohexyl, cyclopentyl, cyclobutyl, azetidinyl, azacyclopentyl, azacyclohexyl or

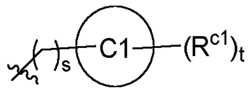

,

and the methyl, ethyl, propyl, isopropyl, cyclopropyl, cyclohexyl, cyclopentyl, cyclobutyl, azetidinyl, azacyclopentyl, or azacyclohexyl is optionally further substituted with 0 to 4 substituents selected from H, F, Cl, Br, I, OH, =O, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy or $C_{3-6}$ cycloalkyl;

in some embodiments, $R^{cn1}$ and $R^{cn2}$ are each independently selected from H, F, Cl, Br, I, cyano, OH, =O, methyl, ethyl, propyl, isopropyl, cyclopropyl, cyclohexyl, cyclopentyl, cyclobutyl or

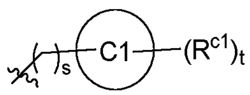

,

and the methyl, ethyl, propyl, isopropyl, cyclopropyl, cyclohexyl, cyclopentyl, or cyclobutyl is optionally further substituted with 0 to 4 substituents selected from H, F, Cl, Br, I, OH, =O, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy or $C_{3-6}$ cycloalkyl;

in some embodiments, $R^{cn1}$ and $R^{cn2}$ are each independently selected from H, methyl, ethyl, propyl, isopropyl, cyclopropyl, cyclohexyl, cyclopentyl, cyclobutyl or

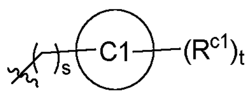

,

and the methyl, ethyl, propyl, isopropyl, cyclopropyl, cyclohexyl, cyclopentyl, or cyclobutyl is optionally further substituted with 0 to 4 substituents selected from H, F, Cl, Br, I, OH, =O, cyano, $NH_2$, $CF_3$, methyl, ethyl, propyl, isopropyl, cyclopropyl, methoxy or ethoxy;

in some embodiments, ring C1 is selected from $C_{3-12}$ carbocyclyl or 3- to 12-membered heterocyclyl, and the heterocyclyl contains 1 to 3 heteroatoms selected from O, S or N;

in some embodiments, ring C1 is selected from $C_{3-7}$monocycloalkyl, $C_{4-11}$fused cycloalkyl, $C_{5-11}$ spirocycloalkyl, $C_{5-12}$biidged cycloalkyl, 4- to 7-membered monoheterocycloalkyl, 4- to 11-membered fused heterocycloalkyl, 5- to 11-membered spiroheterocycloalkyl or 5- to 12-membered bridged heterocycloalkyl, and the heterocycloalkyl contains 1 to 3 heteroatoms selected from O, S or N;

in some embodiments, ring C1 is selected from cyclopropyl, cyclobutyl, cyclopentyl, cyclohexyl, oxetanyl, oxacyclopentyl, oxacyclohexyl, azetidinyl, azacyclopentyl, piperidine, morpholine, piperazine, 1,4-diazepanyl, cyclopropyl fused cyclopentyl, cyclopentyl fused cyclobutyl, cyclopentyl fused cyclopentyl, cyclopentyl fused cyclohexyl, cyclopropyl spirocyclopentyl, cyclobutyl spirocyclobutyl, cyclobutyl spirocyclopentyl, cyclopentyl spirocyclopentyl, cyclopentyl spirocyclohexyl, cyclohexyl spirocyclohexyl, cyclopropyl fused azetidinyl, cyclopropyl fused azacyclopentyl, cyclopropyl fused azacyclohexyl, cyclobutyl fused azetidinyl, cyclobutyl fused azacyclopentyl, cyclobutyl fused azacyclohexyl, cyclopentyl fused azetidinyl, cyclopentyl fused azacyclopentyl, cyclopentyl fused azacyclohexyl, cyclohexyl fused azetidinyl, cyclohexyl fused azacyclopentyl, cyclohexyl fused azacyclohexyl, azetidinyl fused azetidinyl, azetidinyl fused azacyclopentyl, azetidinyl fused azacyclohexyl, azacyclopentyl fused azetidinyl, azacyclopentyl fused azacyclopentyl, azacyclopentyl fused azacyclohexyl, azacyclohexyl fused azetidinyl, azacyclohexyl fused azacyclopentyl, azacyclohexyl fused azacyclohexyl, cyclobutyl spiroazetidinyl, cyclobutyl spiroazacyclopentyl, cyclobutyl spiroazacyclohexyl, cyclopentyl spiroazetidinyl, cyclopentyl spiroazacyclopentyl, cyclopentyl spiroazacyclohexyl, cyclohexyl spiroazetidinyl, cyclohexyl spiroazacyclopentyl, cyclohexyl spiroazacyclohexyl, azetidinyl spiroazetidinyl, azetidinyl spiroazacyclopentyl, azetidinyl spiroazacyclohexyl, azacyclopentyl spiroazetidinyl, azacyclopentyl spiroazacyclopentyl, azacyclopentyl spiroazacyclohexyl, azacyclohexyl spiroazetidinyl, azacyclohexyl spiroazacyclopentyl, azacyclohexyl spiroazacyclohexyl, oxetanyl, oxacyclopentyl, oxacyclohexyl, cyclopropyl fusedoxetanyl, cyclopropyl fusedoxacyclopentyl, cyclopropyl fusedoxacyclohexyl, cyclobutyl fusedoxetanyl, cyclobutyl fusedoxacyclopentyl, cyclobutyl fusedoxacyclohexyl, cyclopentyl fusedoxetanyl, cyclopentyl fusedoxacyclopentyl, cyclopentyl fusedoxacyclohexyl, cyclohexyl fusedoxetanyl, cyclohexyl fusedoxacyclopentyl, cyclohexyl fusedoxacyclohexyl, azetidinyl fusedoxetanyl, azetidinyl fusedoxacyclopentyl, azetidinyl fusedoxacyclohexyl, azacyclopentyl fusedoxetanyl, azacyclopentyl fusedoxacyclopentyl, azacyclopentyl fusedoxacyclohexyl, azacyclohexyl fusedoxetanyl, azacyclohexyl fusedoxacyclopentyl, azacyclohexyl fusedoxacyclohexyl, cyclobutyl spirooxetanyl, cyclobutyl spirooxacyclopentyl, cyclobutyl spirooxacyclohexyl, cyclopentyl spirooxetanyl, cyclopentyl spirooxacyclopentyl, cyclopentyl spirooxacyclohexyl, cyclohexyl spirooxetanyl, cyclohexyl spirooxacyclopentyl, cyclohexyl spirooxacyclohexyl, azetidinyl spirooxetanyl, azetidinyl spirooxacyclopentyl, azetidinyl spirooxacyclohexyl, azacyclopentyl spirooxetanyl, azacyclopentyl spirooxacyclopentyl, azacyclopentyl spirooxacyclohexyl, azacyclohexyl spirooxetanyl, azacyclohexyl spirooxacyclopentyl, azacyclohexyl spirooxacyclohexyl, bicyclo[2.1.1]hexyl, bicyclo[2.2.1]heptyl, bicyclo[3.3.2]decanyl, bicyclo[2.2.2]octanyl, bicyclo[3.2.1]octanyl, bicyclo[3.3.3]undecyl, adamantyl,

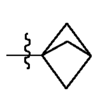, 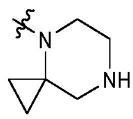, 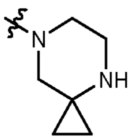, 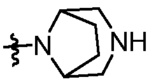,

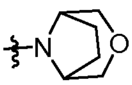, 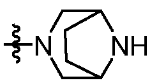, 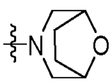, 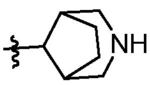, 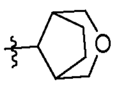, 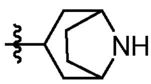, 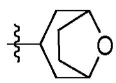,

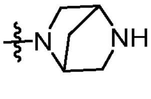, 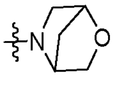, 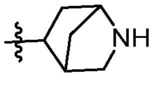, 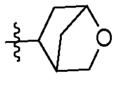, 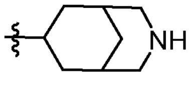, 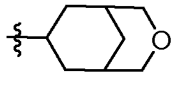,

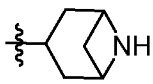, 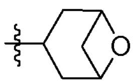, 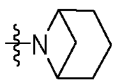, 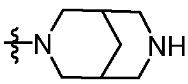, 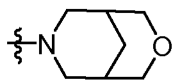, or 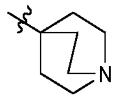;

in some embodiments, ring C1 is selected from cyclopropyl, cyclobutyl, cyclopentyl, cyclohexyl, oxetanyl, oxacyclopentyl, oxacyclohexyl, azetidinyl, azacyclopentyl, piperidine, morpholine, piperazine, 1,4-diazepanyl, cyclopropyl fused cyclopentyl, cyclopentyl fused cyclobutyl, cyclopentyl fused cyclopentyl, cyclopentyl fused cyclohexyl,

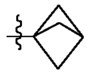,

bicyclo[2.1.1]hexyl, bicyclo[2.2.1]heptyl, bicyclo[3.3.2]decanyl, bicyclo[2.2.2]octanyl, bicyclo[3.2.1]octanyl, bicyclo [3.3.3]undecyl, or adamantyl;

in some embodiments, ring C1 is selected from cyclopropyl, cyclobutyl, cyclopentyl, cyclohexyl, cyclopentyl fused cyclobutyl or

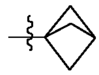
;

in some embodiments, each $R^{c1}$ is independently selected from H, halogen, cyano, OH, =O, $C_{1-6}$ alkyl, $C_{1-6}$ alkoxy, $C_{3-12}$ carbocyclyl or 3- to 12-membered heterocyclyl, and the alkyl, alkoxy, carbocyclyl or heterocyclyl is optionally further substituted with 0 to 4 substituents selected from H, halogen, OH, =O, cyano, $NH_2$, $C_{1-6}$ alkyl, halogen-substituted-$C_{1-6}$ alkyl, hydroxyl-substituted-$C_{1-6}$ alkyl, cyano-substituted-$C_{1-6}$ alkyl, $C_{1-6}$ alkoxy or $C_{3-6}$ cycloalky, the heterocyclyl contains 1 to 3 heteroatoms selected from O, S or N;

in some embodiments, each $R^{c1}$ is independently selected from H, halogen, cyano, OH, =O, $C_{1-4}$ alkyl, $C_{1-4}$ alkoxy, $C_{3-6}$ cycloalkyl or 3- to 6-membered heterocycloalkyl, the alkyl, alkoxy, cycloalkyl or heterocycloalkyl is optionally further substituted with 0 to 4 substituents selected from H, halogen, OH, =O, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy or $C_{3-6}$ cycloalkyl, and the heterocycloalkyl contains 1 to 3 heteroatoms selected from O, S or N;

in some embodiments, each $R^{c1}$ is independently selected from H, F, Cl, Br, I, cyano, OH, =O, methyl, ethyl, propyl, isopropyl, cyclopropyl, cyclohexyl, cyclopentyl, cyclobutyl, azetidinyl, azacyclopentyl, piperidyl, piperazinyl, methoxy or ethoxy, and the methyl, ethyl, propyl, isopropyl, cyclopropyl, cyclohexyl, cyclopentyl, cyclobutyl, azetidinyl, azacyclopentyl, piperidyl, piperazinyl, methoxy or ethoxy is optionally further substituted with 0 to 4 substituents selected from H, F, Cl, Br, I, OH, =O, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy or $C_{3-6}$ cycloalkyl;

in some embodiments, each $R^{c1}$ is independently selected from H, F, Cl, Br, I, cyano, OH, =O, methyl, ethyl, propyl, isopropyl, cyclopropyl, methoxy or ethoxy, and the methyl, ethyl, propyl, isopropyl, cyclopropyl, methoxy or ethoxy is optionally further substituted with 0 to 4 substituents selected from H, F, Cl, Br, I, OH, =O, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy or $C_{3-6}$ cycloalkyl;

in some embodiments, each $R^{c1}$ is independently selected from H, F, Cl, Br, I, cyano, OH, =O, methyl, ethyl, propyl, isopropyl, or cyclopropyl;

in some embodiments, each $R^{c1}$ is independently selected from H, F, or Cl;

in some embodiments, $R^{cn1}$ and $R^{cn2}$ together with the atom to which they are attached form 3- to 12-membered heterocyclyl, and the heterocyclyl is optionally further substituted with 0 to 4 substituents selected from H, halogen, OH, =O, cyano, $NH_2$, $C_{1-6}$ alkyl, halogen-substituted-$C_{1-6}$ alkyl, hydroxyl-substituted-$C_{1-6}$ alkyl, cyano-substituted-$C_{1-6}$ alkyl, $C_{1-6}$ alkoxy or $C_{3-6}$ cycloalkyl, the heterocyclyl contains 1 to 3 heteroatoms selected from O, S or N;

in some embodiments, $R^{cn1}$ and $R^{cn2}$ together with the atom to which they are attached form a 4- to 7-membered monoheterocycloalkyl, 4- to 11-membered fused heterocycloalkyl, 5- to 11-membered spiroheterocycloalkyl or 5- to 12-membered bridged heterocycloalkyl, the heterocycloalkyl is optionally further substituted with 0 to 4 substituents selected from H, halogen, OH, =O, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy or $C_{3-6}$ cycloalkyl, and the heterocycloalkyl contains 1 to 3 heteroatoms selected from O, S or N;

in some embodiments, $R^{cn1}$ and $R^{cn2}$ together with the atom to which they are attached form substituted or unsubstituted azetidinyl, azacyclopentyl, piperidine, piperazine, azetidinyl fused cyclopropyl, azetidinyl fused cyclobutyl, azetidinyl fused cyclopentyl, azetidinyl fused cyclohexyl, azacyclopentyl fused cyclopropyl, azacyclopentyl fused cyclobutyl, azacyclopentyl fused cyclopentyl, azacyclopentyl fused cyclohexyl, azacyclohexyl fused cyclopropyl, azacyclohexyl fused cyclobutyl, azacyclohexyl fused cyclopentyl, azacyclohexyl fused cyclohexyl, azetidinyl fused azetidinyl, azetidinyl fused azacyclopentyl, azetidinyl fused azacyclohexyl, azacyclopentyl fused azetidinyl, azacyclopentyl fuse dazacyclopentyl, azacyclopentyl fused azacyclohexyl, azacyclohexyl fused azetidinyl, azacyclohexyl fused azacyclopentyl, azacyclohexyl fused azacyclohexyl, azetidinyl spiroazetidinyl, azetidinyl spiroazacyclopentyl, azetidinyl spiroazacyclohexyl, azacyclopentyl spiroazetidinyl, azacyclopentyl spiroazacyclopentyl, azacyclopentyl spiroazacyclohexyl, azacyclohexyl spiroazetidinyl, azacyclohexyl spiroazacyclopentyl, azacyclohexyl spiroazacyclohexyl, azacyclopentyl spirocyclobutyl, azacyclopentyl spirocyclopentyl, azacyclohexyl spirocyclobutyl, azacyclohexyl spirocyclopentyl, azacyclohexyl spirocyclohexyl,

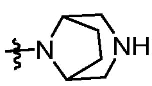
,

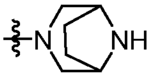, 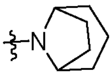, 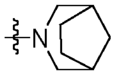, 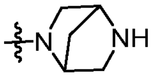, 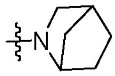, 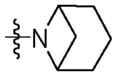, 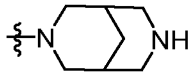,

or

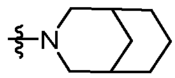,

which, when substituted, is optionally further substituted with 0 to 4 substituents selected from H, halogen, OH, =O, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy or $C_{3-6}$ cycloalkyl;

in some embodiments, $R^{cn1}$ and $R^{cn2}$ together with the atom to which they are attached form substituted or unsubstituted azetidinyl, azacyclopentyl, piperidine, piperazine, azacyclopentyl, spirocyclobutyl, azacyclopentyl, spirocyclopentyl, azacyclohexyl, spirocyclobutyl, azacyclohexyl, spirocyclopentyl, azacyclohexyl, or spirocyclohexyl, which, when substituted, is optionally further substituted with 0 to 4 substituents selected from H, F, Cl, Br, I, OH, =O, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy or $C_{3-6}$ cycloalkyl;

in some embodiments, $R^{cn1}$ and $R^{cn2}$ together with the atom to which they are attached form substituted or unsubstituted azetidinyl, azacyclopentyl, piperidine, piperazine, azacyclopentyl, spirocyclobutyl, azacyclopentyl, spirocyclopentyl, azacyclohexyl, spirocyclobutyl, azacyclohexyl, spirocyclopentyl, azacyclohexyl, or spirocyclohexyl, which, when substituted, is optionally further substituted with 0 to 4 substituents selected from H, F, Cl, Br, I, OH, =O, cyano, $NH_2$, $CF_3$, methyl, ethyl, propyl, isopropyl, cyclopropyl, methoxy or ethoxy;

in some embodiments, $R^{cn1}$ and $R^{cn2}$ together with the atom to which they are attached form

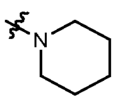, 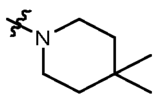, 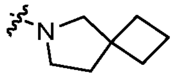, 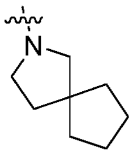, 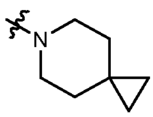,

or

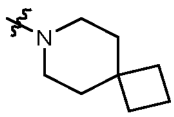;

in some embodiments, $R^{cn1}$ and $R^{cn2}$ together with the atom to which they are attached form

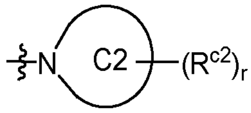;

in some embodiments, ring C2 is selected from azetidinyl, azacyclopentyl, piperidine or piperazine;

in some embodiments, each $R^{c2}$ is independently selected from H, F, Cl, Br, I, OH, =O, cyano, $NH_2$, methyl, ethyl, propyl, isopropyl, cyclopropyl, methoxy, or ethoxy;

in some embodiments, m is selected from 0, 1, 2, or 3;

in some embodiments, m is selected from 0, 1 or 2;

in some embodiments, m is selected from 0 or 1;

in some embodiments, m is selected from 0, 1, 2, 3, 4, 5, 6 or 7;

in some embodiments, n is selected from 0, 1, 2, 3 or 4;

in some embodiments, n is selected from 0, 1, 2 or 3;

in some embodiments, n is selected from 0 or 1;

in some embodiments, p is selected from 0, 1, 2 or 3;

in some embodiments, p is selected from 0, 1 or 2;
in some embodiments, q is selected from 0, 1, 2 or 3;
in some embodiments, q is selected from 0, 1 or 2;
in some embodiments, s is selected from 0, 1, 2 or 3;
in some embodiments, s is selected from 0, 1 or 2;
in some embodiments, s is selected from 0 or 1;
in some embodiments, t is selected from 0, 1, 2, 3, 4, 5, 6 or 7;
in some embodiments, t is selected from 0, 1, 2, 3 or 4;
in some embodiments, t is selected from 0, 1, 2 or 3;
in some embodiments, t is selected from 0, 1 or 2;
in some embodiments, r is selected from 0, 1, 2 or 3.

As a first embodiment of the present invention, provided is a compound of general formula (I) or a stereoisomer, tautomer, deuterate, solvate, prodrug, metabolite, pharmaceutically acceptable salt or co-crystal thereof,

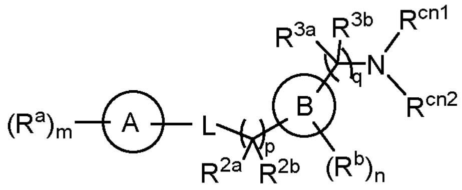
(I)

wherein ring A is selected from

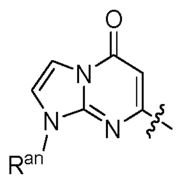
,
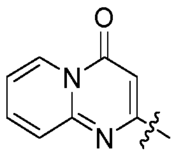
,
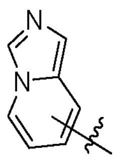
,

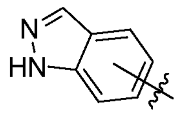
, or
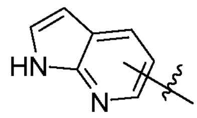
;

L is selected from

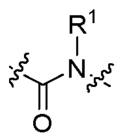

or 5- to 6-membered heteroaryl, L is connected with ring A at the left side, the heteroaryl is optionally further substituted with 0 to 3 substituents selected from H, halogen, OH, cyano, $NH_2$, $C_{1-6}$ alkyl, halogen-substituted-$C_{1-6}$ alkyl, hydroxyl-substituted-$C_{1-6}$ alkyl, cyano-substituted-$C_{1-6}$ alkyl, or $C_{1-6}$ alkoxy, the heteroaryl contains 1 to 3 heteroatoms selected from O, S or N;

$R^{an}$ is selected from H, $C_{1-6}$ alkyl, $C_{3-12}$ carbocyclyl or 3- to 12-membered heterocyclyl, the alkyl, carbocyclyl or heterocyclyl is optionally further substituted with 0 to 4 substituents selected from H, halogen, OH, =O, cyano, $NH_2$, $C_{1-6}$ alkyl, $C_{2-4}$ alkenyl, $C_{2-4}$ alkynyl, -$C_{2-4}$ alkynyl-$C_{3-6}$ cycloalkyl, halogen-substituted-$C_{1-6}$ alkyl, hydroxyl-substituted-$C_{1-6}$ alkyl, cyano-substituted-$C_{1-6}$ alkyl, $C_{1-6}$ alkoxy, $C_{3-12}$ cycloalkyl, phenyl, 3- to 12-membered heterocycloalkyl or 5- to 12-membered heteroaryl, the heterocyclyl, heterocycloalkyl or heteroaryl contains 1 to 3 heteroatoms selected from O, S or N;

ring B is selected from 5- to 10-membered heterocyclyl, wherein the heterocyclyl contains 1 to 5 heteroatoms selected from O, S or N;

$R^a$ or $R^b$ is each independently selected from H, halogen, cyano, OH, =O, $C_{1-6}$ alkyl, $C_{1-6}$ alkoxy, or $C_{3-6}$ cycloalkyl, the

alkyl, alkoxy or cycloalkyl is optionally further substituted with 0 to 4 substituents selected from H, halogen, OH, =O, cyano, $NH_2$, $C_{1-6}$ alkyl, halogen-substituted-$C_{1-6}$ alkyl, hydroxyl-substituted-$C_{1-6}$ alkyl, cyano-substituted-$C_{1-6}$ alkyl, $C_{1-6}$ alkoxy or $C_{3-6}$ cycloalkyl;

alternatively, $R^{an}$ and $R^a$ together with the atom to which they are attached form a 5- to 6-membered heterocycle, the heterocycle is optionally further substituted with 0 to 4 substituents selected from H, halogen, OH, =O, cyano, $NH_2$, $C_{1-6}$ alkyl, halogen-substituted-$C_{1-6}$ alkyl, hydroxyl-substituted-$C_{1-6}$ alkyl, cyano-substituted-$C_{1-6}$ alkyl, $C_{1-6}$ alkoxy or $C_{3-6}$ cycloalkyl, the heterocycle contains 1 to 3 heteroatoms selected from O, S or N;

$R^1$ is selected from H, $C_{1-6}$ alkyl or $C_{3-6}$ cycloalkyl, the alkyl or cycloalkyl is optionally further substituted with 0 to 4 substituents selected from H, halogen, OH, =O, cyano, $NH_2$, $C_{1-6}$ alkyl, halogen-substituted-$C_{1-6}$ alkyl, hydroxyl-substituted-$C_{1-6}$ alkyl, cyano-substituted-$C_{1-6}$ alkyl, $C_{1-6}$ alkoxy or $C_{3-6}$ cycloalkyl;

$R^{2a}$, $R^{2b}$, $R^{3a}$ and $R^{3b}$ are each independently selected from H, halogen, cyano, OH, =O, $C_{1-6}$ alkyl, $C_{1-6}$ alkoxy or $C_{3-6}$ cycloalkyl, the alkyl, alkoxy or cycloalkyl is optionally further substituted with 0 to 4 substituents selected from H, halogen, OH, =O, cyano, $NH_2$, $C_{1-6}$ alkyl, halogen-substituted-$C_{1-6}$ alkyl, hydroxyl-substituted-$C_{1-6}$ alkyl, cyano-substituted-$C_{1-6}$ alkyl, $C_{1-6}$ alkoxy or $C_{3-6}$ cycloalkyl;

alternatively, $R^{2a}$ and $R^{2b}$, $R^{3a}$ and $R^{3b}$ together with the atom to which they are attached form $C_{3-6}$ carbocyclyl or 3- to 6-membered heterocyclyl, respectively, the carbocyclyl or heterocyclyl is optionally further substituted with 0 to 4 substituents selected from H, halogen, OH, =O, cyano, $NH_2$, $C_{1-6}$ alkyl, halogen-substituted-$C_{1-6}$ alkyl, hydroxyl-substituted-$C_{1-6}$ alkyl, cyano-substituted-$C_{1-6}$ alkyl, $C_{1-6}$ alkoxy or $C_{3-6}$ cycloalkyl, the heterocyclyl contains 1 to 3 heteroatoms selected from O, S or N;

$R^{cn1}$ and $R^{cn2}$ are each independently selected from H, halogen, cyano, OH, =O, $C_{1-6}$ alkyl, $C_{1-6}$ alkoxy, $C_{3-10}$ carbocyclyl, 3- to 10-membered heterocyclyl or

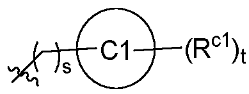,

the alkyl, alkoxy, carbocyclyl or heterocyclyl is optionally further substituted with 0 to 4 substituents selected from H, halogen, OH, =O, cyano, $NH_2$, $C_{1-6}$ alkyl, halogen-substituted-$C_{1-6}$ alkyl, hydroxyl-substituted-$C_{1-6}$ alkyl, cyano-substituted-$C_{1-6}$ alkyl, $C_{1-6}$ alkoxy or $C_{3-6}$ cycloalkyl, the heterocyclyl contains 1 to 3 heteroatoms selected from O, S or N;

ring C1 is selected from $C_{3-12}$ carbocyclyl or 3- to 12-membered heterocyclyl, and the heterocyclyl contains 1 to 3 heteroatoms selected from O, S or N;

$R^{c1}$ is selected from H, halogen, cyano, OH, =O, $C_{1-6}$ alkyl, $C_{1-6}$ alkoxy, $C_{3-12}$ carbocyclyl or 3- to 12-membered heterocyclyl, and the alkyl, alkoxy, carbocyclyl or heterocyclyl is optionally further substituted with 0 to 4 substituents selected from H, halogen, OH, =O, cyano, $NH_2$, $C_{1-6}$ alkyl, halogen-substituted-$C_{1-6}$ alkyl, hydroxyl-substituted-$C_{1-6}$ alkyl, cyano-substituted-$C_{1-6}$ alkyl, $C_{1-6}$ alkoxy or $C_{3-6}$ cycloalky, the heterocyclyl contains 1 to 3 heteroatoms selected from O, S or N;

alternatively, $R^{cn1}$ and $R^{cn2}$ together with the atom to which they are attached form 3- to 12-membered heterocyclyl, and the heterocyclyl is optionally further substituted with 0 to 4 substituents selected from H, halogen, OH, =O, cyano, $NH_2$, $C_{1-6}$ alkyl, halogen-substituted-$C_{1-6}$ alkyl, hydroxyl-substituted-$C_{1-6}$ alkyl, cyano-substituted-$C_{1-6}$ alkyl, $C_{1-6}$ alkoxy or $C_{3-6}$ cycloalkyl, the heterocyclyl contains 1 to 3 heteroatoms selected from O, S or N;

m is selected from 0, 1, 2 or 3;

n is selected from 0, 1, 2, 3, 4, 5, 6 or 7;

p is selected from 0, 1, 2 or 3;

q is selected from 0, 1, 2 or 3;

s is selected from 0, 1, 2 or 3;

t is selected from 0, 1, 2, 3, 4, 5, 6 or 7.

As a second embodiment of the present invention, provided is a compound of general formula (II) or a stereoisomer, tautomer, deuterate, solvate, prodrug, metabolite, pharmaceutically acceptable salt or co-crystal thereof, wherein the compound is selected from a compound of general formula (II),

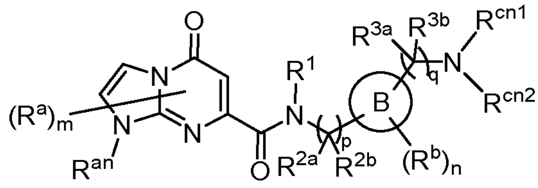

wherein $R^{an}$ is selected from H, $C_{1-6}$ alkyl, $C_{3-12}$ carbocyclyl or 3- to 12-membered heterocyclyl, the alkyl, cycloalkyl, carbocyclyl or heterocyclyl is optionally further substituted with 0 to 4 substituents selected from H, D, halogen, OH, =O, cyano, $NH_2$, $C_{1-6}$ alkyl, $C_{2-4}$ alkenyl, $C_{2-4}$ alkynyl, -$C_{2-4}$ alkynyl-$C_{3-6}$ cycloalkyl, halogen-substituted-$C_{1-6}$ alkyl, hydroxyl-substituted-$C_{1-6}$ alkyl, cyano-substituted-$C_{1-6}$ alkyl, $C_{1-6}$ alkoxy, $C_{3-12}$ cycloalkyl, phenyl, 3- to 12-membered heterocycloalkyl or 5- to 12-membered heteroaryl, and the heterocyclyl, heterocycloalkyl or heteroaryl contains 1 to 3 heteroatoms selected from O, S or N;

ring B is selected from 5- to 10-membered heterocyclyl, wherein the heterocyclyl contains 1 to 5 heteroatoms selected from O, S or N;

$R^a$ or $R^b$ is each independently selected from H, halogen, cyano, OH, =O, $C_{1-6}$ alkyl, $C_{1-6}$ alkoxy, or $C_{3-6}$ cycloalkyl, the alkyl, alkoxy or cycloalkyl is optionally further substituted with 0 to 4 substituents selected from H, halogen, OH, =O, cyano, $NH_2$, $C_{1-6}$ alkyl, halogen-substituted-$C_{1-6}$ alkyl, hydroxyl-substituted-$C_{1-6}$ alkyl, cyano-substituted-$C_{1-6}$ alkyl, $C_{1-6}$ alkoxy or $C_{3-6}$ cycloalkyl;

alternatively, $R^{an}$ and $R^a$ together with the atom to which they are attached form a 5- to 6-membered heterocycle, the heterocycle is optionally further substituted with 0 to 4 substituents selected from H, halogen, OH, =O, cyano, $NH_2$, $C_{1-6}$ alkyl, halogen-substituted-$C_{1-6}$ alkyl, hydroxyl-substituted-$C_{1-6}$ alkyl, cyano-substituted-$C_{1-6}$ alkyl, $C_{1-6}$ alkoxy or $C_{3-6}$ cycloalkyl, the heterocycle contains 1 to 3 heteroatoms selected from O, S or N;

$R^1$ is selected from H, $C_{1-6}$ alkyl or $C_{3-6}$ cycloalkyl, the alkyl or cycloalkyl is optionally further substituted with 0 to 4 substituents selected from H, halogen, OH, =O, cyano, $NH_2$, $C_{1-6}$ alkyl, halogen-substituted-$C_{1-6}$ alkyl, hydroxyl-substituted-$C_{1-6}$ alkyl, cyano-substituted-$C_{1-6}$ alkyl, $C_{1-6}$ alkoxy or $C_{3-6}$ cycloalkyl;

$R^{2a}$, $R^{2b}$, $R^{3a}$ and $R^{3b}$ are each independently selected from H, halogen, cyano, OH, =O, $C_{1-6}$ alkyl, $C_{1-6}$ alkoxy or $C_{3-6}$ cycloalkyl, the alkyl, alkoxy or cycloalkyl is optionally further substituted with 0 to 4 substituents selected from H, halogen, OH, =O, cyano, $NH_2$, $C_{1-6}$ alkyl, halogen-substituted-$C_{1-6}$ alkyl, hydroxyl-substituted-$C_{1-6}$ alkyl, cyano-substituted-$C_{1-6}$ alkyl, $C_{1-6}$ alkoxy or $C_{3-6}$ cycloalkyl;

alternatively, $R^{2a}$ and $R^{2b}$, $R^{3a}$ and $R^{3b}$ together with the atom to which they are attached form $C_{3-6}$ carbocyclyl or 3- to 6-membered heterocyclyl, respectively, the carbocyclyl or heterocyclyl is optionally further substituted with 0 to 4 substituents selected from H, halogen, OH, =O, cyano, $NH_2$, $C_{1-6}$ alkyl, halogen-substituted-$C_{1-6}$ alkyl, hydroxyl-substituted-$C_{1-6}$ alkyl, cyano-substituted-$C_{1-6}$ alkyl, $C_{1-6}$ alkoxy or $C_{3-6}$ cycloalkyl, the heterocyclyl contains 1 to 3 heteroatoms selected from O, S or N;

$R^{cn1}$ and $R^{cn2}$ are each independently selected from H, halogen, cyano, OH, =O, $C_{1-6}$ alkyl, $C_{1-6}$ alkoxy, $C_{3-10}$ carbocyclyl, 3- to 10-membered heterocyclyl or

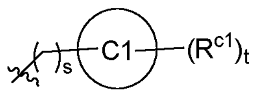

,

the alkyl, alkoxy, carbocyclyl or heterocyclyl is optionally further substituted with 0 to 4 substituents selected from H, halogen, OH, =O, cyano, $NH_2$, $C_{1-6}$ alkyl, halogen-substituted-$C_{1-6}$ alkyl, hydroxyl-substituted-$C_{1-6}$ alkyl, cyano-substituted-$C_{1-6}$ alkyl, $C_{1-6}$ alkoxy or $C_{3-6}$ cycloalkyl, the heterocyclyl contains 1 to 3 heteroatoms selected from O, S or N;

ring C1 is selected from $C_{3-12}$ carbocyclyl or 3- to 12-membered heterocyclyl, and the heterocyclyl contains 1 to 3 heteroatoms selected from O, S or N;

$R^{c1}$ is selected from H, halogen, cyano, OH, =O, $C_{1-6}$ alkyl, $C_{1-6}$ alkoxy, $C_{3-12}$ carbocyclyl or 3- to 12-membered heterocyclyl, and the alkyl, alkoxy, carbocyclyl or heterocyclyl is optionally further substituted with 0 to 4 substituents selected from H, halogen, OH, =O, cyano, $NH_2$, $C_{1-6}$ alkyl, halogen-substituted-$C_{1-6}$ alkyl, hydroxyl-substituted-$C_{1-6}$ alkyl, cyano-substituted-$C_{1-6}$ alkyl, $C_{1-6}$ alkoxy or $C_{3-6}$ cycloalky, the heterocyclyl contains 1 to 3 heteroatoms selected from O, S or N;

alternatively, $R^{cn1}$ and $R^{cn2}$ together with the atom to which they are attached form 3- to 12-membered heterocyclyl, and the heterocyclyl is optionally further substituted with 0 to 4 substituents selected from H, halogen, OH, =O, cyano, $NH_2$, $C_{1-6}$ alkyl, halogen-substituted-$C_{1-6}$ alkyl, hydroxyl-substituted-$C_{1-6}$ alkyl, cyano-substituted-$C_{1-6}$ alkyl, $C_{1-6}$ alkoxy or $C_{3-6}$ cycloalkyl, the heterocyclyl contains 1 to 3 heteroatoms selected from O, S or N;

m is selected from 0, 1, 2 or 3;
n is selected from 0, 1, 2, 3, 4, 5, 6 or 7;
p is selected from 0, 1, 2 or 3;
q is selected from 0, 1, 2 or 3;
s is selected from 0, 1, 2 or 3;
t is selected from 0, 1, 2, 3, 4, 5, 6 or 7.

As a third embodiment of the present invention, provided is the aforementioned compound of general formula (I) or (II) or a stereoisomer, tautomer, deuterate, solvate, prodrug, metabolite, pharmaceutically acceptable salt or co-crystal thereof, wherein

$R^{an}$ is selected from H, $C_{1-4}$ alkyl, $C_{3-6}$ cycloalkyl, or 3- to 6-membered heterocycloalkyl, the alkyl, cycloalkyl, cycloalkyl or heterocycloalkyl is optionally further substituted with 0 to 4 substituents selected from H, D, halogen, OH, =O, cyano, $NH_2$, $C_{1-4}$ alkyl, $C_{2-4}$ alkenyl, $C_{2-4}$ alkynyl, -$C_{2-4}$ alkynyl-$C_{3-6}$ cycloalkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy, $C_{3-6}$ cycloalkyl, phenyl, 3- to 6-membered heterocycloalkyl or 5- to 6-membered heteroaryl, and the heterocycloalkyl or heteroaryl contains 1 to 3 heteroatoms selected from O, S or N;
$R^a$ or $R^b$ is each independently selected from H, halogen, cyano, OH, =O, $C_{1-4}$ alkyl, $C_{1-4}$ alkoxy, or $C_{3-6}$ cycloalkyl, and the alkyl, alkoxy or cycloalkyl is optionally further substituted with 0 to 4 substituents selected from H, halogen, OH, =O, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy or $C_{3-6}$ cycloalkyl;
alternatively, $R^{an}$ and $R^a$ together with the atom to which they are attached form a 5- to 6-membered heteroaromatic ring, the heteroaromatic ring is optionally further substituted with 0 to 4 substituents selected from H, halogen, OH, =O, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy or $C_{3-6}$ cycloalkyl, and the heteroaryl contains 1 to 3 heteroatoms selected from O, S or N;
$R^1$ is selected from H, $C_{1-4}$ alkyl or $C_{3-6}$ cycloalkyl, the alkyl or cycloalkyl is optionally further substituted with 0 to 4 substituents selected from H, halogen, OH, =O, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy or $C_{3-6}$ cycloalkyl;
$R^{2a}$, $R^{2b}$, $R^{3a}$ and $R^{3b}$ are each independently selected from H, halogen, cyano, OH, =O, $C_{1-4}$ alkyl, $C_{1-4}$ alkoxy or $C_{3-6}$ cycloalkyl, and the alkyl, alkoxy or cycloalkyl is optionally further substituted with 0 to 4 substituents selected from H, halogen, OH, =O, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy or $C_{3-6}$ cycloalkyl;
ring B is selected from 5-fused-5-membered bisheteroaryl, 5-fused-6-membered bisheteroaryl or 6-fused-6-membered bisheteroaryl, and the heteroaryl contains 1 to 5 heteroatoms selected from O, S or N;
m is selected from 0, 1 or 2;
n is selected from 0, 1, 2, 3 or 4;
p is selected from 0, 1 or 2;
q is selected from 0, 1 or 2;
the definitions of the others are the same as those in the second embodiment of the present invention.

As a fourth embodiment of the present invention, provided is the aforementioned compound of general formula (I) or (II) or a stereoisomer, tautomer, deuterate, solvate, prodrug, metabolite, pharmaceutically acceptable salt or co-crystal thereof, wherein

$R^{cn1}$ and $R^{cn2}$ are each independently selected from H, halogen, cyano, OH, =O, $C_{1-4}$ alkyl, $C_{1-4}$ alkoxy, $C_{3-10}$ cycloalkyl, 3- to 10-membered heterocycloalkyl or

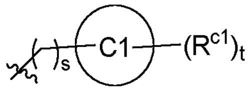
,

the alkyl, alkoxy, cycloalkyl or heterocycloalkyl is optionally further substituted with 0 to 4 substituents selected from H, halogen, OH, =O, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy or $C_{3-6}$ cycloalkyl, and the heterocycloalkyl contains 1 to 3 heteroatoms selected from O, S or N;
ring C1 is selected from $C_{3-7}$monocycloalkyl, $C_{4-11}$fused cycloalkyl, $C_{5-11}$ spirocycloalkyl, $C_{5-12}$bridged cycloalkyl, 4- to 7-membered monoheterocycloalkyl, 4- to 11-membered fused heterocycloalkyl, 5- to 11-membered spiroheterocycloalkyl or 5- to 12-membered bridged heterocycloalkyl, and the heterocycloalkyl contains 1 to 3 heteroatoms

selected from O, S or N;

$R^{c1}$ is selected from H, halogen, cyano, OH, =O, $C_{1-4}$ alkyl, $C_{1-4}$ alkoxy, $C_{3-6}$ cycloalkyl or 3- to 6-membered heterocycloalkyl, the alkyl, alkoxy, cycloalkyl or heterocycloalkyl is optionally further substituted with 0 to 4 substituents selected from H, halogen, OH, =O, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy or $C_{3-6}$ cycloalkyl, and the heterocycloalkyl contains 1 to 3 heteroatoms selected from O, S or N;

alternatively, $R^{cn1}$ and $R^{cn2}$ together with the atom to which they are attached form 4- to 7-membered monoheterocycloalkyl, 4- to 11-membered fused heterocycloalkyl, 5- to 11-membered spiroheterocycloalkyl or 5- to 12-membered bridged heterocycloalkyl, the heterocycloalkyl is optionally further substituted with 0 to 4 substituents selected from H, halogen, OH, =O, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy or $C_{3-6}$ cycloalkyl, and the heterocycloalkyl contains 1 to 3 heteroatoms selected from O, S or N;

s is selected from 0, 1 or 2;

t is selected from 0, 0, 1, 2, 3 or 4;

the remaining definitions are the same as those of the first, second, third or fourth embodiment of the present invention.

As a fifth embodiment of the present invention, provided is the aforementioned compound of general formula (I) or (II) or a stereoisomer, tautomer, deuterate, solvate, prodrug, metabolite, pharmaceutically acceptable salt or co-crystal thereof, wherein

$R^{an}$ is selected from H, methyl, ethyl, propyl, isopropyl, cyclopropyl, cyclohexyl, cyclopentyl, cyclobutyl, oxetanyl, oxacyclopentyl, oxacyclohexyl, azetidinyl, azacyclopentyl, piperidyl or piperazinyl, the methyl, ethyl, propyl, isopropyl, cyclopropyl, cyclohexyl, cyclopentyl, cyclobutyl, oxetanyl, oxacyclopentyl, oxacyclohexyl, azetidinyl, azacyclopentyl, piperidyl or piperazinyl is optionally further substituted with 0 to 4 substituents selected from H, D, F, Cl, Br, I, OH, =O, cyano, $NH_2$, $C_{1-4}$ alkyl, $C_{2-4}$ alkenyl, $C_{2-4}$ alkynyl, -$C_{2-4}$ alkynyl-$C_{3-6}$ cycloalkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy, $C_{3-6}$ cycloalkyl, phenyl, 3- to 6-membered heterocycloalkyl or 5- to 6-membered heteroaryl, and the heterocycloalkyl or heteroaryl contains 1 to 3 heteroatoms selected from O, S or N;

$R^a$ or $R^b$ is each independently selected from H, F, Cl, Br, I, cyano, OH, =O, methyl, ethyl, propyl, isopropyl, cyclopropyl, cyclohexyl, cyclopentyl, cyclobutyl, azetidinyl, azacyclopentyl, azacyclohexyl, piperidyl, methoxy or ethoxy, and the methyl, ethyl, propyl, isopropyl, cyclopropyl, cyclohexyl, cyclopentyl, cyclobutyl, azetidinyl, azacyclopentyl, azacyclohexyl, piperidyl, methoxy or ethoxy is optionally further substituted with 0 to 4 substituents selected from H, F, Cl, Br, I, OH, =O, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy or $C_{3-6}$ cycloalkyl;

alternatively, $R^{an}$ and $R^a$ together with the atom to which they are attached form a pyridine ring, pyrimidine ring, pyridazine ring or pyrazine ring, and the pyridine ring, pyrimidine ring or pyrazine ring is optionally further substituted with 0 to 4 substituents selected from H, F, Cl, Br, I, OH, =O, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy or $C_{3-6}$ cycloalkyl;

$R^1$ is selected from H, methyl, ethyl, propyl, isopropyl or cyclopropyl, and the methyl, ethyl, propyl, isopropyl or cyclopropyl is optionally further substituted with 0 to 4 substituents selected from H, F, Cl, Br, I, OH, =O, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy or $C_{3-6}$ cycloalkyl;

$R^{2a}$, $R^{2b}$, $R^{3a}$ and $R^{3b}$ are each independently selected from H, F, Cl, Br, I, cyano, OH, =O, methyl, ethyl, propyl, isopropyl, methoxy, ethoxy or cyclopropyl, and the methyl, ethyl, propyl, isopropyl, methoxy, ethoxy or cyclopropyl is optionally further substituted with 0 to 4 substituents selected from H, F, Cl, Br, I, OH, =O, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy or $C_{3-6}$ cycloalkyl;

ring B is selected from pyrrolopyrrolyl, pyrrolopyrazolyl, pyrroloimidazolyl, pyrazolopyrazolyl, pyrazoloimidazolyl, imidazoimidazolyl, benzopyrrolyl, benzopyrazolyl, benzoimidazolyl, pyridopyrrolyl, pyridopyrazolyl, pyridoimidazolyl, pyrimidopyrrolyl, pyrimidopyrazolyl, pyrimidoimidazolyl, pyrazinopyrrolyl, pyrazinopyrazolyl, pyrazinoimidazolyl, pyridazinopyrrolyl, pyridazinopyrazolyl, pyridazinoimidazolyl, benzopyridyl, benzopyrimidyl, benzopyrazinyl, benzopyridazinyl, pyridopyridyl, pyridopyrimidinyl, pyridopyrazinyl or pyridopyridazinyl;

$R^{cn1}$ and $R^{cn2}$ are each independently selected from H, F, Cl, Br, I, cyano, OH, =O, methyl, ethyl, propyl, isopropyl, cyclopropyl, cyclohexyl, cyclopentyl, cyclobutyl, azetidinyl, azacyclopentyl, azacyclohexyl or

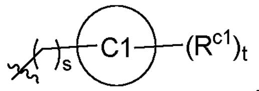

,

and the methyl, ethyl, propyl, isopropyl, cyclopropyl, cyclohexyl, cyclopentyl, cyclobutyl, azetidinyl, azacyclopentyl, or azacyclohexyl is optionally further substituted with 0 to 4 substituents selected from H, F, Cl, Br, I, OH, =O, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy or $C_{3-6}$ cycloalkyl;

ring C1 is selected from cyclopropyl, cyclobutyl, cyclopentyl, cyclohexyl, oxetanyl, oxacyclopentyl, oxacyclohexyl, azetidinyl, azacyclopentyl, piperidine, morpholine, piperazine, 1,4-diazepanyl, cyclopropyl fused cyclopentyl, cyclobutyl fused cyclopentyl, cyclopentyl fused cyclopentyl, cyclopentyl fused cyclohexyl, cyclopropyl spirocyclopentyl, cyclobutyl spirocyclobutyl, cyclobutyl spirocyclopentyl, cyclopentyl spirocyclopentyl, cyclopentyl spirocyclohexyl, cyclohexyl spirocyclohexyl, cyclopropyl fused azetidinyl, cyclopropyl fused azacyclopentyl, cyclopropyl fused azacyclohexyl, cyclobutyl fused azetidinyl, cyclobutyl fused azacyclopentyl, cyclobutyl fused azacyclohexyl, cyclopentyl fused azetidinyl, cyclopentyl fused azacyclopentyl, cyclopentyl fused azacyclohexyl, cyclohexyl fused azetidinyl, cyclohexyl fused azacyclopentyl, cyclohexyl fused azacyclohexyl, azetidinyl fused azetidinyl, azetidinyl fused azacyclopentyl, azetidinyl fused azacyclohexyl, azacyclopentyl fused azetidinyl, azacyclopentyl fused azacyclopentyl, azacyclopentyl fused azacyclohexyl, azacyclohexyl fused azetidinyl, azacyclohexyl fused azacyclopentyl, azacyclohexyl fused azacyclohexyl, cyclobutyl spiroazetidinyl, cyclobutyl spiroazacyclopentyl, cyclobutyl spiroazacyclohexyl, cyclopentyl spiroazetidinyl, cyclopentyl spiroazacyclopentyl, cyclopentyl spiroazacyclohexyl, cyclohexyl spiroazetidinyl, cyclohexyl spiroazacyclopentyl, cyclohexyl spiroazacyclohexyl, azetidinyl spiroazetidinyl, azetidinyl spiroazacyclopentyl, azetidinyl spiroazacyclohexyl, azacyclopentyl spiroazetidinyl, azacyclopentyl spiroazacyclopentyl, azacyclopentyl spiroazacyclohexyl, azacyclohexyl spiroazetidinyl, azacyclohexyl spiroazacyclopentyl, azacyclohexyl spiroazacyclohexyl, oxetanyl, oxacyclopentyl, oxacyclohexyl, cyclopropyl fusedoxetanyl, cyclopropyl fusedoxacyclopentyl, cyclopropyl fusedoxacyclohexyl, cyclobutyl fusedoxetanyl, cyclobutyl fusedoxacyclopentyl, cyclobutyl fusedoxacyclohexyl, cyclopentyl fusedoxetanyl, cyclopentyl fusedoxacyclopentyl, cyclopentyl fusedoxacyclohexyl, cyclohexyl fusedoxetanyl, cyclohexyl fusedoxacyclopentyl, cyclohexyl fusedoxacyclohexyl, azetidinyl fusedoxetanyl, azetidinyl fusedoxacyclopentyl, azetidinyl fusedoxacyclohexyl, azacyclopentyl fusedoxetanyl, azacyclopentyl fusedoxacyclopentyl, azacyclopentyl fusedoxacyclohexyl, azacyclohexyl fusedoxetanyl, azacyclohexyl fusedoxacyclopentyl, azacyclohexyl fusedoxacyclohexyl, cyclobutyl spirooxetanyl, cyclobutyl spirooxacyclopentyl, cyclobutyl spirooxacyclohexyl, cyclopentyl spirooxetanyl, cyclopentyl spirooxacyclopentyl, cyclopentyl spirooxacyclohexyl, cyclohexyl spirooxetanyl, cyclohexyl spirooxacyclopentyl, cyclohexyl spirooxacyclohexyl, azetidinyl spirooxetanyl, azetidinyl spirooxacyclopentyl, azetidinyl spirooxacyclohexyl, azacyclopentyl spirooxetanyl, azacyclopentyl spirooxacyclopentyl, azacyclopentyl spirooxacyclohexyl, azacyclohexyl spirooxetanyl, azacyclohexyl spirooxacyclopentyl, azacyclohexyl spirooxacyclohexyl, bicyclo[2.1.1]hexyl, bicyclo[2.2.1]heptyl, bicyclo[3.3.2]decanyl, bicyclo[2.2.2]octanyl, bicyclo[3.2.1]octanyl, bicyclo[3.3.3]undecyl, adamantyl,

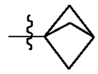 , 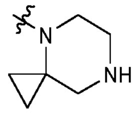 ,

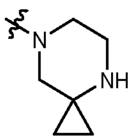 , 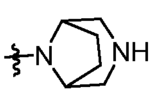 , 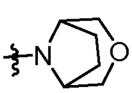 , 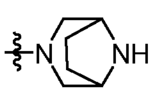 , 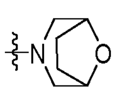 , 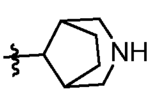 , 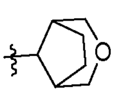 ,

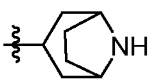 , 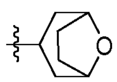 , 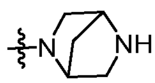 , 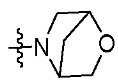 , 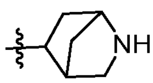 , 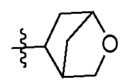 ,

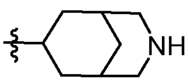 , 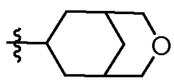 , 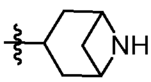 , 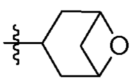 , 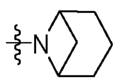 , 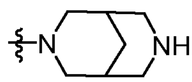 ,

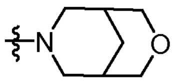, or 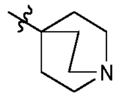;

each $R^{c1}$ is independently selected from H, F, Cl, Br, I, cyano, OH, =O, methyl, ethyl, propyl, isopropyl, cyclopropyl, cyclohexyl, cyclopentyl, cyclobutyl, azetidinyl, azacyclopentyl, piperidyl, piperazinyl, methoxy or ethoxy, and the methyl, ethyl, propyl, isopropyl, cyclopropyl, cyclohexyl, cyclopentyl, cyclobutyl, azetidinyl, azacyclopentyl, piperidyl, piperazinyl, methoxy or ethoxy is optionally further substituted with 0 to 4 substituents selected from H, F, Cl, Br, I, OH, =O, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy or $C_{3-6}$ cycloalkyl;

alternatively, $R^{cn1}$ and $R^{cn2}$ together with the atom to which they are attached form substituted or unsubstituted azetidinyl, azacyclopentyl, piperidine, piperazine, azetidinyl fused cyclopropyl, azetidinyl fused cyclobutyl, azetidinyl fused cyclopentyl, azetidinyl fused cyclohexyl, azacyclopentyl fused cyclopropyl, azacyclopentyl fused cyclobutyl, azacyclopentyl fused cyclopentyl, azacyclopentyl fused cyclohexyl, azacyclohexyl fused cyclopropyl, azacyclohexyl fused cyclobutyl, azacyclohexyl fused cyclopentyl, azacyclohexyl fused cyclohexyl, azetidinyl fused azetidinyl, azetidinyl fused azacyclopentyl, azetidinyl fused azacyclohexyl, azacyclopentyl fused azetidinyl, azacyclopentyl fused azacyclopentyl, azacyclopentyl fused azacyclohexyl, azacyclohexyl fused azetidinyl, azacyclohexyl fused azacyclopentyl, azacyclohexyl fused azacyclohexyl, azetidinyl spiroazetidinyl, azetidinyl spiroazacyclopentyl, azetidinyl spiroazacyclohexyl, azacyclopentyl spiroazetidinyl, azacyclopentyl spiroazacyclopentyl, azacyclopentyl spiroazacyclohexyl, azacyclohexyl spiroazetidinyl, azacyclohexyl spiroazacyclopentyl, azacyclohexyl spiroazacyclohexyl,

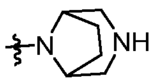, 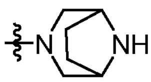,

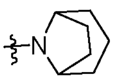, 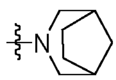, 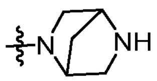, 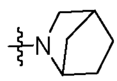, 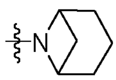, 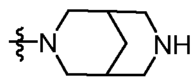,

or

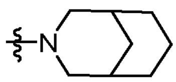,

which, when substituted, is optionally further substituted with 0 to 4 substituents selected from H, halogen, OH, =O, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy or $C_{3-6}$ cycloalkyl;

the remaining definitions are the same as those of the first, second, third or fourth embodiment of the present invention.

As a sixth embodiment of the present invention, provided is the aforementioned compound of general formula (I) or (II) or a stereoisomer, tautomer, deuterate, solvate, prodrug, metabolite, pharmaceutically acceptable salt or co-crystal thereof, wherein

$R^{an}$ is selected from H, methyl, ethyl, propyl, isopropyl, cyclopropyl, cyclobutyl, cyclopentyl, oxetanyl, oxacyclopentyl or oxacyclohexyl, the methyl, ethyl, propyl, isopropyl, cyclopropyl, cyclobutyl, cyclopentyl, oxetanyl, oxacyclopentyl or oxacyclohexyl is optionally further substituted with 0 to 4 substituents selected from H, D, F, Cl, Br, I, OH, =O, cyano, $NH_2$, $C_{1-4}$ alkyl, halogen-substituted-$C_{1-4}$ alkyl, $C_{2-4}$ alkenyl, $C_{2-4}$ alkynyl, -$C_{2-4}$ alkynyl-$C_{3-6}$ cycloalkyl, hydroxyl-substituted-$C_{1-4}$ alkyl, cyano-substituted-$C_{1-4}$ alkyl, $C_{1-4}$ alkoxy, $C_{3-6}$ cycloalkyl, phenyl, 3- to 6-membered heterocycloalkyl or 5- to 6-membered heteroaryl, and the heterocycloalkyl or heteroaryl contains 1 to 3 heteroatoms selected from O, S or N;

$R^a$ or $R^b$ is each independently selected from H, F, Cl, Br, I, cyano, OH, =O, methyl, ethyl, propyl, isopropyl, or cyclopropyl, and the methyl, ethyl, propyl, isopropyl or cyclopropyl is optionally further substituted with 0 to 4 substituents selected from H, F, Cl, Br, I, OH, =O, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy or $C_{3-6}$ cycloalkyl;

alternatively, $R^{an}$ and $R^a$ together with the atom to which they are attached form a pyridine ring, and the pyridine ring is

optionally further substituted with 0 to 4 substituents selected from H, F, Cl, Br, I, OH, =O, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy or $C_{3-6}$ cycloalkyl;

ring B is selected from benzopyrrolyl, benzopyrazolyl, benzoimidazolyl, pyridopyrrolyl, pyridopyrazolyl, pyridoimidazolyl, pyrimidopyrrolyl, pyrimidopyrazolyl, pyrimidoimidazolyl, pyrazinopyrrolyl, pyrazinopyrazolyl, pyrazinoimidazolyl, pyridazinopyrrolyl, pyridazinopyrazolyl, or pyridazinoimidazolyl;

$R^{cn1}$ and $R^{cn2}$ are each independently selected from H, F, Cl, Br, I, cyano, OH, =O, methyl, ethyl, propyl, isopropyl, cyclopropyl, cyclohexyl, cyclopentyl, cyclobutyl or

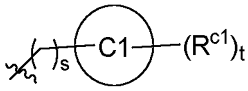

,

and the methyl, ethyl, propyl, isopropyl, cyclopropyl, cyclohexyl, cyclopentyl, or cyclobutyl is optionally further substituted with 0 to 4 substituents selected from H, F, Cl, Br, I, OH, =O, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy or $C_{3-6}$ cycloalkyl;

ring C1 is selected from cyclopropyl, cyclobutyl, cyclopentyl, cyclohexyl, oxetanyl, oxacyclopentyl, oxacyclohexyl, azetidinyl, azacyclopentyl, piperidine, morpholine, piperazine, 1,4-diazepanyl,

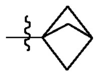

,

bicyclo[2.1.1]hexyl, bicyclo[2.2.1]heptyl, bicyclo[3.3.2]decanyl, bicyclo[2.2.2]octanyl, bicyclo[3.2.1]octanyl, bicyclo [3.3.3]undecyl, or adamantyl;

each $R^{c1}$ is independently selected from H, F, Cl, Br, I, cyano, OH, =O, methyl, ethyl, propyl, isopropyl, cyclopropyl, methoxy or ethoxy, and the methyl, ethyl, propyl, isopropyl, cyclopropyl, methoxy or ethoxy is optionally further substituted with 0 to 4 substituents selected from H, F, Cl, Br, I, OH, =O, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy or $C_{3-6}$ cycloalkyl;

alternatively, $R^{cn1}$ and $R^{cn2}$ together with the atom to which they are attached form substituted or unsubstituted azetidinyl, azacyclopentyl, piperidine or piperazine, which, when substituted, is optionally further substituted with 0 to 4 substituents selected from H, F, Cl, Br, I, OH, =O, cyano, NH2, $C_{1-4}$ alkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy or $C_{3-6}$ cycloalkyl;

the remaining definitions are the same as those of the first, second, third, fourth or fifth embodiment of the present invention.

As a seventh embodiment of the present invention, provided is the compound of the following general formula (II-1) or general formula (II-2) or a stereoisomer, tautomer, deuterate, solvate, prodrug, metabolite, pharmaceutically acceptable salt or co-crystal thereof,

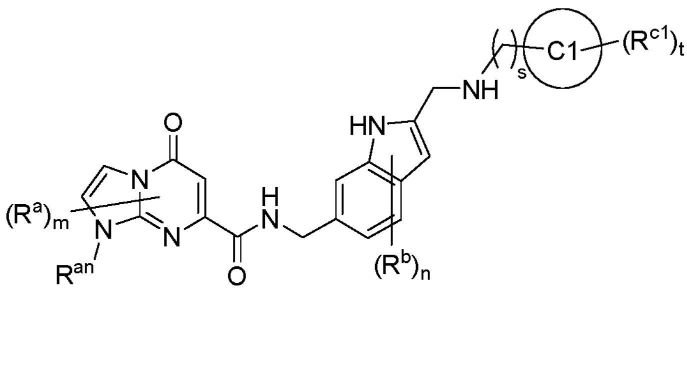

(II-1)

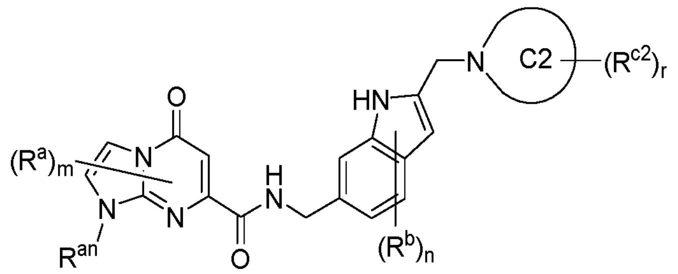

(II-2)

wherein $R^{an}$ is selected from H, methyl, $CD_3$, ethyl, propyl, isopropyl, cyclopropyl, oxetanyl, oxacyclopentyl or oxacyclohexyl, and the methyl, ethyl, propyl, isopropyl, cyclopropyl, oxetanyl, oxacyclopentyl or oxacyclohexyl is optionally further substituted with 0 to 4 substituents selected from H, F, Cl, Br, I, OH, =O, cyano, $NH_2$, methyl, ethyl, propyl, isopropyl, ethenyl, 1-propenyl, 2-propenyl, ethynyl, 1-propynyl, 2-propynyl,

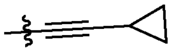,

cyclopropyl, methoxy, ethoxy, or phenyl;

$R^a$ or $R^b$ is each independently selected from H, F, Cl, Br, I, cyano, OH, =O, methyl, ethyl, propyl, isopropyl, or cyclopropyl;

alternatively, $R^{an}$ and $R^a$ together with the atom to which they are attached form a pyridine ring;

ring C1 is selected from cyclopropyl, cyclobutyl, cyclopentyl, cyclohexyl or

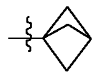;

each $R^{c1}$ is independently selected from H, F, Cl, Br, I, cyano, OH, =O, methyl, ethyl, propyl, isopropyl or cyclopropyl;

ring C2 is selected from azetidinyl, azacyclopentyl, piperidine or piperazine;

each $R^{c2}$ is independently selected from H, F, Cl, Br, I, OH, =O, cyano, $NH_2$, methyl, ethyl, propyl, isopropyl, cyclopropyl, methoxy, or ethoxy;

m is selected from 0 or 1;

n is selected from 0 or 1;

r is selected from 0, 1, 2 or 3;

s is selected from 0 or 1;

t is selected from 0, 1, 2 or 3;

the remaining definitions are the same as those of the first, second, third, fourth, fifth or sixth embodiment of the present invention.

As an eighth embodiment of the present invention, provided is the compound of the following general formula (III) or a stereoisomer, tautomer, deuterate, solvate, prodrug, metabolite, pharmaceutically acceptable salt or co-crystal thereof,

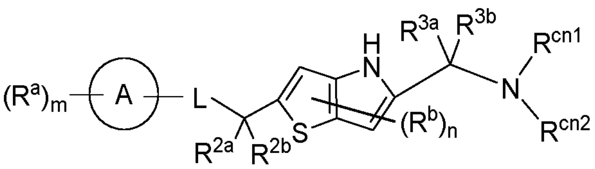

(III)

wherein ring A is selected from

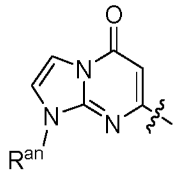, 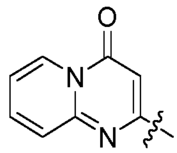, 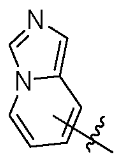,

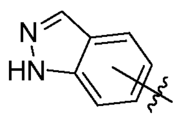, or 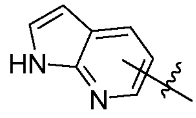;

L is selected from

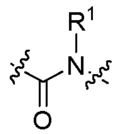

or 5- to 6-membered heteroaryl, L is connected with ring A at the left side, the heteroaryl is optionally further substituted with 0 to 3 substituents selected from H, halogen, OH, cyano, $NH_2$, $C_{1-6}$ alkyl, halogen-substituted-$C_{1-6}$ alkyl, hydroxyl-substituted-$C_{1-6}$ alkyl, cyano-substituted-$C_{1-6}$ alkyl, or $C_{1-6}$ alkoxy, the heteroaryl contains 1 to 3 heteroatoms selected from O, S or N;

$R^{an}$ is selected from H, $C_{1-6}$ alkyl, $C_{3-12}$ carbocyclyl or 3- to 12-membered heterocyclyl, the alkyl, carbocyclyl or heterocyclyl is optionally further substituted with 0 to 4 substituents selected from H, halogen, OH, =O, cyano, $NH_2$, $C_{1-6}$ alkyl, $C_{2-4}$ alkenyl, $C_{2-4}$ alkynyl, -$C_{2-4}$ alkynyl-$C_{3-6}$ cycloalkyl, halogen-substituted-$C_{1-6}$ alkyl, hydroxyl-substituted-$C_{1-6}$ alkyl, cyano-substituted-$C_{1-6}$ alkyl, $C_{1-6}$ alkoxy, $C_{3-12}$ cycloalkyl, phenyl, 3- to 12-membered heterocycloalkyl or 5- to 12-membered heteroaryl, the heterocyclyl, heterocycloalkyl or heteroaryl contains 1 to 3 heteroatoms selected from O, S or N;

$R^a$ or $R^b$ is each independently selected from H, halogen, cyano, OH, =O, $C_{1-6}$ alkyl, $C_{1-6}$ alkoxy, or $C_{3-6}$ cycloalkyl, the alkyl, alkoxy or cycloalkyl is optionally further substituted with 0 to 4 substituents selected from H, halogen, OH, =O, cyano, $NH_2$, $C_{1-6}$ alkyl, halogen-substituted-$C_{1-6}$ alkyl, hydroxyl-substituted-$C_{1-6}$ alkyl, cyano-substituted-$C_{1-6}$ alkyl, $C_{1-6}$ alkoxy or $C_{3-6}$ cycloalkyl;

$R^1$ is selected from H, $C_{1-6}$ alkyl or $C_{3-6}$ cycloalkyl, the alkyl or cycloalkyl is optionally further substituted with 0 to 4 substituents selected from H, halogen, OH, =O, cyano, $NH_2$, $C_{1-6}$ alkyl, halogen-substituted-$C_{1-6}$ alkyl, hydroxyl-substituted-$C_{1-6}$ alkyl, cyano-substituted-$C_{1-6}$ alkyl, $C_{1-6}$ alkoxy or $C_{3-6}$ cycloalkyl;

$R^{2a}$, $R^{2b}$, $R^{3a}$ and $R^{3b}$ are each independently selected from H, halogen, cyano, OH, =O, $C_{1-6}$ alkyl, $C_{1-6}$ alkoxy or $C_{3-6}$ cycloalkyl, the alkyl, alkoxy or cycloalkyl is optionally further substituted with 0 to 4 substituents selected from H, halogen, OH, =O, cyano, $NH_2$, $C_{1-6}$ alkyl, halogen-substituted-$C_{1-6}$ alkyl, hydroxyl-substituted-$C_{1-6}$ alkyl, cyano-substituted-$C_{1-6}$ alkyl, $C_{1-6}$ alkoxy or $C_{3-6}$ cycloalkyl;

alternatively, $R^{2a}$ and $R^{2b}$, $R^{3a}$ and $R^{3b}$ together with the atom to which they are attached form $C_{3-6}$ carbocyclyl or 3- to 6-membered heterocyclyl, respectively, the carbocyclyl or heterocyclyl is optionally further substituted with 0 to 4 substituents selected from H, halogen, OH, =O, cyano, $NH_2$, $C_{1-6}$ alkyl, halogen-substituted-$C_{1-6}$ alkyl, hydroxyl-substituted-$C_{1-6}$ alkyl, cyano-substituted-$C_{1-6}$ alkyl, $C_{1-6}$ alkoxy or $C_{3-6}$ cycloalkyl, the heterocyclyl contains 1 to 3 heteroatoms selected from O, S or N;

$R^{cn1}$ and $R^{cn2}$ are each independently selected from H, halogen, cyano, OH, =O, $C_{1-6}$ alkyl, $C_{1-6}$ alkoxy, $C_{3-10}$ carbocyclyl, 3- to 10-membered heterocyclyl or

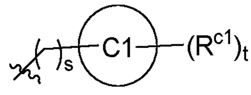,

the alkyl, alkoxy, carbocyclyl or heterocyclyl is optionally further substituted with 0 to 4 substituents selected from H, halogen, OH, =O, cyano, $NH_2$, $C_{1-6}$ alkyl, halogen-substituted-$C_{1-6}$ alkyl, hydroxyl-substituted-$C_{1-6}$ alkyl, cyano-substituted-$C_{1-6}$ alkyl, $C_{1-6}$ alkoxy or $C_{3-6}$ cycloalkyl, the heterocyclyl contains 1 to 3 heteroatoms selected from O, S or N;

ring C1 is selected from $C_{3-12}$ carbocyclyl or 3- to 12-membered heterocyclyl, and the heterocyclyl contains 1 to 3

heteroatoms selected from O, S or N;

each $R^{c1}$ is independently selected from H, halogen, cyano, OH, =O, $C_{1-6}$ alkyl, $C_{1-6}$ alkoxy, $C_{3-12}$ carbocyclyl or 3- to 12-membered heterocyclyl, and the alkyl, alkoxy, carbocyclyl or heterocyclyl is optionally further substituted with 0 to 4 substituents selected from H, halogen, OH, =O, cyano, $NH_2$, $C_{1-6}$ alkyl, halogen-substituted-$C_{1-6}$ alkyl, hydroxyl-substituted-$C_{1-6}$ alkyl, cyano-substituted-$C_{1-6}$ alkyl, $C_{1-6}$ alkoxy or $C_{3-6}$ cycloalky, the heterocyclyl contains 1 to 3 heteroatoms selected from O, S or N;

alternatively, $R^{cn1}$ and $R^{cn2}$ together with the atom to which they are attached form 3- to 12-membered heterocyclyl, and the heterocyclyl is optionally further substituted with 0 to 4 substituents selected from H, halogen, OH, =O, cyano, $NH_2$, $C_{1-6}$ alkyl, halogen-substituted-$C_{1-6}$ alkyl, hydroxyl-substituted-$C_{1-6}$ alkyl, cyano-substituted-$C_{1-6}$ alkyl, $C_{1-6}$ alkoxy or $C_{3-6}$ cycloalkyl, the heterocyclyl contains 1 to 3 heteroatoms selected from O, S or N;

m is selected from 0, 1, 2 or 3;

n is selected from 0, 1, 2 or 3;

s is selected from 0, 1, 2 or 3;

t is selected from 0, 1, 2, 3 or 4.

As a ninth embodiment of the present invention, provided is the aforementioned compound of general formula (I) or (III) or a stereoisomer, a tautomer, a deuterate, a solvate, a prodrug, a metabolite, a pharmaceutically acceptable salt or a co-crystal thereof, wherein

$R^{an}$ is selected from H, $C_{1-4}$ alkyl, $C_{3-6}$ cycloalkyl, or 3- to 6-membered heterocycloalkyl, the alkyl, cycloalkyl or heterocycloalkyl is optionally further substituted with 0 to 4 substituents selected from H, halogen, OH, =O, cyano, $NH_2$, $C_{1-4}$ alkyl, $C_{2-4}$ alkenyl, $C_{2-4}$ alkynyl, -$C_{2-4}$ alkynyl-$C_{3-6}$ cycloalkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy, $C_{3-6}$ cycloalkyl, phenyl, 3- to 6-membered heterocycloalkyl or 5- to 6-membered heteroaryl, and the heterocycloalkyl or heteroaryl contains 1 to 3 heteroatoms selected from O, S or N;

$R^a$ or $R^b$ is each independently selected from H, halogen, cyano, OH, =O, $C_{1-4}$ alkyl, $C_{1-4}$ alkoxy, or $C_{3-6}$ cycloalkyl, and the alkyl, alkoxy or cycloalkyl is optionally further substituted with 0 to 4 substituents selected from H, halogen, OH, =O, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy or $C_{3-6}$ cycloalkyl;

$R^1$ is selected from H, $C_{1-4}$ alkyl or $C_{3-6}$ cycloalkyl, the alkyl or cycloalkyl is optionally further substituted with 0 to 4 substituents selected from H, halogen, OH, =O, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy or $C_{3-6}$ cycloalkyl;

$R^{2a}$, $R^{2b}$, $R^{3a}$ and $R^{3b}$ are each independently selected from H, halogen, cyano, OH, =O, $C_{1-4}$ alkyl, $C_{1-4}$ alkoxy or $C_{3-6}$ cycloalkyl, and the alkyl, alkoxy or cycloalkyl is optionally further substituted with 0 to 4 substituents selected from H, halogen, OH, =O, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy or $C_{3-6}$ cycloalkyl;

m is selected from 0, 1 or 2;

n is selected from 0, 1 or 2;

the definitions of the others are the same as those in the eighth embodiment of the present invention.

As a tenth embodiment of the present invention, provided is the aforementioned compound of general formula (I) or (III) or a stereoisomer, a tautomer, a deuterate, a solvate, a prodrug, a metabolite, a pharmaceutically acceptable salt or a co-crystal thereof, wherein

L is selected from

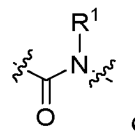

or 5-membered heteroaryl, L is connected with ring A at the left side, the heteroaryl is optionally further substituted with 0 to 2 substituents selected from H, halogen, OH, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, or $C_{1-4}$ alkoxy, and the heteroaryl contains 1 to 3 heteroatoms selected from O, S or N;

$R^1$ is selected from H, methyl, ethyl, propyl, isopropyl or cyclopropyl, and the methyl, ethyl, propyl, isopropyl or cyclopropyl is optionally further substituted with 0 to 4 substituents selected from H, F, Cl, Br, I, OH, =O, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy or

$C_{3-6}$ cycloalkyl;

$R^{cn1}$ and $R^{cn2}$ are each independently selected from H, halogen, cyano, OH, =O, $C_{1-4}$ alkyl, $C_{1-4}$ alkoxy, $C_{3-7}$monocycloalkyl, $C_{4-10}$fused cycloalkyl, $C_{5-10}$-membered spirocycloalkyl, $C_{5-10}$bridged cycloalkyl, 4- to 7-membered monoheterocycloalkyl, 4- to 10-membered fused heterocycloalkyl, 5- to 10-membered spiroheterocycloalkyl, 5- to 10-membered bridged heterocycloalkyl or

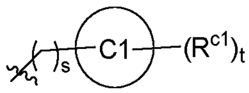

,

the alkyl, alkoxy, cycloalkyl or heterocycloalkyl is optionally further substituted with 0 to 4 substituents selected from H, halogen, OH, =O, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy or $C_{3-6}$ cycloalkyl, and the heterocycloalkyl contains 1 to 3 heteroatoms selected from O, S or N;

ring C1 is selected from $C_{3-7}$monocycloalkyl, $C_{4-11}$fused cycloalkyl, $C_{5-11}$ spirocycloalkyl, $C_{5-12}$bridged cycloalkyl, 4- to 7-membered monoheterocycloalkyl, 4- to 11-membered fused heterocycloalkyl, 5- to 11-membered spiroheterocycloalkyl or 5- to 12-membered bridged heterocycloalkyl, and the heterocycloalkyl contains 1 to 3 heteroatoms selected from O, S or N;

each $R^{c1}$ is independently selected from H, halogen, cyano, OH, =O, $C_{1-4}$ alkyl, $C_{1-4}$ alkoxy, $C_{3-6}$ cycloalkyl or 3- to 6-membered heterocycloalkyl, the alkyl, alkoxy, cycloalkyl or heterocycloalkyl is optionally further substituted with 0 to 4 substituents selected from H, halogen, OH, =O, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy or $C_{3-6}$ cycloalkyl, and the heterocycloalkyl contains 1 to 3 heteroatoms selected from O, S or N;

alternatively, $R^{cn1}$ and $R^{cn2}$ together with the atom to which they are attached form 4- to 7-membered monoheterocycloalkyl, 4- to 11-membered fused heterocycloalkyl, 5- to 11-membered spiroheterocycloalkyl or 5- to 12-membered bridged heterocycloalkyl, the heterocycloalkyl is optionally further substituted with 0 to 4 substituents selected from H, halogen, OH, =O, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy or $C_{3-6}$ cycloalkyl, and the heterocycloalkyl contains 1 to 3 heteroatoms selected from O, S or N;

p is selected from 0, 1, or 2;

t is selected from 0, 1, 2, 3 or 4;

the remaining definitions are the same as those in the eighth or ninth embodiment of the present invention.

As an eleventh embodiment of the present invention, provided is the aforementioned compound of general formula (I) or (III) or a stereoisomer, a tautomer, a deuterate, a solvate, a prodrug, a metabolite, a pharmaceutically acceptable salt or a co-crystal thereof, wherein

$R^{an}$ is selected from H, methyl, ethyl, propyl, isopropyl, cyclopropyl, cyclohexyl, cyclopentyl, cyclobutyl, oxetanyl, oxacyclopentyl, oxacyclohexyl, azetidinyl, azacyclopentyl, piperidyl or piperazinyl, the methyl, ethyl, propyl, isopropyl, cyclopropyl, cyclohexyl, cyclopentyl, cyclobutyl, oxetanyl, oxacyclopentyl, oxacyclohexyl, azetidinyl, azacyclopentyl, piperidyl or piperazinyl is optionally further substituted with 0 to 4 substituents selected from H, F, Cl, Br, I, OH, =O, cyano, $NH_2$, $C_{1-4}$ alkyl, $C_{2-4}$ alkenyl, $C_{2-4}$ alkynyl, -$C_{2-4}$ alkynyl-$C_{3-6}$ cycloalkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy, $C_{3-6}$ cycloalkyl, phenyl, 3- to 6-membered heterocycloalkyl or 5- to 6-membered heteroaryl, and the heterocycloalkyl or heteroaryl contains 1 to 3 heteroatoms selected from O, S or N;

$R^a$ or $R^b$ is each independently selected from H, F, Cl, Br, I, cyano, OH, =O, methyl, ethyl, propyl, isopropyl, cyclopropyl, cyclohexyl, cyclopentyl, cyclobutyl, azetidinyl, azacyclopentyl, azacyclohexyl, piperidyl, methoxy or ethoxy, and the methyl, ethyl, propyl, isopropyl, cyclopropyl, cyclohexyl, cyclopentyl, cyclobutyl, azetidinyl, azacyclopentyl, azacyclohexyl, piperidyl, methoxy or ethoxy is optionally further substituted with 0 to 4 substituents selected from H, F, Cl, Br, I, OH, =O, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy or $C_{3-6}$ cycloalkyl;

L is selected from

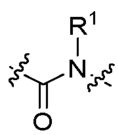

,

oxazolyl, thiazolyl, thienyl, furyl, pyrrolyl, pyrazolyl, triazolyl or imidazolyl, L is connected with ring A at the left side, and the oxazolyl, thiazolyl, thienyl, furyl, pyrrolyl, pyrazolyl, triazolyl or imidazolyl is optionally further substituted with 0 to 2 substituents selected from H, F, Cl, Br, I, OH, cyano, $NH_2$, methyl, ethyl, propyl, isopropyl, methoxy, or ethoxy;
$R^1$ is selected from H, methyl, ethyl, propyl, or isopropyl;
$R^{2a}$, $R^{2b}$, $R^{3a}$ and $R^{3b}$ are each independently selected from H, F, Cl, Br, I, cyano, OH, =O, methyl, ethyl, propyl, isopropyl, methoxy, ethoxy or cyclopropyl, and the methyl, ethyl, propyl, isopropyl, methoxy, ethoxy or cyclopropyl is optionally further substituted with 0 to 4 substituents selected from H, F, Cl, Br, I, OH, =O, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy or $C_{3-6}$ cycloalkyl;
$R^{cn1}$ and $R^{cn2}$ are each independently selected from H, F, Cl, Br, I, cyano, OH, =O, methyl, ethyl, propyl, isopropyl, cyclopropyl, cyclohexyl, cyclopentyl, cyclobutyl, azetidinyl, azacyclopentyl, azacyclohexyl or

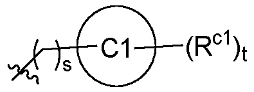
,

and the methyl, ethyl, propyl, isopropyl, cyclopropyl, cyclohexyl, cyclopentyl, cyclobutyl, azetidinyl, azacyclopentyl, or azacyclohexyl is optionally further substituted with 0 to 4 substituents selected from H, F, Cl, Br, I, OH, =O, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy or $C_{3-6}$ cycloalkyl;
ring C1 is selected from cyclopropyl, cyclobutyl, cyclopentyl, cyclohexyl, oxetanyl, oxacyclopentyl, oxacyclohexyl, azetidinyl, azacyclopentyl, piperidine, morpholine, piperazine, 1,4-diazepanyl, cyclopropyl fused cyclopentyl, cyclopentyl fused cyclobutyl, cyclopentyl fused cyclopentyl, cyclopentyl fused cyclohexyl, cyclopropyl spirocyclopentyl, cyclobutyl spirocyclobutyl, cyclobutyl spirocyclopentyl, cyclopentyl spirocyclopentyl, cyclopentyl spirocyclohexyl, cyclohexyl spirocyclohexyl, cyclopropyl fused azetidinyl, cyclopropyl fused azacyclopentyl, cyclopropyl fused azacyclohexyl, cyclobutyl fused azetidinyl, cyclobutyl fused azacyclopentyl, cyclobutyl fused azacyclohexyl, cyclopentyl fused azetidinyl, cyclopentyl fused azacyclopentyl, cyclopentyl fused azacyclohexyl, cyclohexyl fused azetidinyl, cyclohexyl fused azacyclopentyl, cyclohexyl fused azacyclohexyl, azetidinyl fused azetidinyl, azetidinyl fused azacyclopentyl, azetidinyl fused azacyclohexyl, azacyclopentyl fused azetidinyl, azacyclopentyl fused azacyclopentyl, azacyclopentyl fused azacyclohexyl, azacyclohexyl fused azetidinyl, azacyclohexyl fused azacyclopentyl, azacyclohexyl fused azacyclohexyl, cyclobutyl spiroazetidinyl, cyclobutyl spiroazacyclopentyl, cyclobutyl spiroazacyclohexyl, cyclopentyl spiroazetidinyl, cyclopentyl spiroazacyclopentyl, cyclopentyl spiroazacyclohexyl, cyclohexyl spiroazetidinyl, cyclohexyl spiroazacyclopentyl, cyclohexyl spiroazacyclohexyl, azetidinyl spiroazetidinyl, azetidinyl spiroazacyclopentyl, azetidinyl spiroazacyclohexyl, azacyclopentyl spiroazetidinyl, azacyclopentyl spiroazacyclopentyl, azacyclopentyl spiroazacyclohexyl, azacyclohexyl spiroazetidinyl, azacyclohexyl spiroazacyclopentyl, azacyclohexyl spiroazacyclohexyl, oxetanyl, oxacyclopentyl, oxacyclohexyl, cyclopropyl fusedoxetanyl, cyclopropyl fusedoxacyclopentyl, cyclopropyl fusedoxacyclohexyl, cyclobutyl fusedoxetanyl, cyclobutyl fusedoxacyclopentyl, cyclobutyl fusedoxacyclohexyl, cyclopentyl fusedoxetanyl, cyclopentyl fusedoxacyclopentyl, cyclopentyl fusedoxacyclohexyl, cyclohexyl fusedoxetanyl, cyclohexyl fusedoxacyclopentyl, cyclohexyl fusedoxacyclohexyl, azetidinyl fusedoxetanyl, azetidinyl fusedoxacyclopentyl, azetidinyl fusedoxacyclohexyl, azacyclopentyl fusedoxetanyl, azacyclopentyl fusedoxacyclopentyl, azacyclopentyl fusedoxacyclohexyl, azacyclohexyl fusedoxetanyl, azacyclohexyl fusedoxacyclopentyl, azacyclohexyl fusedoxacyclohexyl, cyclobutyl spirooxetanyl, cyclobutyl spirooxacyclopentyl, cyclobutyl spirooxacyclohexyl, cyclopentyl spirooxetanyl, cyclopentyl spirooxacyclopentyl, cyclopentyl spirooxacyclohexyl, cyclohexyl spirooxetanyl, cyclohexyl spirooxacyclopentyl, cyclohexyl spirooxacyclohexyl, azetidinyl spirooxetanyl, azetidinyl spirooxacyclopentyl, azetidinyl spirooxacyclohexyl, azacyclopentyl spirooxetanyl, azacyclopentyl spirooxacyclopentyl, azacyclopentyl spirooxacyclohexyl, azacyclohexyl spirooxetanyl, azacyclohexyl spirooxacyclopentyl, azacyclohexyl spirooxacyclohexyl, bicyclo[2.1.1]hexyl, bicyclo[2.2.1]heptyl, bicyclo[3.3.2]decanyl, bicyclo[2.2.2]octanyl, bicyclo[3.2.1]octanyl, bicyclo[3.3.3]undecyl, adamantyl,

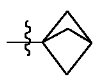
,
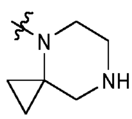
,

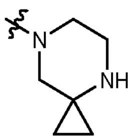, 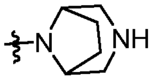, 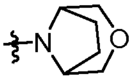, 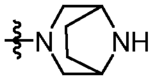, 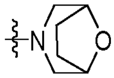, 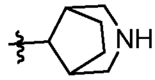, 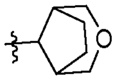,

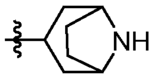, 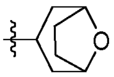, 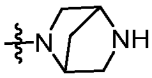, 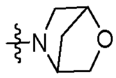, 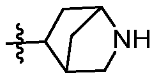, 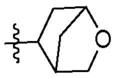,

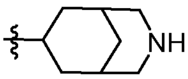, 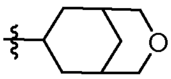, 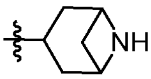, 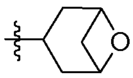, 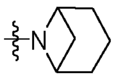, 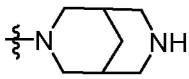,

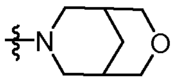, or 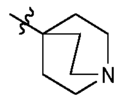;

each $R^{c1}$ is independently selected from H, F, Cl, Br, I, cyano, OH, =O, methyl, ethyl, propyl, isopropyl, cyclopropyl, cyclohexyl, cyclopentyl, cyclobutyl, azetidinyl, azacyclopentyl, piperidyl, piperazinyl, methoxy or ethoxy, and the methyl, ethyl, propyl, isopropyl, cyclopropyl, cyclohexyl, cyclopentyl, cyclobutyl, azetidinyl, azacyclopentyl, piperidyl, piperazinyl, methoxy or ethoxy is optionally further substituted with 0 to 4 substituents selected from H, F, Cl, Br, I, OH, =O, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy or $C_{3-6}$ cycloalkyl;

alternatively, $R^{cn1}$ and $R^{cn2}$ together with the atom to which they are attached form substituted or unsubstituted azetidinyl, azacyclopentyl, piperidine, piperazine, azetidinyl fused cyclopropyl, azetidinyl fused cyclobutyl, azetidinyl fused cyclopentyl, azetidinyl fused cyclohexyl, azacyclopentyl fused cyclopropyl, azacyclopentyl fused cyclobutyl, azacyclopentyl fused cyclopentyl, azacyclopentyl fused cyclohexyl, azacyclohexyl fused cyclopropyl, azacyclohexyl fused cyclobutyl, azacyclohexyl fused cyclopentyl, azacyclohexyl fused cyclohexyl, azetidinyl fused azetidinyl, azetidinyl fused azacyclopentyl, azetidinyl fused azacyclohexyl, azacyclopentyl fused azetidinyl, azacyclopentyl fuse dazacyclopentyl, azacyclopentyl fused azacyclohexyl, azacyclohexyl fused azetidinyl, azacyclohexyl fused azacyclopentyl, azacyclohexyl fused azacyclohexyl, azetidinyl spiroazetidinyl, azetidinyl spiroazacyclopentyl, azetidinyl spiroazacyclohexyl, azacyclopentyl spiroazetidinyl, azacyclopentyl spiroazacyclopentyl, azacyclopentyl spiroazacyclohexyl, azacyclohexyl spiroazetidinyl, azacyclohexyl spiroazacyclopentyl, azacyclohexyl spiroazacyclohexyl, azacyclopentyl spirocyclobutyl, azacyclopentyl spirocyclopentyl, azacyclohexyl spirocyclobutyl, azacyclohexyl spirocyclopentyl, azacyclohexyl spirocyclohexyl,

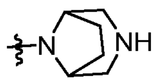,

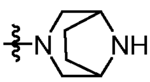, 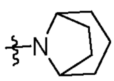, 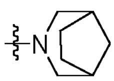, 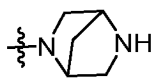, 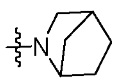, 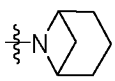, 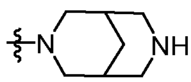,

or

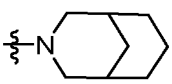,

which, when substituted, is optionally further substituted with 0 to 4 substituents selected from H, halogen, OH, =O, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy or $C_{3-6}$ cycloalkyl;

the remaining definitions are the same as those of the eighth, ninth, or tenth embodiment of the present invention.

As a twelfth embodiment of the present invention, provided is the aforementioned compound of general formula

(I) or (III) or a stereoisomer, a tautomer, a deuterate, a solvate, a prodrug, a metabolite, a pharmaceutically acceptable salt or a co-crystal thereof, wherein

$R^{an}$ is selected from H, methyl, ethyl, propyl, isopropyl, or cyclopropyl, and the methyl, ethyl, propyl, isopropyl, or cyclopropyl is optionally further substituted with 0 to 4 substituents selected from H, F, Cl, Br, I, OH, =O, cyano, $NH_2$, methyl, ethyl, propyl, isopropyl, ethenyl, 1-propenyl, 2-propenyl, ethynyl, 1-propynyl, 2-propynyl,

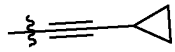,

cyclopropyl, methoxy, ethoxy, or phenyl;

$R^a$ or $R^b$ is each independently selected from H, F, Cl, Br, I, cyano, OH, =O, methyl, ethyl, propyl, isopropyl, or cyclopropyl, and the methyl, ethyl, propyl, isopropyl or cyclopropyl is optionally further substituted with 0 to 4 substituents selected from H, F, Cl, Br, I, OH, =O, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy or $C_{3-6}$ cycloalkyl;

$R^{cn1}$ and $R^{cn2}$ are each independently selected from H, F, Cl, Br, I, cyano, OH, =O, methyl, ethyl, propyl, isopropyl, cyclopropyl, cyclohexyl, cyclopentyl, cyclobutyl or

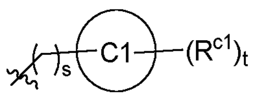,

and the methyl, ethyl, propyl, isopropyl, cyclopropyl, cyclohexyl, cyclopentyl, or cyclobutyl is optionally further substituted with 0 to 4 substituents selected from H, F, Cl, Br, I, OH, =O, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy or $C_{3-6}$ cycloalkyl;

ring C1 is selected from cyclopropyl, cyclobutyl, cyclopentyl, cyclohexyl, oxetanyl, oxacyclopentyl, oxacyclohexyl, azetidinyl, azacyclopentyl, piperidine, morpholine, piperazine, 1,4-diazepanyl, cyclopropyl fused cyclopentyl, cyclopentyl fused cyclobutyl, cyclopentyl fused cyclopentyl, cyclopentyl fused cyclohexyl,

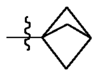,

bicyclo[2.1.1]hexyl, bicyclo[2.2.1]heptyl, bicyclo[3.3.2]decanyl, bicyclo[2.2.2]octanyl, bicyclo[3.2.1]octanyl, bicyclo [3.3.3]undecyl, or adamantyl;

each $R^{c1}$ is independently selected from H, F, Cl, Br, I, cyano, OH, =O, methyl, ethyl, propyl, isopropyl, cyclopropyl, methoxy or ethoxy, and the methyl, ethyl, propyl, isopropyl, cyclopropyl, methoxy or ethoxy is optionally further substituted with 0 to 4 substituents selected from H, F, Cl, Br, I, OH, =O, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy or $C_{3-6}$ cycloalkyl;

alternatively, $R^{cn1}$ and $R^{cn2}$ together with the atom to which they are attached form substituted or unsubstituted azetidinyl, azacyclopentyl, piperidine, piperazine, azacyclopentyl spirocyclobutyl, azacyclopentyl spirocyclopentyl, azacyclohexyl spirocyclobutyl, azacyclohexyl spirocyclopentyl, or azacyclohexyl spirocyclohexyl, which, when substituted, is optionally further substituted with 0 to 4 substituents selected from H, F, Cl, Br, I, OH, =O, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy or $C_{3-6}$ cycloalkyl;

the remaining definitions are the same as those of the eighth, ninth, tenth or eleventh embodiment of the present invention.

As a thirteenth embodiment of the present invention, provided is the aforementioned compound of general formula (I) or (III) or a stereoisomer, tautomer, deuterate, solvate, prodrug, metabolite, pharmaceutically acceptable salt or co-crystal thereof, wherein

ring A is selected from

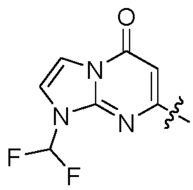, 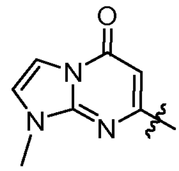, 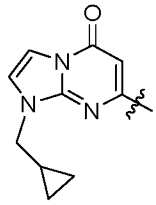,

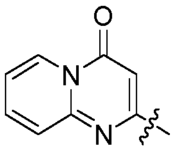, 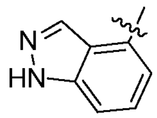, 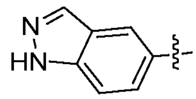, 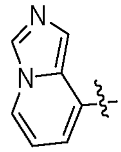, 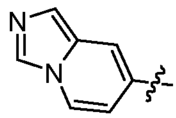,

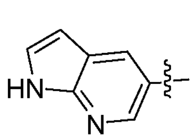, or 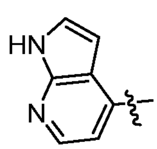;

L is selected from

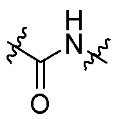, 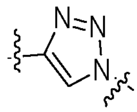, 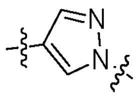, or 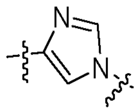,

and is connected with ring A at the left side;

$R^a$ or $R^b$ is each independently selected from H, F, Cl, Br, I, cyano, OH, =O, $CF_3$, methyl, ethyl, propyl, isopropyl, or cyclopropyl;

$R^{2a}$, $R^{2b}$, $R^{3a}$ and $R^{3b}$ are each independently selected from H;

$R^{cn1}$ and $R^{cn2}$ are each independently selected from H, methyl, ethyl, propyl, isopropyl, cyclopropyl, cyclohexyl, cyclopentyl, cyclobutyl or

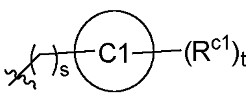,

and the methyl, ethyl, propyl, isopropyl, cyclopropyl, cyclohexyl, cyclopentyl, or cyclobutyl is optionally further substituted with 0 to 4 substituents selected from H, F, Cl, Br, I, OH, =O, cyano, $NH_2$, $CF_3$, methyl, ethyl, propyl, isopropyl, cyclopropyl, methoxy or ethoxy;

ring C1 is selected from cyclopropyl, cyclobutyl, cyclopentyl, cyclohexyl, cyclopentyl fused cyclobutyl or

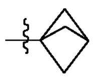;

each $R^{c1}$ is independently selected from H, F, or Cl;

alternatively, $R^{cn1}$ and $R^{cn2}$ together with the atom to which they are attached form substituted or unsubstituted azetidinyl, azacyclopentyl, piperidine, piperazine, azacyclopentyl spirocyclobutyl, azacyclopentyl spirocyclopentyl, azacyclohexyl spirocyclobutyl, azacyclohexyl spirocyclopentyl, or azacyclohexyl spirocyclohexyl, which, when substituted, is optionally further substituted with 0 to 4 substituents selected from H, F, Cl, Br, I, OH, =O, cyano, $NH_2$, $CF_3$, methyl, ethyl, propyl, isopropyl, cyclopropyl, methoxy or ethoxy;

m is selected from 0 or 1;

n is selected from 0 or 1;

s is selected from 0 or 1;

t is selected from 0, 1 or 2;

the remaining definitions are the same as those of the eighth, ninth, tenth, eleventh, or twelfth embodiment of the present invention.

The present invention relates to a compound as described below or a stereoisomer, tautomer, deuterate, solvate, prodrug, metabolite, pharmaceutically acceptable salt or co-crystal thereof, wherein the compound is selected from one of the structures shown in Table S-1 or Table S-2 below:

Table S-1

| | |
|---|---|
| 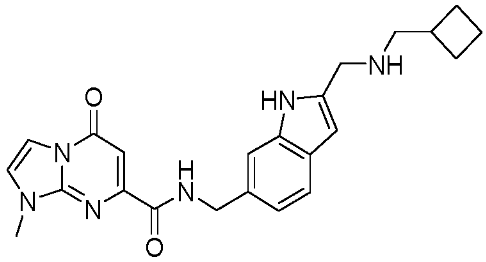 | 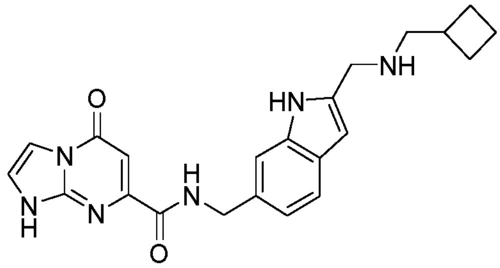 |
| 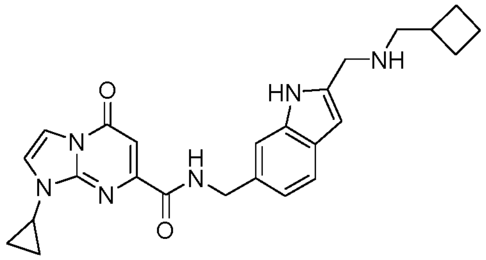 | 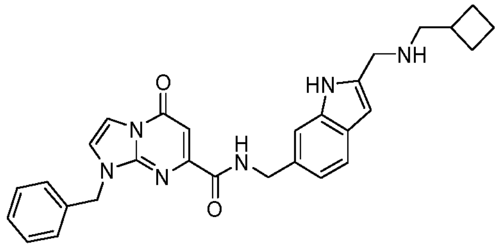 |
| 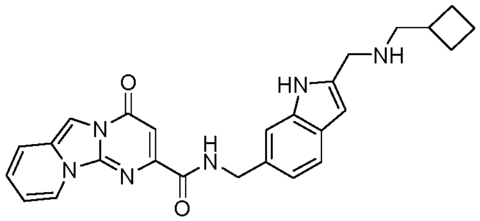 | 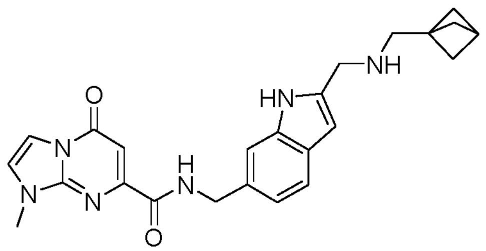 |
| 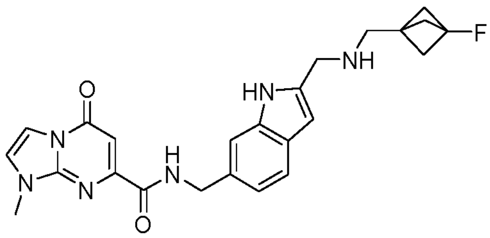 | 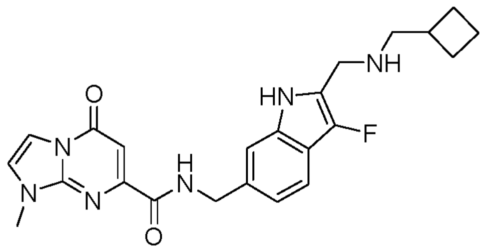 |
| 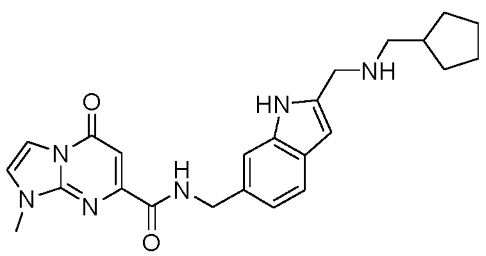 | 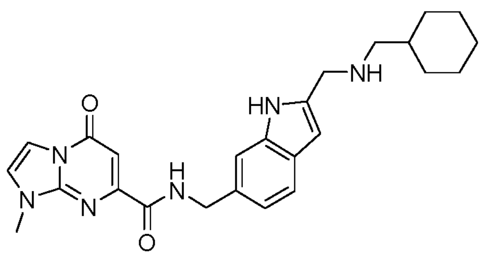 |
| 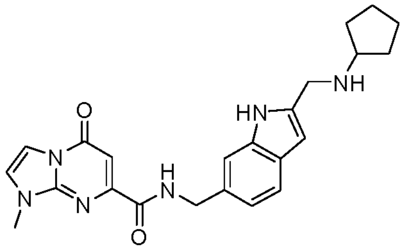 | 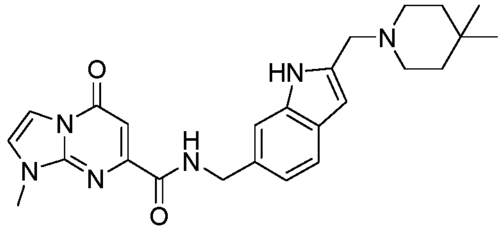 |

| | |
|---|---|
| 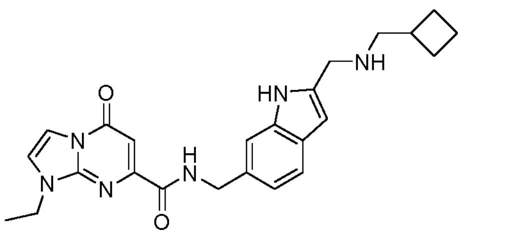 | 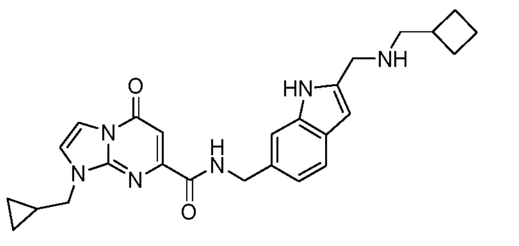 |
| 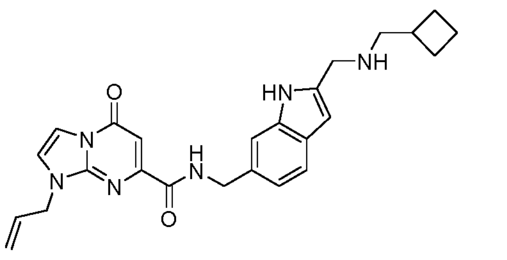 | 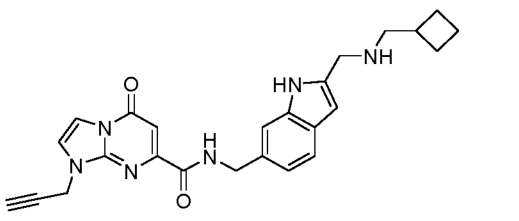 |
| 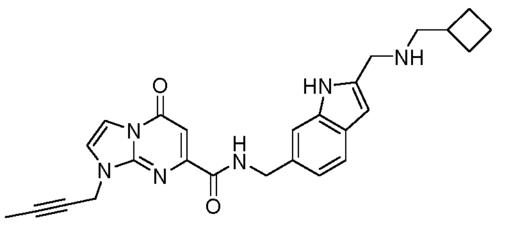 | 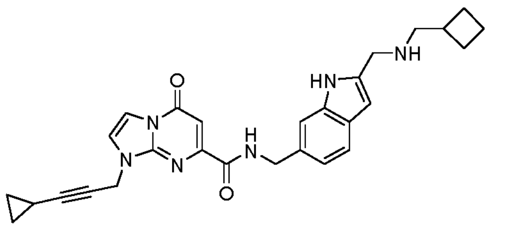 |
| 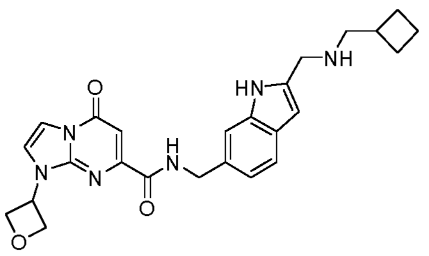 | 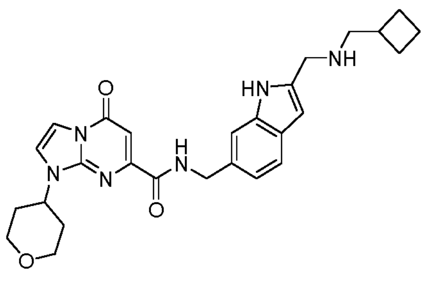 |
| 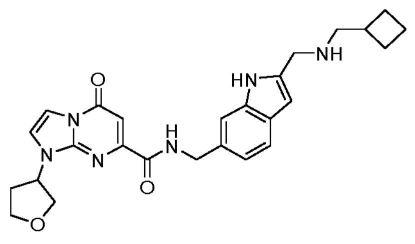 | 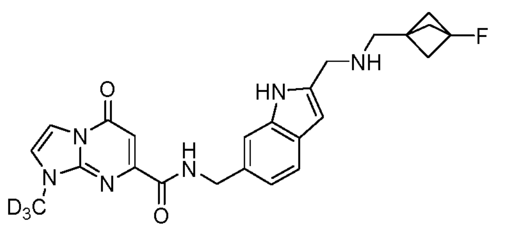 |
| 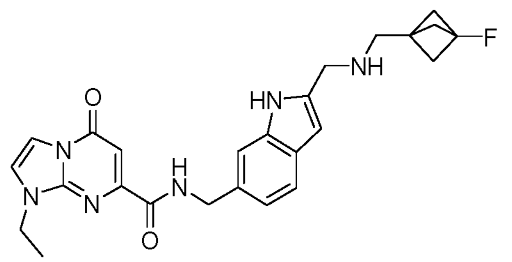 | 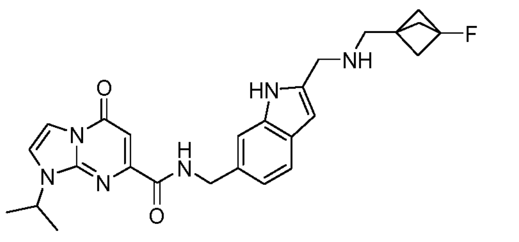 |

| | |
|---|---|
| 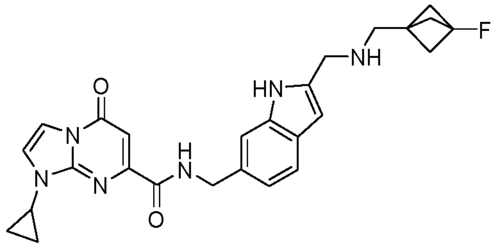 | 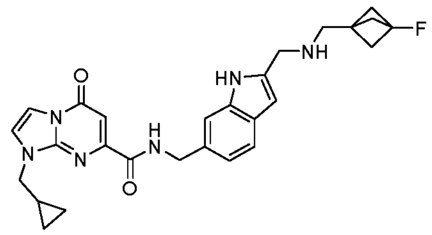 |
| 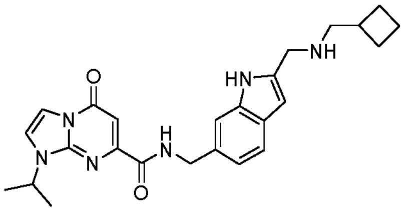 | 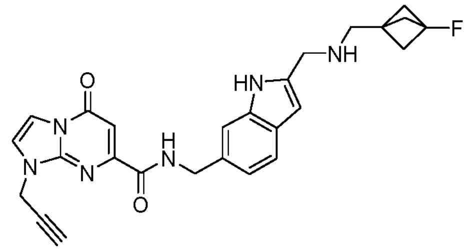 |
| 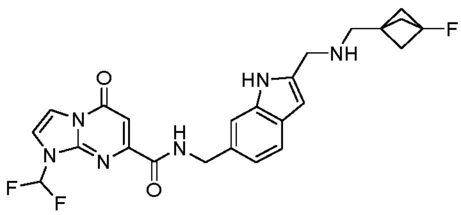 | 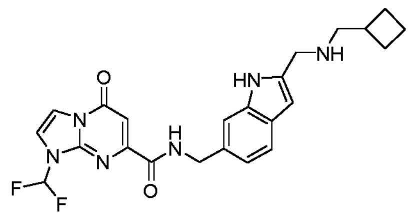 |
| 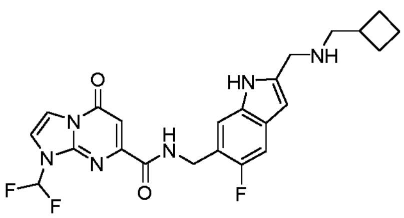 | 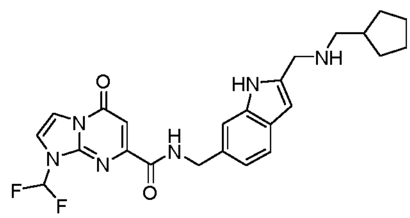 |
| 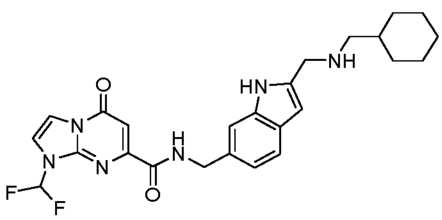 | 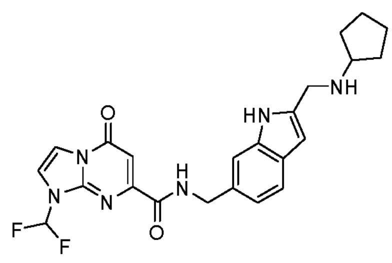 |
| 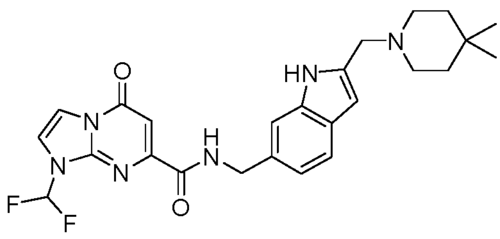 | 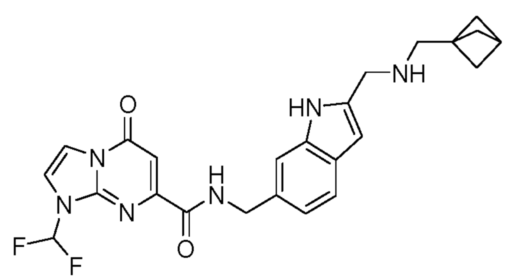 |

,

Table S-2

| | |
|---|---|
| 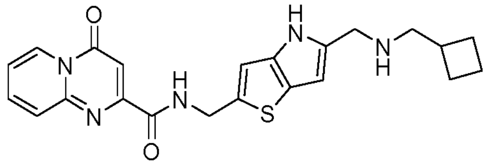 | 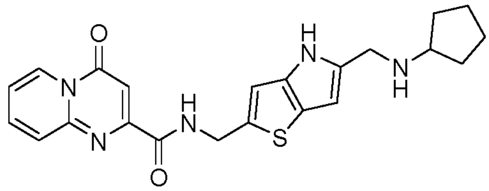 |
| 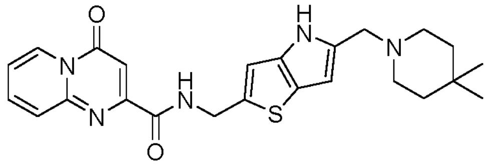 | 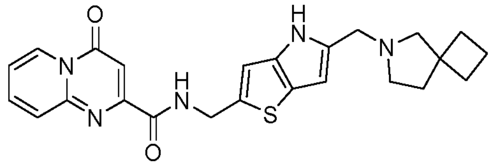 |
| 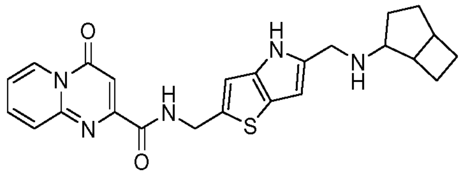 | 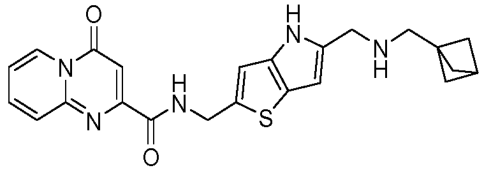 |
| 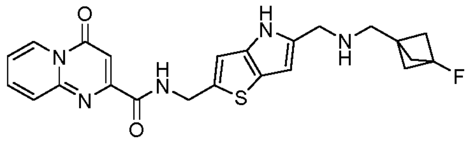 | 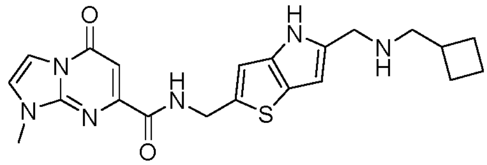 |
| 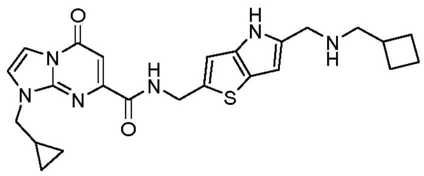 | 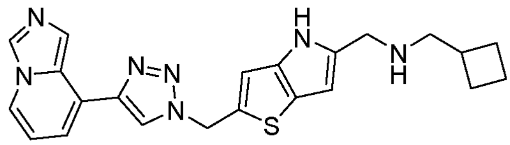 |
| 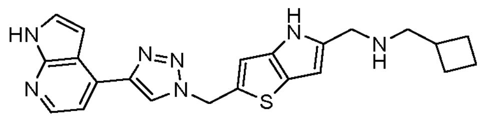 | 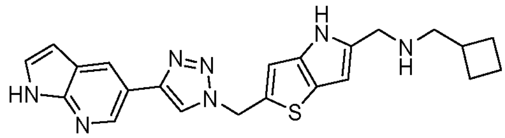 |
| 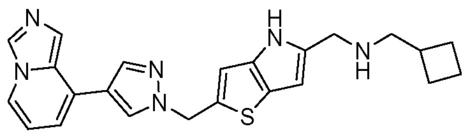 | 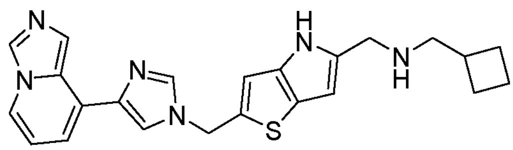 |
| 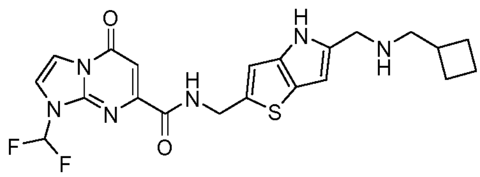 | 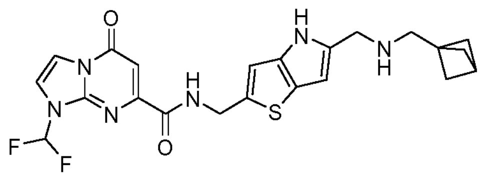 |
| 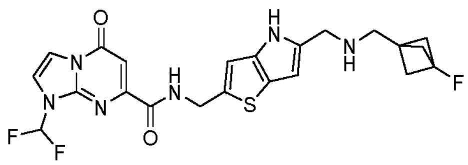 | 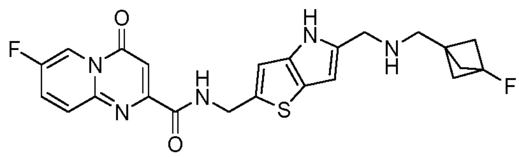 |

| | |
|---|---|
| 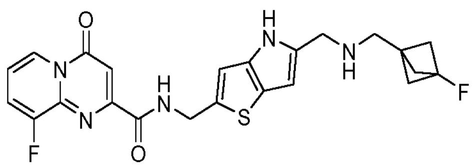 | 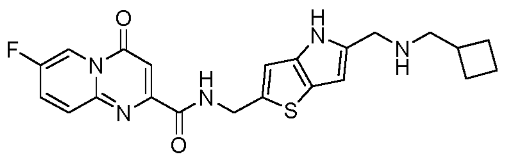 |
| 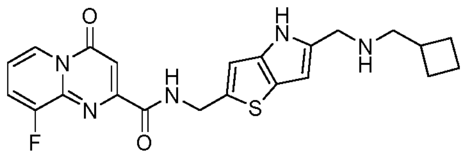 | 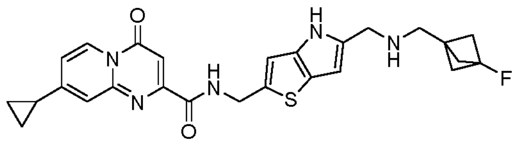 |
| 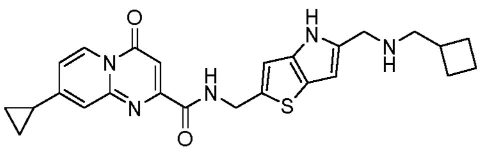 | 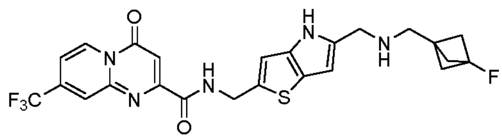 |
| 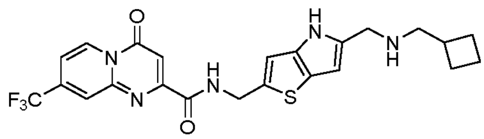 | |

The present invention provides a pharmaceutical composition comprising the aforementioned compound or a stereoisomer, tautomer, deuterate, solvate, prodrug, metabolite, pharmaceutically acceptable salt or co-crystal thereof, and a pharmaceutically acceptable carrier.

The present invention relates to a pharmaceutical composition, comprising a therapeutically effective amount of the above-mentioned compound or the stereoisomer, deuterate, solvate, prodrug, metabolite, pharmaceutically acceptable salt or co-crystal thereof according to the present invention, and a pharmaceutically acceptable carrier.

In some embodiments, the pharmaceutical composition of the present invention can be in a unit preparation form (the amount of the drug substance in the unit preparation is also referred to as the "preparation strength").

The term "effective amount" or "therapeutically effective amount" according to the present application refers to a sufficient amount of the compound disclosed in the present application that is administered to ameliorate, to some extent, one or more symptoms of a disease or condition (such as an METTL3 enzyme-related disease, such as cancer) being treated. In some embodiments, the outcome is the reduction and/or remission of signs, symptoms or causes of the disease, or any other desired change in the biological system. For example, an "effective amount" in terms of the therapeutic use is an amount of the composition comprising the compound disclosed in the present application that is required to provide clinically significant reduction of the symptoms of the disease. Examples of the therapeutically effective amount include, but are not limited to 1-1500 mg, 1-1400 mg, 1-1200 mg, 1-1000 mg, 1-900 mg, 1-800 mg, 1-700 mg, 1-600 mg, 2-600 mg, 3-600 mg, 4-600 mg, 5-600 mg, 6-600 mg, 10-600 mg, 20-600 mg, 25-600 mg, 30-600 mg, 40-600 mg, 50-600 mg, 60-600 mg, 70-600 mg, 75-600 mg, 80-600 mg, 90-600 mg, 100-600 mg, 200-600 mg, 1-500 mg, 2-500 mg, 3-500 mg, 4-500 mg, 5-500 mg, 6-500 mg, 10-500 mg, 20-500 mg, 25-500 mg, 30-500 mg, 40-500 mg, 50-500 mg, 60-500 mg, 70-500 mg, 75-500 mg, 80-500 mg, 90-500 mg, 100-500 mg, 125-500 mg, 150-500 mg, 200-500 mg, 250-500 mg, 300-500 mg, 400-500 mg, 5-400 mg, 10-400 mg, 20-400 mg, 25-400 mg, 30-400 mg, 40-400 mg, 50-400 mg, 60-400 mg, 70-400 mg, 75-400 mg, 80-400 mg, 90-400 mg, 100-400 mg, 125-400 mg, 150-400 mg, 200-400 mg, 250-400 mg, 300-400 mg, 1-300 mg, 2-300 mg, 5-300 mg, 10-300 mg, 20-300 mg, 25-300 mg, 30-300 mg, 40-300 mg, 50-300 mg, 60-300 mg, 70-300 mg, 75-300 mg, 80-300 mg, 90-300 mg, 100-300 mg, 125-300 mg, 150-300 mg, 200-300 mg, 250-300 mg, 1-200 mg, 2-200 mg, 5-200 mg, 10-200 mg, 20-200 mg, 25-200 mg, 30-200 mg, 40-200 mg, 50-200 mg, 60-200 mg, 70-200 mg, 75-200 mg, 80-200 mg, 90-200 mg, 100-200 mg, 125-200 mg, 150-200 mg, 80-1000 mg, or 80-800 mg.

In some embodiments, the pharmaceutical composition comprises the compound, or the stereoisomer, deuterate, solvate, prodrug, metabolite, pharmaceutically acceptable salt or co-crystal thereof according to the present invention in an amount including but not limited to 1-1500 mg, 1-1400 mg, 1-1200 mg, 1-1000 mg, 20-800 mg, 40-800 mg, 40-400 mg, 25-200 mg, 1 mg, 5 mg, 10 mg, 15 mg, 20 mg, 25 mg, 30 mg, 35 mg, 40 mg, 45 mg, 50 mg, 55 mg, 65 mg, 70 mg, 75 mg, 80 mg, 85 mg, 90 mg, 95 mg, 100 mg, 110 mg, 120 mg, 125 mg, 130 mg, 140 mg, 150 mg, 160 mg, 170 mg, 180 mg, 190 mg, 200 mg, 210 mg, 220 mg, 230 mg, 240 mg, 250 mg, 300 mg, 320 mg, 400 mg, 480 mg, 500 mg, 600 mg, 640 mg, and 840 mg.

The present invention further provides a method for treating a disease in a mammal, the method comprising administering to a subject a therapeutically effective amount of the compound, or the stereoisomer, deuterate, solvate, prodrug, metabolite, pharmaceutically acceptable salt, or co-crystal thereof according to the present invention, wherein the therapeutically effective amount is preferably 1-1500 mg, and the disease is such as an METTL3 enzyme-related disease, such as cancer.

The present invention further provides a method for treating a disease in a mammal, the method comprising administering to a subject a drug, i.e., the compound, or the stereoisomer, deuterate, solvate, prodrug, metabolite, pharmaceutically acceptable salt, or co-crystal thereof according to the present invention in a daily dose of 1-1500 mg/day, wherein the daily dose can be a single dose or divided doses; in some embodiments, the daily dose includes, but is not limited to 10-1500 mg/day, 10-1000 mg/day, 10-800 mg/day, 25-800 mg/day, 50-800 mg/day, 100-800 mg/day, 200-800 mg/day, 25-400 mg/day, 50-400 mg/day, 100-400 mg/day, or 200-400 mg/day; in some embodiments, the daily dose includes, but is not limited to 10 mg/day, 20 mg/day, 25 mg/day, 50 mg/day, 80 mg/day, 100 mg/day, 125 mg/day, 150 mg/day, 160 mg/day, 200 mg/day, 300 mg/day, 320 mg/day, 400 mg/day, 480 mg/day, 600 mg/day, 640 mg/day, 800 mg/day, 1000 mg/day, 1200 mg/day, or 1400 mg/day.

The present invention relates to a kit, wherein the kit can comprise a composition in the form of a single dose or multiple doses and comprises the compound or the stereoisomer, deuterate, solvate, prodrug, metabolite, pharmaceutically acceptable salt or co-crystal thereof according to the present invention, and the amount of the compound or the stereoisomer, deuterate, solvate, prodrug, metabolite, pharmaceutically acceptable salt or co-crystal thereof according to the present invention is identical to the amount of same in the above-mentioned pharmaceutical composition.

The present invention also provides the above-mentioned compound or a stereoisomer, tautomer, deuterate, solvate, prodrug, metabolite, pharmaceutically acceptable salt or co-crystal thereof, or the use of the above-mentioned pharmaceutical composition in the preparation of a drug for treating a disease related to the inhibition of METTL3 enzyme, wherein the disease is selected from cancer.

The amount of the compound or the stereoisomer, deuterate, solvate, prodrug, metabolite, pharmaceutically acceptable salt or co-crystal thereof according to the present invention is calculated in the form of a free base in each case.

Unless stated to the contrary, the terms used in the description and claims of the present application have the following meanings.

The carbon, hydrogen, oxygen, sulphur, nitrogen or F, Cl, Br, I involved in the groups and compounds of the present invention all comprise their isotopes, and the carbon, hydrogen, oxygen, sulphur or nitrogen involved in the groups and compounds of the present invention is optionally further substituted with one or more of their corresponding isotopes, wherein the isotopes of carbon comprise $^{12}C$, $^{13}C$ and $^{14}C$, the isotopes of hydrogen comprise protium (H), deuterium (D, also known as heavy hydrogen), tritium (T, also known as superheavy hydrogen), the isotopes of oxygen comprise $^{16}O$, $^{17}O$ and $^{18}O$, the isotopes of sulphur comprise $^{32}S$, $^{33}S$, $^{34}S$ and $^{36}S$, the isotopes of nitrogen comprise $^{14}N$ and $^{15}N$, the isotopes of fluorine comprise $^{17}F$ and $^{19}F$, the isotopes of chlorine comprise $^{35}Cl$ and $^{37}Cl$, and the isotopes of bromine comprise $^{79}Br$ and $^{81}Br$.

"Halogen" refers to F, Cl, Br or I.

"Halogen-substituted" refers to F, Cl, Br or I substitution, including but not limited to a substitution with 1 to 10 substituents selected from F, Cl, Br or I, a substitution with 1 to 6 substituents selected from F, Cl, Br or I, or a substitution with 1 to 4 substituents selected from F, Cl, Br or I. "Halogen-substituted" is referred to simply as "halo".

"Alkyl" refers to a substituted or unsubstituted linear or branched saturated aliphatic hydrocarbyl group, including but not limited to an alkyl group of 1 to 20 carbon atoms, an alkyl group of 1 to 8 carbon atoms, an alkyl group of 1 to 6 carbon atoms, or an alkyl group of 1 to 4 carbon atoms. Non-limiting examples include methyl, ethyl, n-propyl, isopropyl, n-butyl, sec-butyl, neobutyl, tert-butyl, n-pentyl, isoamyl, neopentyl, n-hexyl and various branched isomers thereof. The definition of the "alkyl" herein is consistent with this definition. Alkyl can be monovalent, divalent, trivalent or tetravalent.

"Alkylene" refers to a substituted or unsubstituted linear or branched divalent saturated hydrocarbyl group, including $-(CH_2)_v-$ (v is an integer from 1 to 10), and examples of alkylene include, but are not limited to, methylene, ethylene, propylene, butylene, *etc.*

"Cycloalkyl" refers to a substituted or unsubstituted saturated carbocyclic hydrocarbyl group, usually having from 3 to 10 carbon atoms, and non-limiting examples include cyclopropyl, cyclobutyl, cyclopentyl, cyclohexyl, cycloheptyl, etc. The cycloalkyl described herein is as defined above. Cycloalkyl can be monovalent, divalent, trivalent or tetravalent.

"Heterocycloalkyl" refers to a substituted or unsubstituted saturated heteroatom-containing cyclic hydrocarbyl group, including but not limited to 3 to 10 atoms, 3 to 8 atoms, or 1 to 3 heteroatoms selected from N, O or S. N and S selectively substituted in the heterocycloalkyl ring can be oxidised to various oxidation states. Heterocycloalkyl can be connected to a heteroatom or a carbon atom; heterocycloalkyl can be connected to an aromatic ring or a non-aromatic ring; and heterocycloalkyl can be connected to a bridged ring or a spiro ring. Non-limiting examples include oxiranyl, azacyclopropyl, oxacyclobutyl, azetidinyl, tetrahydrofuryl, tetrahydro-2H-pyranyl, dioxolanyl, dioxanyl, pyrrolidyl, piperidyl, imidazolidinyl, oxazolidinyl, oxazinanyl, morpholinyl, hexahydropyrimidyl or piperazinyl. Heterocycloalkyl group can be monovalent, divalent, trivalent or tetravalent.

"Alkenyl" refers to a substituted or unsubstituted linear or branched unsaturated hydrocarbyl group, having at least 1, usually 1, 2 or 3 carbon-carbon double bonds, with a main chain including but not limited to 2 to 10, 2 to 6, or 2 to 4 carbon atoms. Examples of alkenyl include, but are not limited to, ethenyl, allyl, 1-propenyl, 2-propenyl, 1-butenyl, 2-butenyl, 3-butenyl, 1-pentenyl, 2-pentenyl, 3-pentenyl, 4-pentenyl, 1-methyl-1-butenyl, 2-methyl-1-butenyl, 2-methyl-3-butenyl, 1-hexenyl, 2-hexenyl, 3-hexenyl, 4-hexenyl, 5-hexenyl, 1-methyl-1-pentenyl, 2-methyl-1-pentenyl, 1-heptenyl, 2-heptenyl, 3-heptenyl, 4-heptenyl, 1-octenyl, 3-octenyl, 1-nonenyl, 3-nonenyl, 1-decenyl, 4-decenyl, 1,3-butadiene, 1,3-pentadiene, 1,4-pentadiene, 1,4-hexadiene, etc. The definition of the alkenyl described herein is consistent with this definition. Alkenyl can be monovalent, divalent, trivalent or tetravalent.

"Alkynyl" refers to a substituted or unsubstituted linear or branched unsaturated hydrocarbyl group, having at least 1, usually 1, 2 or 3 carbon-carbon triple bonds, with a main chain including 2 to 10 carbon atoms, including but not limited to a main chain including 2 to 6 carbon atoms, or a main chain including 2 to 4 carbon atoms. Examples of alkynyl include, but are not limited to, ethynyl, propargyl, 1-propynyl, 2-propynyl, 1-butynyl, 2-butynyl, 3-butynyl, 1-pentynyl, 2-pentynyl, 3-pentynyl, 4-pentynyl, 1-methyl-1-butynyl, 2-methyl-1-butynyl, 2-methyl-3-butynyl, 1-hexynyl, 2-hexynyl, 3-hexynyl, 4-hexynyl, 5-hexynyl, 1-methyl-1-pentynyl, 2-methyl-1-pentynyl, 1-heptynyl, 2-heptynyl, 3-heptynyl, 4-heptynyl, 1-octynyl, 3-octynyl, 1-nonynyl, 3-nonynyl, 1-decynyl, 4-decynyl, *etc.* Alkynyl can be monovalent, divalent, trivalent or tetravalent.

"Alkoxy" refers to a substituted or unsubstituted -O-alkyl group. Non-limiting examples include methoxy, ethoxy, n-propoxy, isopropoxy, n-butoxy, sec-butoxy, tert-butoxy, n-pentoxy, n-hexyloxy, cyclopropoxy and cyclobutoxy.

"Carbocyclyl" or "carbocycle" refers to a substituted or unsubstituted saturated or unsaturated aromatic ring or non-aromatic ring, wherein the aromatic ring or non-aromatic ring can be a 3- to 8-membered monocyclic ring, a 4- to 12-membered bicyclic ring or a 10- to 15-membered tricyclic ring system. Carbocyclyl can be connected to an aromatic ring or a non-aromatic ring. The aromatic ring or non-aromatic ring is optionally a monocyclic ring, a bridged ring or a spiro ring. Non-limiting examples include cyclopropane, cyclobutane, cyclopentane, cyclohexane, cycloheptane, 1-cyclopentyl-1-enyl, 1-cyclopentyl-2-enyl, 1-cyclopentyl-3-enyl, cyclohexyl, 1-cyclohexyl-2-enyl, 1-cyclohexyl-3-enyl, cyclohexenyl, a benzene ring, a naphthalene ring, bicyclo[1.1.1]pentyl, bicyclo[2.1.1]hexyl, bicyclo[2.2.1]heptyl, bicyclo[3.3.2]decanyl, bicyclo[2.2.2]octanyl, bicyclo[3.2.1]octanyl, bicyclo[3.3.3]undecyl, adamantyl,

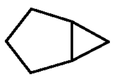, 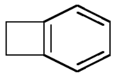,

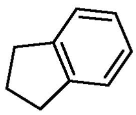 or 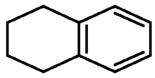

"Carbocyclyl" or "carbocycle" can be monovalent, divalent, trivalent or tetravalent.

"Heterocyclyl" or "heterocycle" refers to a substituted or unsubstituted saturated or unsaturated aromatic ring or non-aromatic ring, wherein the aromatic ring or non-aromatic ring can be 3- to 8-membered monocyclic ring, 4- to 12-membered bicyclic ring or 10- to 15-membered tricyclic ring system, and contains one or more (including but not limited to 2, 3, 4 or 5) heteroatoms selected from N, O or S, and the selectively substituted N and S in the heterocyclyl ring can be oxidised to various oxidation states. Heterocyclyl can be connected to a heteroatom or a carbon atom; heterocyclyl can be connected to an aromatic ring or a non-aromatic ring; and heterocyclyl can be connected to a bridged ring or a spiro ring. Non-limiting examples include oxiranyl, azacyclopropyl, oxetanyl, azetidinyl, 1,3-dioxolanyl, 1,4-dioxolanyl, 1,3-dioxanyl, azacycloheptyl, pyridyl, furyl, thienyl, pyranyl, N-alkylpyrrolyl, pyrimidyl, pyrazinyl, pyridazinyl, imidazolyl, piperidyl, morpholinyl, thiomorpholinyl, 1,3-dithianyl, dihydrofuryl, dihydropyranyl, dithiolanyl, tetrahydrofuryl, tetrahydropyrrolyl, tetrahydroimidazolyl, tetrahydrothiazolyl, tetrahydropyranyl, benzoimidazolyl, benzopyridyl, pyrrolopyridyl, benzodihydrofuryl, pyrrolyl, pyrazolyl, thiazolyl, oxazolyl, pyrazinyl, indazolyl, benzothienyl, benzofuryl, benzopyrrolyl, benzoimidazolyl, benzothiazolyl, benzoxazolyl, benzopyridyl, benzopyrimidyl, benzopyrazinyl, piperazinyl, azabicyclo[3.2.1]octanyl, azabicyclo[5.2.0]nonanyl, oxatricyclo[5.3.1.1]dodecyl, azaadamantyl, oxaspiro[3.3]heptanyl,

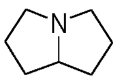,

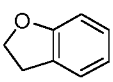, 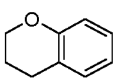, 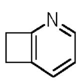, 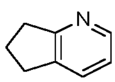, 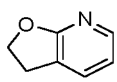, 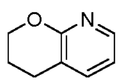, 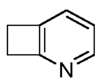, 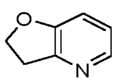,

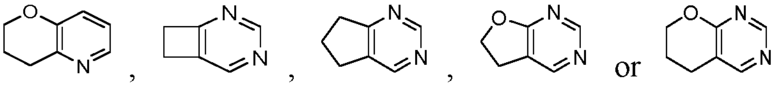, , , or

"Heterocyclyl" or "heterocycle" can be monovalent, divalent, trivalent or tetravalent.

"Spiro ring" or "spiro ring group" refers to a polycyclic group that shares one atom (called a spiro atom) between substituted or unsubstituted monocyclic rings. The number of ring atoms in the spiro ring system includes but is not limited to 5 to 20, 6 to 14, 6 to 12, or 6 to 10, wherein one or more rings may contain 0 or more (including but not limited to 1, 2, 3 or 4) double bonds, and can optionally contain 0 to 5 heteroatoms selected from N, O or $S(=O)_n$.

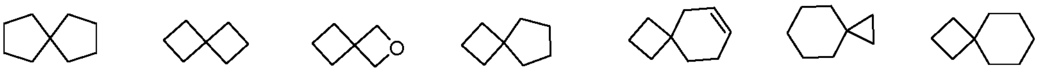

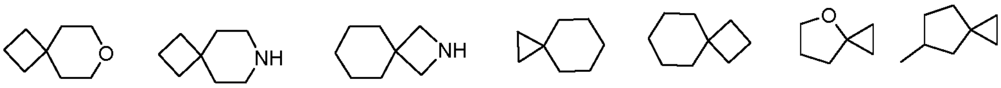

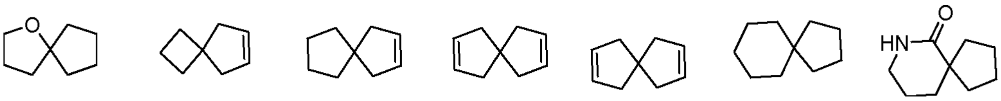

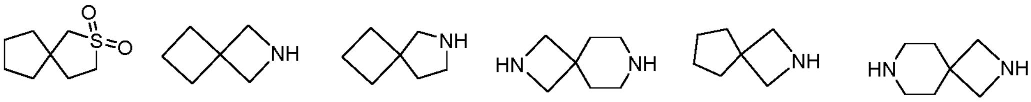

"Spiro ring" or "spiro ring group" can be monovalent, divalent, trivalent or tetravalent.

"Fused ring" or "fused ring group" refers to a polycyclic group in which each ring in the system shares an adjacent pair of atoms with other rings in the system, wherein one or more rings may contain 0 or more (including but not limited to 1, 2, 3 or 4) double bonds, and may be substituted or unsubstituted, and each ring in the fused ring system may contain 0 to 5 heteroatoms or groups containing heteroatoms (including but not limited to N, $S(=O)_n$ or O, wherein n is 0, 1 or 2). The number of ring atoms in the fused ring system includes but is not limited to 5 to 20, 5 to 14, 5 to 12, or 5 to 10. Non-limiting examples include:

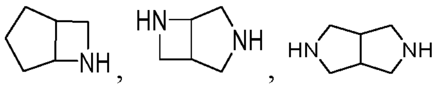, , ,

,

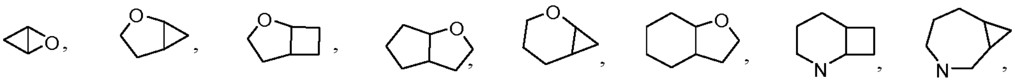, , , , , , , ,

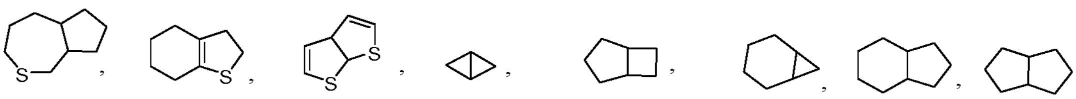, , , , , , , ,

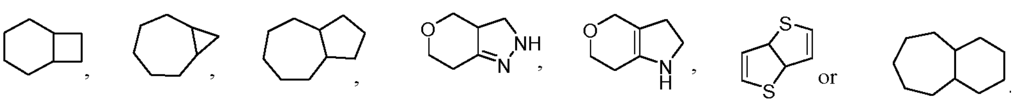, , , , , , or .

"Fused ring" or "fused ring group" can be monovalent, divalent, trivalent or tetravalent.

"Bridged ring" or "bridged ring group" refers to a substituted or unsubstituted polycyclic group containing any two atoms that are not directly connected, and may contain 0 or more double bonds. Any ring in the fused ring system may contain 0 to 5 groups selected from heteroatoms or groups containing heteroatoms (including but not limited to N, S(=O)n or O, wherein n is 0, 1 or 2). The number of ring atoms includes but is not limited to 5 to 20, 5 to 14, 5 to 12 or 5 to 10. Non-

limiting examples include

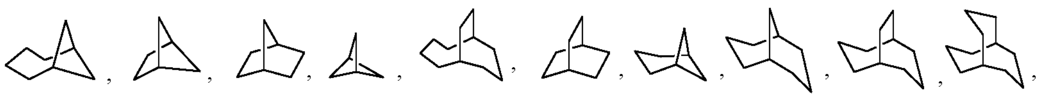
, , , , , , , , , ,

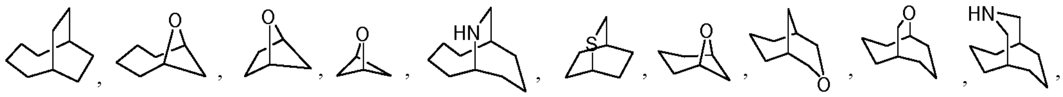
, , , , , , , , , ,

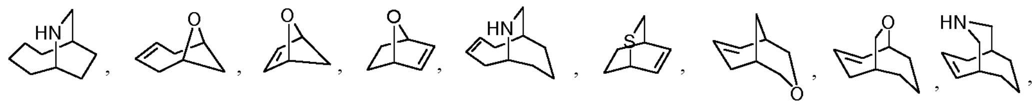
, , , , , , , , ,

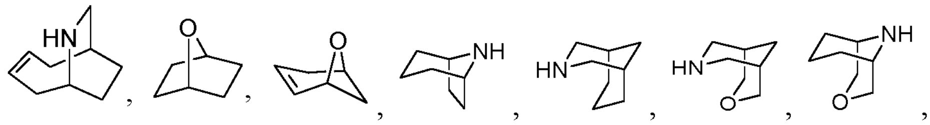
, , , , , , , ,

cubane or adamantane. "Bridged ring" or "bridged ring group" can be monovalent, divalent, trivalent or tetravalent.

"Carbospiro ring", "spiro ring carbocyclyl", "spirocarbocyclyl" or "carbospiro ring group" refers to a "spiro ring" with a ring system consisting only of carbon atoms. The definition of the "carbospiro ring", "spiro ring carbocyclyl", "spirocarbocyclyl" or "carbospiro ring group" herein is consistent with that of a spiro ring.

"Carbo-fused ring", "fused ring carbocyclyl", "fused carbocyclyl" or "carbo-fused ring group" refers to a "fused ring" with a ring system consisting only of carbon atoms. The definition of the "carbo-fused ring", "fused ring carbocyclyl", "fused carbocyclyl" or "carbo-fused ring group" herein is consistent with that of a fused ring.

"Carbo-bridged ring", "bridged ring carbocyclyl", "bridged carbocyclyl" or "carbo-bridged ring group" refers to a "bridged ring" with a ring system consisting only of carbon atoms. The definition of the "carbo-bridged ring", "bridged ring carbocyclyl", "bridged carbocyclyl" or "carbo-bridged ring group" herein is consistent with that of a bridged ring.

"Mono-heterocyclic ring", "monocyclic heterocyclyl" or "mono-heterocyclic ring group" refers to "heterocyclyl" or "heterocycle" with a monocyclic system. The definition of the "heterocyclyl", "monocyclic heterocyclyl" or "mono-heterocyclic ring group" herein is consistent with that of heterocycle.

"Fused-heterocyclic ring", "fused-heterocyclic ring group", "fused ring heterocyclyl" or "fused-heterocyclic ring group" refers to a "fused ring" containing a heteroatom. The definition of the "fused-heterocyclic ring", "fused-heterocyclic ring group", "fused ring heterocyclyl" or "fused-heterocyclic ring group" herein is consistent with that of a fused ring.

"Spiro-heterocyclic ring", "spiro-heterocyclic ring group", "spiro ring heterocyclyl" or "spiro-heterocyclic ring group" refers to a "spiro ring" containing a heteroatom. The definition of the "spiro-heterocyclic ring", "spiro-heterocyclic ring group", "spiro ring heterocyclyl" or "spiro-heterocyclic ring group" herein is consistent with that of a spiro ring.

"Bridged-heterocyclic ring", "bridged-heterocyclic ring group", "bridged ring heterocyclyl" or "bridged-heterocyclic ring group" refers to a "bridged ring" containing a heteroatom. The definition of the "bridged-heterocyclic ring", "bridged-heterocyclic ring group", "bridged ring heterocyclyl" or "bridged-heterocyclic ring group" herein is consistent with that of a bridged ring.

"Aryl" or "aromatic ring" refers to a substituted or unsubstituted aromatic hydrocarbyl group with a monocyclic ring or a fused ring, wherein the number of ring atoms in the aromatic ring includes but is not limited to 6 to 18, 6 to 12 or 6 to 10 carbon atoms. The aryl ring can be fused to a saturated or unsaturated carbocycle or heterocycle, wherein the ring connected to the parent structure is an aryl ring. Non-limiting examples include a benzene ring, a naphthalene ring, or

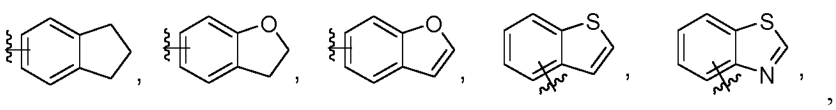
, , , , ,

and "aryl" or "aromatic ring" may be monovalent, divalent, trivalent or tetravalent. When divalent, trivalent or tetravalent, the point of connection is on the aryl ring.

"Heteroaryl" or "heteroaromatic ring" refers to a substituted or unsubstituted aromatic hydrocarbyl group containing 1 to 5 heteroatoms or groups containing heteroatoms (including but not limited to N, O or S(=O)n, wherein n is 0, 1 or 2), wherein the number of ring atoms in the heteroaromatic ring includes but is not limited to 5-15, 5-10 or 5-6.

Non-limiting examples of heteroaryl include, but are not limited to pyridyl, furyl, thienyl, pyridyl, pyranyl, N-alkylpyrrolyl, pyrimidyl, pyrazinyl, pyridazinyl, imidazolyl, benzopyrazole, benzoimidazole, benzopyridine, pyrrolopyridine, etc. The heteroaryl ring may be fused to a saturated or unsaturated carbocycle or heterocycle, wherein the ring connected to the parent structure is a heteroaryl ring. Non-limiting examples include

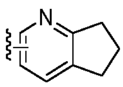, 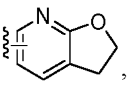, 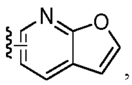, 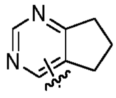, 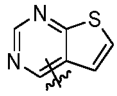, 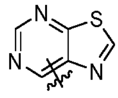, 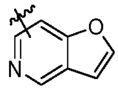

and

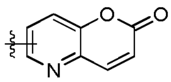

The definition of the heteroaryl described herein is consistent with this definition. Heteroaryl can be monovalent, divalent, trivalent or tetravalent. When divalent, trivalent or tetravalent, the point of connection is on the heteroaryl ring.

"5-membered ring fused 5-membered heteroaromatic ring" refers to a 5-fused-5-membered fused heteroaromatic ring, wherein at least one of the two fused rings contains at least one heteroatom (including but not limited to O, S or N), and the entire group is aromatic. Non-limiting examples include a pyrrolopyrrole ring, a pyrazolopyrrole ring, a pyrazolopyrazole ring, a pyrrolofuran ring, a pyrazolofuran ring, a pyrrolothiophene ring and a pyrazolothiophene ring.

"5-fused-6-membered heteroaromatic ring" refers to a 5-fused-6-membered fused heteroaromatic ring, wherein at least one of the two fused rings contains at least one heteroatom (including but not limited to O, S or N), and the entire group is aromatic. Non-limiting examples include a benzo 5-membered heteroaryl and 6-membered heteroaromatic ring fused 5-membered heteroaromatic ring.

"Substitution" or "substituted" refers to a substitution with 1 or more (including, but not limited to 2, 3, 4 or 5) substituents including, but not limited to H, F, Cl, Br, I, alkyl, cycloalkyl, alkoxy, haloalkyl, mercaptan, hydroxyl, nitro, mercapto, amino, cyano, isocyano, aryl, heteroaryl, heterocyclyl, bridged ring group, spiro ring group, fused ring group, hydroxyalkyl, =O, carbonyl, aldehyde, carboxylic acid, carboxylate, $-(CH_2)_m-C(=O)-R^a$, $-O-(CH_2)_m-C(=O)-R^a$, $-(CH_2)_m-C(=O)-NR^bR^c$, $-(CH_2)_mS(=O)_nR^a$, $-(CH_2)_m$-alkenyl-$R^a$, $OR^d$ or $-(CH_2)_m$-alkynyl-$R^a$ (wherein m and n are 0, 1 or 2), arylthio, thiocarbonyl, silyl, $-NR^bR^c$, etc., wherein $R^b$ and $R^c$ are independently selected from H, hydroxyl, amino, carbonyl, alkyl, alkoxy, cycloalkyl, heterocyclyl, aryl, heteroaryl, sulphonyl, or trifluoromethylsulphonyl. Alternatively, $R^b$ and $R^c$ can form a five- or six-membered cycloalkyl or heterocyclyl. $R^a$ and $R^d$ are each independently selected from aryl, heteroaryl, alkyl, alkoxy, cycloalkyl, heterocyclyl, carbonyl, ester group, bridged ring group, spiro ring group or bicyclic ring group.

"Containing 1 to 5 heteroatoms selected from O, S or N" means containing 1, 2, 3, 4 or 5 heteroatoms selected from O, S or N.

"Substituted with 0 to X substituents" refers to the circumstance where the specified entity is substituted with 0, 1, 2, 3 ... X substituents, wherein X is selected from any integer between 1 and 10. For example, "substituted with 0 to 4 substituents" refers to the circumstance where the specified entity is substituted with 0, 1, 2, 3 or 4 substituents. For example, "substituted with 0 to 5 substituents" refers to the circumstance where the specified entity is substituted with 0, 1, 2, 3, 4 or 5 substituents. For example, "bridged-heterocyclic ring is optionally further substituted with 0 to 4 substituents selected from H or F" means that the bridged-heterocyclic ring is optionally further substituted with 0, 1, 2, 3 or 4 substituents selected from H or F.

An X- to Y-membered ring ($3 \leq X < Y$, and Y is selected from any integer between 4 and 12) includes X, X+1-, X+2-, X+3-, X+4-, ... to Y-membered rings. Rings include heterocycle, carbocycle, an aromatic ring, aryl, heteroaryl, cycloalkyl, a mono-heterocyclic ring, a fused-heterocyclic ring, a spiro-heterocyclic ring or a bridged-heterocyclic ring. For example, a "4- to 7-membered mono-heterocyclic ring" refers to a 4-, 5-, 6- or 7-membered mono-heterocyclic ring, and a "5- to 10-membered fused-heterocyclic ring" refers to a 5-, 6-, 7-, 8-, 9- or 10-membered fused-heterocyclic ring.

The term "optional" or "optionally" refers to that the events or circumstances subsequently described may but not necessarily occur, and the description includes the occasions where the events or circumstances occur or do not occur. For example, "alkyl optionally substituted with F" means that the alkyl may but not necessarily be substituted with F, and the description includes the case where the alkyl is substituted with F and the case where the alkyl is not substituted with F.

"Pharmaceutically acceptable salt" or "pharmaceutically acceptable salt thereof" refers to a salt of the compound according to the present invention, which salt maintains the biological effectiveness and characteristics of a free acid or a free base, and is obtained by reacting the free acid with a non-toxic inorganic base or organic base, or reacting the free base with a non-toxic inorganic acid or organic acid.

"Pharmaceutical composition" refers to a mixture of one or more compounds of the present invention, or

stereoisomers, tautomers, deuterates, solvates, prodrugs, metabolites, pharmaceutically acceptable salts or co-crystals thereof and other chemical components, wherein "other chemical components" refer to pharmaceutically acceptable carriers, excipients and/or one or more other therapeutic agents.

The term "preparation strength" refers to the weight of the drug substance contained in each vial, tablet or other unit preparation.

"Carrier" refers to a material that does not cause significant irritation to an organism and does not eliminate the biological activity and characteristics of a compound administered.

"Excipient" refers to an inert substance added to a pharmaceutical composition to facilitate the administration of a compound. Non-limiting examples include calcium carbonate, calcium phosphate, sugar, starch, cellulose derivatives (including microcrystalline cellulose), gelatin, vegetable oils, polyethylene glycols, diluents, granulating agents, lubricants, adhesives and disintegrants.

"Prodrug" refers to a compound that can be converted into the compound of the present invention with the biological activity by metabolism *in vivo.* The prodrug of the present invention is prepared by modifying an amino or carboxyl group in the compound of the present invention, and the modification can be removed by conventional operations or *in vivo* to obtain a parent compound. When the prodrug of the present invention is administered to a mammalian individual, the prodrug is split to form a free amino or carboxyl group.

The term "co-crystal" refers to a crystal formed by the combination of active pharmaceutical ingredient (API) and co-crystal former (CCF) under the action of hydrogen bonds or other non-covalent bonds. The pure state of API and CCF are both solid at room temperature, and there is a fixed stoichiometric ratio between various components. The co-crystal is a multi-component crystal, which includes both a binary co-crystal formed between two neutral solids and a multi-element co-crystal formed between a neutral solid and a salt or solvate.

"Animal" is meant to include mammals, such as humans, companion animals, zoo animals, and domestic animals, preferably humans, horses, or dogs.

The term "stereoisomer" refers to an isomer produced as a result of different spatial arrangement of atoms in molecules, including cis-trans isomers, enantiomers and conformational isomers.

"Tautomer" refers to a functional group isomer produced by the rapid movement of an atom in two positions in a molecule, such as keto-enol isomerization and amide-imino alcohol isomerization.

"$IC_{50}$" refers to the concentration of a drug or inhibitor required to inhibit half of a given biological process (or a component of the process such as an enzyme, a receptor and a cell).

## Detailed Description of Embodiments

The technical solutions of the present invention will be described in detail by the following examples, but the scope of protection of the present invention includes but is not limited thereto.

The structures of the compounds are determined by nuclear magnetic resonance (NMR) or (and) mass spectrometry (MS). The NMR shift (δ) is given in the unit of $10^{-6}$ (ppm). NMR is measured with (Bruker Avance III 400 and Bruker Avance 300) NMR instrument, and the solvent for determination is deuterated dimethyl sulphoxide (DMSO-$d_6$), deuterated chloroform ($CDCl_3$), and deuterated methanol ($CD_3OD$), and the internal standard is tetramethylsilane (TMS);

MS is determined with Agilent 6120B (ESI) and Agilent 6120B (APCI);

HPLC is determined with Agilent 1260DAD high pressure liquid chromatograph (Zorbax SB-C18 100 × 4.6 mm, 3.5 μM);

Yantai Huanghai $HSGF_{254}$ or Qingdao $GF_{254}$ silica gel plate is used as a thin layer chromatography silica plate, and the silica gel plate for the thin layer chromatography (TLC) is of the specification of 0.15 mm-0.20 mm, and the specification when separating and purifying a product by thin layer chromatography is 0.4 mm - 0.5 mm;

for the column chromatography, Yantai Huanghai silica gel of 200-300 mesh silica gel is generally used as a carrier;

Boc: tert-butoxycarbonyl; Ts: p-toluenesulphonyl; CDI: N,N-carbonyldiimidazole; Cbz: benzyloxycarbonyl; TMS: Trimethylsilane; DDQ: 2,3-dichloro-5,6-dicyanobenzoquinone; DMAP: 4-dimethylaminopyridine; TBTU: CAS 125700-67-6; DIPEA: N,N-diisopropylethylamine; DMF: N,N-dimethylformamide; THF: tetrahydrofuran; HATU: CAS: 148893-10-1.

**Example 1**: Preparation of compound 1

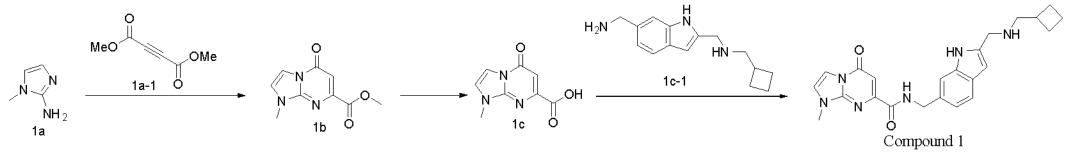

1a 1b 1c Compound 1

Step 1: Preparation of **1b**

Compound 1a (2.0 g, 20.59 mmol) was dissolved in 100 mL of water and 1a-1 (3.83 g, 26.96 mmol) was added. The reaction liquid was maintained at room temperature and stirred for reaction for 16 hours. 100 mL of dichloromethane was added for separation by extraction. The aqueous phase was extracted with dichloromethane (100 mL × 1) again, and the organic phases were combined. The organic phase was washed with saturated brine (100 mL × 1) once, dried over anhydrous sodium sulphate, and concentrated under reduced pressure. The residue was purified by column chromatography, to obtain 1b (1.2 g, yield 28.1%).
LC-MS: m/z (ESI): 208.1$[M+H]^+$

Step 2: Preparation of **1c**

Under nitrogen protection, **1b** (1.20 g, 5.79 mmol) was dissolved in an anhydrous THF (20 mL) solution, and potassium trimethylsilanolate (1.49 g, 11.58 mmol), was added and reacted at 70°C for 3 h. The reaction was cooled to room temperature, and 4 M hydrogen chloride dioxane solution (3.2 mL) was added and then filtered. The resulting solid from filtration was washed with diethyl ether, and dried to obtain the target compound (**1c**) (0.75 g, 67.0%).
LC-MS: m/z (ESI): 194.1$[M+H]^+$

Step 3: Preparation of **compound 1**

Compound **1c** (58 mg, 0.3 mmol) was dissolved in 5 mL of ultra-dried DMF, and then 1c-1 (100 mg, 0.41 mmol), HATU (171 mg, 0.45 mmol), and DIPEA (0.25 mL) were added into the reaction vessel in sequence. The reaction liquid was maintained at room temperature and stirred for reaction for 16 h, and 10 mL of ethyl acetate and 10 mL of a saturated $NaHCO_3$ aqueous solution were added for separation by extraction. The aqueous phase was extracted with ethyl acetate (10 mL × 1) again, and the organic phases were combined. The organic phase was washed with saturated brine (10 mL × 1) once, dried over anhydrous sodium sulphate and concentrated under reduced pressure. The residue was subjected to prep-HPLC to obtain the trifluoroacetate of compound **1** (43 mg, yield 34.2%).
Preparation conditions: Instrument: SHIMADZU LC-20AP; chromatographic column: Phenomenex C18; mobile phase: A: 0.1% TFA aqueous solution; B: acetonitrile; gradient: mobile phase B: increasing from 10% to 40% over 15 min; flow rate: 25 mL/min; column temperature: room temperature; detection wavelength: 210 nm; sample treatment: sample concentration: 100 mg/ml, solvents: acetonitrile and water. injection volume: 1 ml/injection.
Post treatment: freeze drying after separation to obtain a solid powder.

$^1$H NMR (400 MHz, DMSO-$d_6$) δ 11.06 (s, 1H) 9.25-9.17 (m, 1H),8.83- 8.68 (m, 2H), 7.74-7.69 (m, 2H),7.53-7.48 (m, 1H), 7.41-7.37 (m, 1H), 7.07-7.02 (m, 1H), 6.57-6.53 (m, 1H), 6.46 (s, 1H), 4.61-4.54 (m, 2H), 4.29-4.20 (m, 2H),3.78 (s, 3H), 3.04- 2.95 (m, 2H), 2.65-2.55(m, 1H), 2.10-2.01 (m, 2H), 1.93-1.70 (m, 4H).
LC-MS: m/z =419.2$[M+H]^+$

**Example 2**: Preparation of compound 2

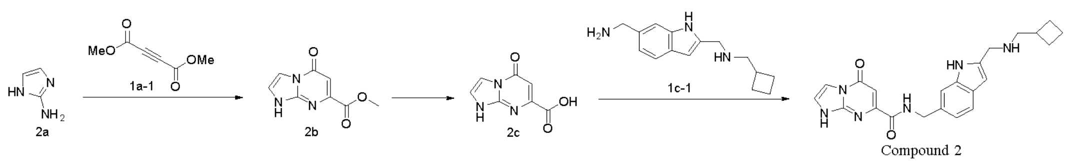

2a 2b 2c Compound 2

Step 1: Preparation of **2b**

Compound 2a (2.0 g, 24.07 mmol) was dissolved in 100 mL of water and 1a-1 (3.42 g, 24.07 mmol) was added.

The reaction liquid was maintained at room temperature and stirred for reaction for 16 hours. 100 mL of dichloromethane was added for separation by extraction. The aqueous phase was extracted with dichloromethane (100 mL × 1) again, and the organic phases were combined. The organic phase was washed with saturated brine (100 mL × 1) once, dried over anhydrous sodium sulphate, and concentrated under reduced pressure. The residue was separated and purified by column chromatography, to obtain 2b (0.6 g, yield 12.9%).
LC-MS: m/z (ESI):194.1[M+H]$^+$

Step 2: Preparation of**2c**

Under nitrogen protection, **2b** (0.60 g, 3.11 mmol) was dissolved in an anhydrous THF (20 mL) solution, and then potassium trimethylsilanolate (0.80 g, 6.22 mmol) was added and reacted under stirring at 70°C for 3 h. The reaction was cooled to room temperature, and 4 M hydrogen chloride dioxane solution (0.5 mL) was added and then filtered. The resulting solid from filtration was washed with diethyl ether and dried to obtain the target compound (**2c**) (0.45 g, 80.7%).

Step 3: Preparation of **compound 2**

Compound **2c** (54 mg, 0.3 mmol) was dissolved in 5 mL of ultra-dried DMF, and then 1c-1 (100 mg, 0.41 mmol), HATU (171 mg, 0.45 mmol), and DIPEA (0.15 mL) were added into the reaction vessel in sequence. The reaction liquid was maintained at room temperature and stirred for reaction for 16 h, and 10 mL of ethyl acetate and 10 mL of a saturated $NaHCO_3$ aqueous solution were added for separation by extraction. The aqueous phase was extracted with ethyl acetate (10 mL × 1) again, and the organic phases were combined. The organic phase was washed with saturated brine (10 mL × 1) once, dried over anhydrous sodium sulphate and concentrated under reduced pressure. The residue was subjected to prep-HPLC to obtain the trifluoroacetate of compound **2** (18 mg, yield 14.83%).

Preparation conditions: Instrument: SHIMADZU LC-20AP; chromatographic column: Phenomenex C18; mobile phase: A: 0.1% TFA aqueous solution; B: acetonitrile; gradient: mobile phase B: increasing from 8% to 38% over 15 min; flow rate: 25 mL/min; column temperature: room temperature; detection wavelength: 210 nm; sample treatment: sample concentration: 100 mg/ml, solvents: acetonitrile and water. injection volume: 1 ml/injection.

Post treatment: freeze drying after separation to obtain a solid powder.
$^1$H NMR (400 MHz, DMSO-$d_6$) δ 12.91 (s, 1H), 11.07 (s, 1H), 8.85-8.70 (m, 3H),7.71-7.68 (m, 1H), 7.67-7.62 (m, 1H),7.53-7.48 (m, 1H), 7.41-7.37 (m, 1H), 7.06-7.01 (m, 1H), 6.58-6.52 (m, 1H), 6.45 (s, 1H), 4.59-4.53 (m, 2H), 4.29-4.22 (m, 2H),3.03-2.95 (m, 2H), 2.67-2.55(m, 1H), 2.11-1.99 (m, 2H), 1.92-1.68 (m, 4H).
LC-MS: m/z =405.2[M+H]$^+$.

**Example 3**: Preparation of compound 3

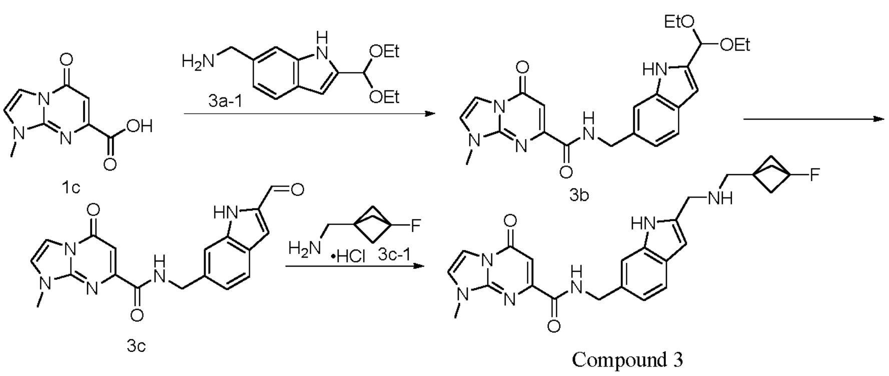

Compound 3

Step 1: Preparation of **3b**

Compound **1c** (200 mg, 1.04 mmol), TBTU (500 mg, 1.56 mmol), and DIPEA (0.52 mL) were dissolved in 5 mL of ultra-dried DMF, and the reaction liquid was reacted at room temperature for 1 hour. Then 3a-1 (260 mg, 1.05 mmol) was added into the reaction vessel and reacted at room temperature for 16 hours. 10 mL of ethyl acetate and 10 mL of a saturated $NaHCO_3$ aqueous solution were added for separation by extraction. The organic phase was taken and the aqueous phase was extracted with ethyl acetate (10 mL) again, and the organic phases were combined. The organic

phase was washed with saturated brine (10 mL), dried over anhydrous sodium sulphate and concentrated under reduced pressure. The residue was separated and purified by column chromatography, to obtain compound **3b** (300 mg, yield 68.1%).

Step 2: Preparation of**3c**

Under nitrogen protection, **3b** (300 mg, 0.71 mmol) was dissolved in an anhydrous THF (5 mL) solution, and water (1 mL) and acetic acid (1 mL) were added and reacted at room temperature for 2 h. The reaction liquid was concentrated and 10 mL of water was added. A solid precipitated out and then was filtered. The resulting solid from filtration was washed with diethyl ether and dried to obtain the target compound (**3c**) (220 mg, 88.70%).

$^1$H NMR (400 MHz, DMSO-$d_6$): δ 11.88 (s, 1H), 9.81 (s, 1H), 9.33-9.25 (m, 1H), 7.75-7.66 (m, 3H), 7.43-7.33 (m, 2H), 7.15-7.08 (m, 1H), 6.46 (s, 1H), 4.62-4.57 (m, 2H), 3.79 (s, 3H).

Step 3: Preparation of **compound 3**

At room temperature, compound **3c** (120 mg, 0.34 mmol) was added into a round bottom flask with 4 mL of ultra-dried DMSO, and then 3c-1 (103 mg, 0.68 mmol) and AcOH (0.1 mL) were added into the reaction vessel in sequence. The reaction liquid was maintained at room temperature for reaction for 2 h, and then to the mixed reaction liquid, sodium triacetoxyborohydride (216 mg, 1.02 mmol) was added and reacted overnight. 10 mL of an organic solvent (dichloromethane/methanol = 10/1) and 10 mL of a saturated $NaHCO_3$ aqueous solution were added for separation by extraction, to obtain the organic phase. The aqueous phase was extracted with 10 mL of an organic solvent (dichloromethane/-methanol = 10/1) again, the organic phases were combined, washed with saturated brine (10 mL × 2), dried over anhydrous sodium sulphate, and concentrated under reduced pressure. The residue was separated and purified by silica gel chromatographic column to obtain **compound 3** (46 mg, 30.1%).

LC-MS: m/z (ESI): 449.3 [M+1]$^+$
$^1$H NMR (400 MHz, DMSO-$d_6$): δ 10.83 (s, 1H), 9.17-9.07 (m, 1H), 7.77-7.67 (m, 2H), 7.41-7.33 (m, 1H), 7.28 (s, 1H), 6.98-6.91 (m, 1H), 6.47 (s, 1H), 6.20 (s, 1H), 4.55 (d, 2H), 3.80 (s, 2H), 3.77 (s, 3H), 2.76 (s, 2H), 1.95-1.90 (m, 6H).

**Example 4**: Preparation of compound 4

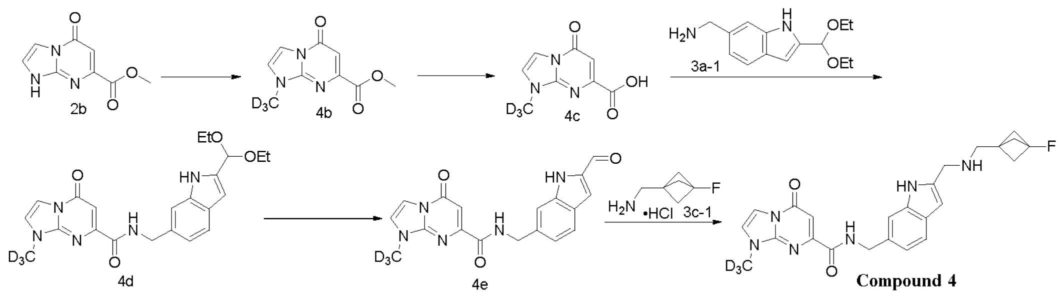


Step 1: Preparation of **4b**

Compound **2b** (500 mg, 2.59 mmol) was dissolved in 10 mL of ultra-dried DMF, and under an ice bath, sodium hydride (204 mg, 5.10 mmol) was added and reacted for 1 hour. Then deuteriodomethane (750 mg, 5.18 mmol) was added into the reaction vessel at room temperature and reacted for 16 hours. After the completion of the reaction, 1N hydrochloric acid was added to adjust the PH to 7, and 10 mL of dichloromethane and 10 mL of a saturated $NaHCO_3$ aqueous solution were added for separation by extraction. The organic phase was taken and the aqueous phase was extracted with dichloromethane (10 mL × 1) again, and the organic phases were combined. The organic phase was washed with saturated brine (10 mL), dried over anhydrous sodium sulphate and concentrated under reduced pressure. The residue was purified by column chromatography, to obtain compound **4b** (200 mg, yield 36.7%).
LC-MS: m/z (ESI): 211.1[M+H]$^+$

Step 2: Preparation of**4c**

Under nitrogen protection, **4b** (200 mg, 0.95 mmol) was dissolved in an anhydrous THF (5 mL) solution, and then potassium trimethylsilanolate (243 mg, 1.90 mmol) was added and reacted at 70°C for 1 h. The reaction was cooled to room temperature, and 4 M hydrogen chloride dioxane solution (0.75 mL) was added and then filtered. The resulting solid from filtration was washed with diethyl ether and dried to obtain the target compound (**4c**) (160 mg, 85.8%).
LC-MS: m/z (ESI): 197.1 $[M+H]^+$

Step 3: Preparation of 4d

Compound **4c** (160 mg, 0.82 mmol) and CDI (133 mg, 0.82 mmol) were dissolved in 5 mL of ultra-dried DMF, and the reaction liquid was reacted at 70°C for 3 hour and then cooled to room temperature. 3a-1 (203 mg, 0.82 mmol) was added into the reaction vessel and reacted at room temperature for 16 hours. After the completion of the reaction, 10 mL of ethyl acetate and 10 mL of water were added for separation by extraction. The organic phase was taken and the aqueous phase was extracted with ethyl acetate (10 mL) again, and the organic phases were combined. The organic phase was washed with saturated brine (10 mL $\times$ 1) once, dried over anhydrous sodium sulphate and concentrated under reduced pressure. The residue was purified by column chromatography, to obtain compound **4d** (330 mg, yield 94.3%).

Step 4: Preparation of**4e**

Under nitrogen protection, **4d** (330 mg, 0.77 mmol) was dissolved in an anhydrous THF (10 mL) solution, and then water (1 mL) and acetic acid (1 mL) were added and reacted at room temperature for 2 h. The reaction solution was concentrated under reduced pressure and 10 mL of water was added. A solid precipitated out and then was filtered. The resulting solid from filtration was washed with diethyl ether and dried to obtain the target compound **(4e)** (270 mg, 99.5%).
LC-MS: m/z (ESI): 353.0 $[M+H]^+$

Step 5: Preparation of **compound 4**

At room temperature, compound **4e** (270 mg, 0.77 mmol) was added into a round bottom flask with 8 mL of ultra-dried DMSO, and then 3c-1 (175 mg, 1.16 mmol) and AcOH (0.5 mL) were added into the reaction vessel in sequence. The reaction liquid was maintained at 70°C for reaction for 2 h, and then to the mixed reaction liquid, sodium triacetoxyborohydride (489 mg, 2.31 mmol) was added and reacted overnight. After the completion of the reaction, 10 mL of dichloromethane/methanol (v : v = 10 : 1) and 10 mL of a saturated $NaHCO_3$ aqueous solution were added for separation by extraction. The aqueous phase was extracted with 10 mL of dichloromethane/methanol (v : v = 10 : 1) again. The organic phases were combined and washed with saturated brine twice (10 mL $\times$ 2), dried over anhydrous sodium sulphate and concentrated under reduced pressure. The residue was separated and purified by silica gel chromatographic column to obtain **compound 4** (160 mg, 46.0%).

LC-MS: m/z (ESI): 452.3 $[M+H]^+$
$^1$H NMR (400 MHz, DMSO-$d_6$): $\delta$ 10.83 (s, 1H), 9.15-9.07 (m, 1H), 7.73-7.67 (m, 2H), 7.39-7.33 (m, 1H), 7.29 (s, 1H), 6.98-6.91 (m, 1H), 6.47 (s, 1H), 6.21 (s, 1H), 4.55 (d, 2H), 3.81 (s, 2H), 2.76 (s, 2H), 1.95-1.89 (m, 6H).

**Example 5**: Preparation of compound 5

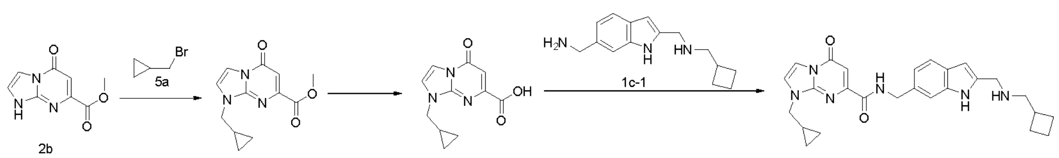


2b 5b 5c Compound 5

Step 1: Preparation of **5b**

2b (500 mg, 2.6 mmol), 5a (1.04 g, 7.8 mmol), sodium iodide (40 mg, 0.26 mmol) and potassium carbonate (1.07 g, 7.8 mmol) were added into acetone (20 mL), and reacted under stirring at 70°C for 12 hours. The reaction was cooled to room temperature and concentrated under reduced pressure. The residue was separated and purified by silica gel column

to obtain **5b** (360 mg, yield 54%).
LC-MS: m/z (ESI): 248.1 $[M+H]^+$

Step 2: Preparation of**5c**

**5b** (99 mg, 0.4 mmol) and lithium hydroxide monohydrate (84 mg, 2.0 mmol) were added into a mixed solvent of tetrahydrofuran (5 mL) and water (1.5 mL), and reacted under stirring at room temperature for 1 hour. The reaction liquid was adjusted to pH = 6 using 0.5 M hydrochloric acid and the resulting mixture was concentrated to dryness under reduced pressure, to obtain crude **5c**.
LC-MS: m/z (ESI): 234.1$[M+H]^+$

Step 3: Preparation of **compound 5**

The crude **5c** obtained form the previous step and carbonyldiimidazole (73 mg, 0.45 mmol) were added into DMF (7 mL), and stirred at 70°C for 5 hours, and then the reaction was brought to room temperature. To the reaction system, **1c-1** (121 mg, 0.5 mmol) was added, and the reaction was continued under stirring at room temperature for 10 hours. Then the reaction liquid was concentrated to dryness under reduced pressure, and the residue was sent to prep-HPLC to obtain the **trifluoroacetate of compound 5** (15 mg).

Prep-HPLC conditions: instrument: Waters 2767 preparative liquid phase chromatographic instrument; chromatographic column: XBridge@Prep C18 (19 mm × 250 mm). The sample was dissolved in DMF and filtered with a 0.45 uM filter head to prepare a sample solution. Preparative chromatography conditions: a. mobile phase A: acetonitrile; b. water (containing 0.1% trifluoroacetic acid); mobile phase A, gradient elution, 10% to 50%; flow rate: 12 mL/minute; elution time: 20 minutes.

LC-MS: m/z (ESI): 459.3$[M+H]^+$
$^1$H NMR (400 MHz, DMSO-$d_6$): δ 11.09(s, 1H), 9.26 (t, 1H), 8.81 (s, 2H), 7.83 (d, 1H), 7.76 (d, 1H), 7.50 (d, 1H), 7.38 (s, 1H), 7.04 (d, 1H), 6.55 (s, 1H), 6.47 (s, 1H), 4.58 (d, 2H), 4.25 (t, 2H), 4.10 (d, 2H), 3.04- 2.94 (m, 2H), 2.66- 2.56 (m, 1H), 2.11- 2.00 (m, 2H), 1.92- 1.69 (m, 4H), 1.38- 1.26 (m, 1H), 0.59- 0.44 (m, 4H).

**Example 6**: Preparation of compound 6

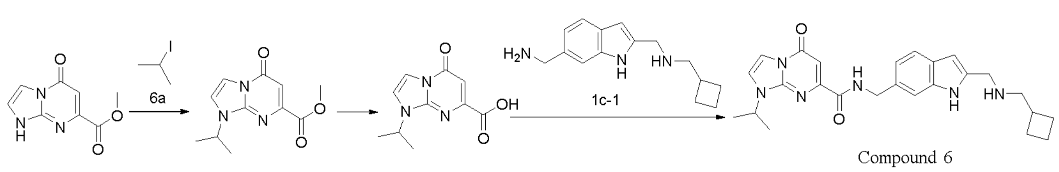


2b 6b 6c

**The trifluoroacetate of Compound 6** was obtained using intermediate **2b** and **6a** as starting material, by referring to the synthesis method of example 5.

Prep-HPLC conditions: instrument: Waters 2767 preparative liquid phase chromatographic instrument; chromatographic column: XBridge@Prep C18 (19 mm × 250 mm). The sample was dissolved in DMF and filtered with a 0.45 uM filter head to prepare a sample solution. Preparative chromatography conditions: a. mobile phase A: acetonitrile; b. water (containing 0.1% trifluoroacetic acid); mobile phase A, gradient elution, 10% to 50%; flow rate: 12 mL/minute; elution time: 20 minutes.
LC-MS: m/z (ESI): 447.3$[M+H]^+$

**Example 7**: Preparation of compound 7

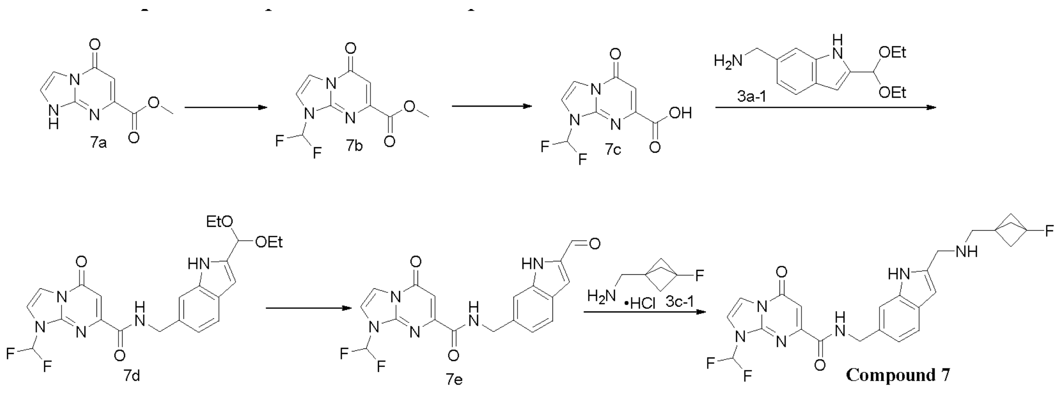


7a 7b 7c 7d 7e Compound 7

Step 1: Preparation of **7b**

Compound **7a** (500 mg, 2.59 mmol) was dissolved into ultra-dried DMF (20 mL), cesium carbonate (1.50 g, 4.62 mmol) was added and heated to 110°C, and then sodium difluorochloroacetate (770 mg, 5.05 mmol) was added into the reaction vessel for reaction for 20 minutes. After the completion of the reaction, the mixture was cooled to room temperature, and 20 mL of dichloromethane and 20 mL of water were added for separation by extraction. The aqueous phase was extracted with dichloromethane (20 mL) again, and the dichloromethane layers were combined, washed once with saturated brine (30 mL), dried over anhydrous sodium sulphate, and concentrated under reduced pressure. The residue was purified by column chromatography to obtain compound **7b** (570 mg, yield 90.5%).
LC-MS: m/z (ESI): 244.1[M+ H]$^+$

Step 2: Preparation of**7c**

Under nitrogen protection, **7b** (570 mg, 2.34 mmol) was dissolved in an anhydrous THF (10 mL) solution, and then potassium trimethylsilanolate (450 mg, 3.51 mmol) was added and reacted at 70°C for 1 h. The reaction was cooled to room temperature, and a hydrogen chloride-dioxane solution at a concentration of 4 M (1.2 mL) was added and then filtered. The resulting solid from filtration was washed with diethyl ether and dried to obtain the target compound **7c** (500 mg, 93.2%).
LC-MS: m/z (ESI): 230.1 [M+ H]$^+$

Step 3: Preparation of **7d**

Compound **7c** (100 mg, 0.44 mmol) and CDI (70 mg, 0.43 mmol) were dissolved in ultra-dried DMF (4 mL), and the reaction liquid was reacted at 70°C for 3 hours and then cooled to room temperature. 3a-1 (100 mg, 0.40 mmol) was added into the reaction vessel and reacted at room temperature for 16 hours. After the completion of the reaction, ethyl acetate (10 mL) and water (10 mL) were added for separation by extraction. The aqueous phase was extracted with ethyl acetate (10 mL) again, and the organic phases were combined. The organic phase was washed with saturated brine (10 mL) once, dried over anhydrous sodium sulphate and concentrated under reduced pressure. The residue was purified by column chromatography, to obtain compound **7d** (180 mg, yield 89.0%).

Step 4: Preparation of **7e**

Under nitrogen protection, **7d** (180 mg, 0.39 mmol) was dissolved in an anhydrous THF (5 mL) solution, and then water (1 mL) and acetic acid (1 mL) were added and reacted at room temperature for 2 h. The reaction solution was spun to remove tetrahydrofuran and water (10 mL) was added. A solid precipitated out and then was filtered. The resulting solid from filtration was washed with diethyl ether and dried to obtain the target compound **7e** (157 mg, 97.8%).
LC-MS: m/z (ESI): 386.0 [M+ H]$^+$

Step 5: Preparation of **compound 7**

At room temperature, compound **7e** (120 mg, 0.31 mmol) was added into a round bottom flask with ultra-dried DMSO (4 mL), and then 3c-1 (70 mg, 0.46 mmol) and AcOH (0.1 mL) were added into the reaction vessel in sequence. The reaction liquid was maintained at 70°C for reaction for 2 h, and then to the mixed reaction liquid, sodium triacetoxyborohydride (190 mg, 0.90 mmol) was added and reacted overnight. After the completion of the reaction, dichloromethane/methanol (v : v = 10/1, 5 mL) and a saturated $NaHCO_3$ aqueous solution (5 mL) were added for separation by extraction. The aqueous phase was extracted with dichloromethane/methanol (v : v = 10/1, 5 mL) again. The organic phase was combined and washed with saturated brine twice (5 mL × 2), dried over anhydrous sodium sulphate and concentrated under reduced pressure. The residue was separated and purified by silica gel column chromatography to obtain **compound 7** (82 mg, 54.6%).

LC-MS: m/z (ESI): 485.3 $[M+H]^+$
$^1H$ NMR (400 MHz, DMSO-$d_6$): δ 10.84 (s, 1H), 9.30-9.23 (m, 1H), 8.37-8.04 (m, 2H), 7.92-7.88 (m, 1H), 7.41-7.34 (m, 1H), 7.28 (s, 1H), 6.97-6.90 (m, 1H), 6.62 (s, 1H), 6.23-6.17 (m, 1H), 4.60-4.54 (m, 2H), 3.80 (s, 2H), 2.75 (s, 2H), 2.08-1.96 (br.s, 1H), 1.90-1.94 (m, 6H).

**Example 8**: Preparation of compound 8

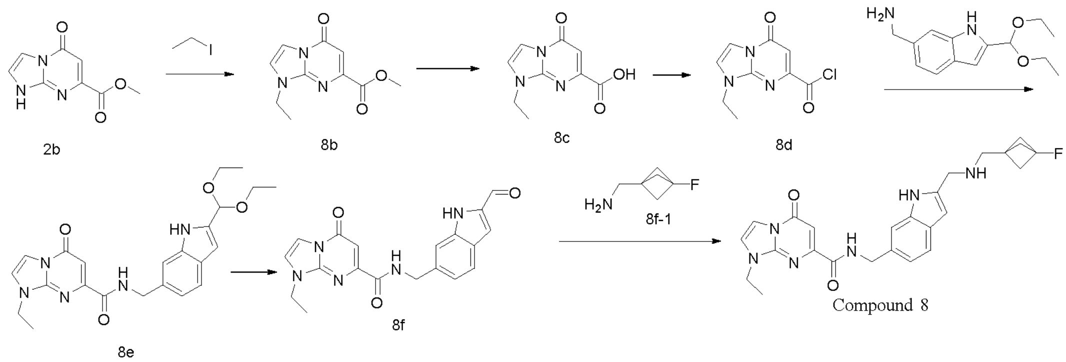


2b 8b 8c 8d 8e 8f Compound 8

Reference can be made to example 4 for the reaction conditions and operations to obtain **compound 8** (21.0 mg).

LC-MS: m/z (ESI): 463.3 $[M+H]^+$.
$^1H$ NMR (400 MHz, DMSO-d6): δ 11.03 (s, 1H), 9.22 (t, 1H), 9.03-8.86 (m, 2H), 7.80 (d, 1H), 7.75 (d, 1H), 7.51 (d, 1H), 7.40 (s, 1H), 7.08-7.03 (m, 1H), 6.57 (s, 1H), 6.46 (s, 1H), 4.58 (d, 2H), 4.34-4.21 (m, 4H), 2.11 (d, 6H), 1.40 (t, 3H).

**Example 9**: Preparation of compound 9

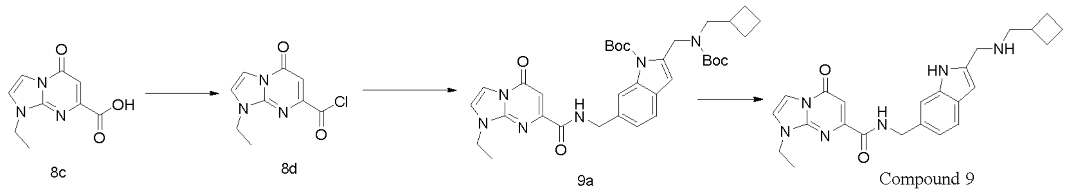

8c 8d 9a Compound 9

Reference can be made to example 5 for the reaction conditions and operations to obtain compound 9 (12.0 mg).

LC-MS: m/z (ESI): 433.3 $[M+H]^+$.
$^1H$ NMR (400 MHz, DMSO-d6): δ 11.03 (s, 1H), 9.27-9.18 (m, 1H), 8.28-8.67 (m, 2H), 7.81 (d, 1H), 7.75 (d, 1H), 7.50 (d, 1H), 7.39 (s, 1H), 7.08-7.01 (m, 1H), 6.56 (s, 1H), 6.46 (s, 1H), 4.58 (d, 2H), 4.33-4.19 (m, 4H), 3.08-2.93 (m, 2H), 2.66-2.57 (m, 1H), 2.11-2.00 (m, 2H), 1.93-1.70 (m, 4H), 1.40 (t, 3H).

**Example 10**: Preparation of compound 10

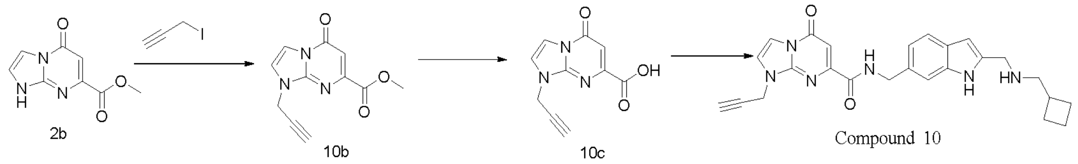

2b 10b 10c Compound 10

Reference can be made to example 5 for the reaction conditions and operations to obtain compound 10 (4.0 mg).

LC-MS: m/z (ESI): 443.3 $[M+H]^+$.
$^1$H NMR (400 MHz, DMSO-d6): δ 11.03 (s, 1H), 9.35 (t, 1H), 8.84-8.62 (m, 2H), 7.81-7.76 (m, 2H), 7.50 (d, 1H), 7.39 (s, 1H), 7.07-7.02 (m, 1H), 6.55 (s, 1H), 6.50 (s, 1H), 5.16 (d, 2H), 4.58 (d, 2H), 4.24 (s, 2H), 3.58 (t, 1H), 2.99 (d, 2H), 2.64-2.54 (m, 1H), 2.11-2.00 (m, 2H), 1.91-1.69 (m, 4H).

**Example 11**: Preparation of compound 11

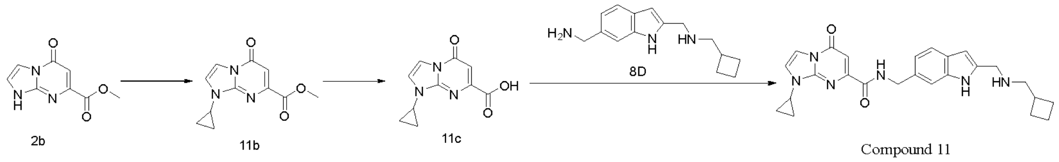

2b 11b 11c 8D Compound 11

Reference can be made to example 5 for the reaction conditions and operations to obtain t**he trifluoroacetate of compound 11** (6 mg).
Prep-HPLC conditions for the final product: instrument: waters 2767 preparative chromatographic column: SunFire@Prep C18 (19 mm × 150 mm); composition of mobile phases: mobile phase A: acetonitrile, and mobile phase B: water (containing 0.1% TFA).

LC-MS: m/z (ESI): 445.3 $[M+H]^+$
$^1$H NMR (400 MHz, DMSO-d6): δ 11.08 (s, 1H), 9.01 (t, 1H), 8.78 (s, 2H), 7.70 (d, 1H), 7.65 (d, 1H), 7.51 (d, 1H), 7.40 (s, 1H), 7.05 (d, 1H), 6.56 (s, 1H), 6.49(s, 1H), 4.60 (d, 2H), 4.25 (s, 2H), 3.82-3.69 (m, 1H), 3.04-2.95 (m, 2H), 2.69-2.55 (m, 1H), 2.09-2.01 (m, 2H), 2.89-1.69 (m, 4H), 1.15-1.05 (m, 4H).

**Example 12**: Preparation of compound 12

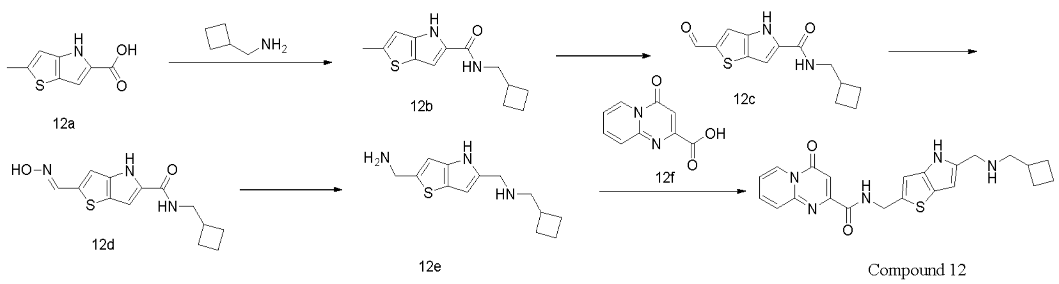

12a 12b 12c 12f 12d 12e Compound 12

Step 1: Preparation of **12b**

**12a** (3.81 g, 21.0 mmol) and carbonyldiimidazole (3.41 g, 21.0 mmol) were added to DMF (40 mL), reacted under stirring at room temperature for 1 hour, and to the system cyclobutylmethanamine hydrochloride (3.06 g, 25.2 mmol) was added for further reaction under stirring at room temperature for 18 hours. The reaction was quenched by addition of water (400 mL) to the reaction system, a large an amount of solids precipitated out and was filtered and the filter cake was washed with water (35 mL × 2). The filter cake was collected, and dried under vacuum under reduced pressure to obtain **12b** (4.6 g, yield 88%).
LC-MS: m/z (ESI): 249.1 $[M+H]^+$

Step 2: Preparation of **12c**

**12b** (1.15 g, 4.63 mmol) and DDQ (1.58 g, 6.95 mmol) were added to a mixed solvent of methanol (100 mL) and water (20 mL) and then reacted under stirring at 45°C for 4 hours. The reaction liquid was cooled to room temperature, poured into a saturated aqueous sodium bicarbonate solution (200 mL) with thorough stirring, a solid precipitated out, and the filter cake was removed by filtration through celite. The resulting mother liquor was concentrated and purified on a silica gel column to obtain the product **12c** (600 mg, yield 49%).
LC-MS: m/z (ESI): 263.1[M+ H]$^+$

Step 3: Preparation of **12d**

**12c** (500 mg, 1.91 mmol) and hydroxylamine hydrochloride (140 mg, 2.02 mmol) were added to a mixed solvent of tetrahydrofuran (5 mL), ethanol (15 mL) and water (2 mL) and reacted under stirring at room temperature for 2 hours. The reaction liquid was then concentrated directly and spin-dried to obtain crude compound **12d**, which was used directly in the next step.
LC-MS: m/z (ESI): 278.1[M+ H]$^+$

Step 4: Preparation of **12e**.

The crude product **12d** obtained in the previous step was added to 1,4-dioxane (30 mL) and stirred uniformly at room temperature, and then lithium aluminium hydride (2.5 M solution in toluene) (6 mL, 15 mmol) was slowly added dropwise to the system, and reacted under stirring at 100°C for 2 hours. The reaction was cooled to room temperature, water (15 mL) was slowly added dropwise to the reaction liquid followed by a 15% aqueous sodium hydroxide solution (15 mL), and stirred for 5 minutes. Water (45 mL) was added dropwise, then stirred for 10 minutes, filtered and the filtrate was spin-dried, to obtain crude compound **12e**, which was used directly in the next step.

Step 5: Preparation of **compound 12.**

**12f** (152 mg, 0.8 mmol) and carbonyldiimidazole (130 mg, 0.8 mmol) were added to DMF (5 mL), reacted under stirring at room temperature for 3 hours, and then the crude **12e** from the previous step was added to the system, and further stirred at room temperature for 3 hours. After the reaction liquid was directly concentrated and spin-dried, and then sent to prep-HPLC to obtain the **trifluoroacetate of compound 12** (72 mg, 10% overall yield for 3 steps).

Prep-HPLC conditions: instrument: Waters 2767 preparative liquid phase chromatographic instrument; chromatographic column: XBridge@Prep C18 (19 mm × 250 mm). The sample was dissolved in DMF and filtered with a 0.45 uM filter head to prepare a sample solution. Preparative chromatography conditions: a. mobile phase A: acetonitrile; b. water (containing 0.1% trifluoroacetic acid); mobile phase A, gradient elution, 10% to 50%; flow rate: 12 mL/minute; elution time: 20 minutes.

LC-MS: m/z (ESI): 420.1[M-H]$^-$
$^1$H NMR (400 MHz, DMSO-d6): δ 11.12(s, 1H), 9.32 (t, 1H), 9.02 (d, 1H), 8.64 (s, 2H), 8.13 - 7.98(m, 1H), 7.76(d, 1H), 7.51 - 7.40 (m, 1H), 7.03 (s, 1H), 6.89(s, 1H), 6.47(s, 1H), 4.65 (d, 2H), 4.16 (t, 2H), 2.99 - 2.86 (m, 2H), 2.62- 2.54 (m, 1H), 2.10 - 1.97 (m, 2H), 1.89 - 1.68 (m, 4H).

**Example 13**: Preparation of compound 13

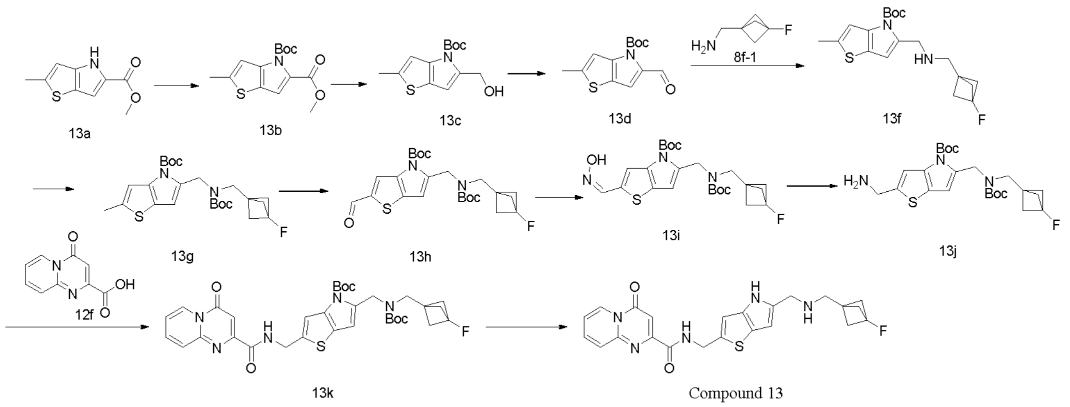


13a 13b 13c 13d 13f 13g 13h 13i 13j 12f 13k Compound 13

Step 1: Preparation of **13b**.

**13a** (1.95 g, 10.0 mmol), DMAP (122 mg, 1.0 mmol) and di-tert-butyl dicarbonate (2.61 g, 12.0 mmol) were added into acetonitrile (50 mL), and reacted under stirring at 45°C for 2 hours, and then the reaction liquid was concentrated. The residue was separated and purified by silica gel column chromatography to obtain **13b** (2.9 g, yield 98%).
LC-MS: m/z (ESI): 240.1 $[M-55]^+$

Step 2: Preparation of **13c**.

**13b** (2.8 g, 9.5 mmol) was added into tetrahydrofuran (100 mL), and stirred at -30°C for 10 minutes, and then to the system, DIBAL (15.8 mL, 23.7 mmol) was slowly added dropwise for further reaction under stirring at the temperature for 3 hours. The reaction liquid was poured into 0.5 M hydrochloric acid (50 mL) with thorough stirring, and then extracted with ethyl acetate (50 mL × 3). The organic phase was dried over anhydrous sodium sulphate, then filtered and concentrated. The residue was separated and purified by silica gel column, to obtain product **13c** (1.3 g, yield 51%).
LC-MS: m/z (ESI): 250.1 $[M-17]^+$

Step 3: Preparation of **13d**.

**13c** (900 mg, 3.37 mmol) and activated manganese dioxide (2.93 g, 33.7 mmol) were added to dichloromethane (20 mL) and stirred at room temperature for 5 hours. The reaction liquid was filtered through celite, and the filtrate was concentrated. The residue was separated and purified by silica gel column to obtain the product **13d** (830 mg, yield 93%).
LC-MS: m/z (ESI): 210.1$[M-55]^+$

Step 4: Preparation of **13f**.

**13d** (830 mg, 3.13 mmol) and 8f-1 (709 mg, 4.7 mmol) were added to N,N-dimethylacetamide (20 mL), and then glacial acetic acid (0.1 mL) was dropwise added, and reacted under stirring at 65°C for 3 hours. Then the reaction was cooled to room temperature, and to the system, sodium triacetoxyborohydride (1.66 g, 7.82 mmol) was added for further reaction under stirring at room temperature for 10 hours. To the reaction liquid, 0.5 M hydrochloric acid (20 mL) was slowly added dropwise and stirred for 5 minutes, then extracted with ethyl acetate (50 mL × 3). The organic phase was dried over anhydrous sodium sulphate dried, then filtered. The filtrate was concentrated, and the residue was separated and purified by silica gel column, to obtain product **13f** (911 mg, yield 80%).

Step 5: Preparation of **13g**.

**13f** (911 mg, 2.5 mmol), di-tert-butyl dicarbonate (610 mg, 2.8 mmol) and DMAP (30 mg, 0.25 mmol) were added to dichloromethane (30 mL), and reacted under stirring at room temperature for 1 hour. The reaction liquid was concentrated and the residue was separated and purified by silica gel column, to obtain the product **13g** (1.1 g, yield 94%).

Step 6: Preparation of **13h**.

**13g** (1.1 g, 2.37 mmol) and DDQ (681 mg, 3.0 mmol) were added to a mixed solvent of methanol (30 mL) and water (5 mL), and reacted under stirring at room temperature for 3 hours. The reaction liquid was then added to a saturated aqueous sodium bicarbonate solution (100 mL), stirred for 5 minutes, and then extracted with ethyl acetate (50 mL × 3).

The organic phase was dried over anhydrous sodium sulphate and filtered. The filtrate was concentrated and the residue was separated and purified by silica gel column, to obtain the product **13h** (550 mg, yield 48%).
LC-MS: m/z (ESI): 479.1 [M+ H]$^+$

Step 7: Preparation of **13i**.

**13h** (550 mg, 1.15 mmol) and hydroxylamine hydrochloride (159 mg, 2.3 mmol) were added to a mixed solvent of tetrahydrofuran (50 mL), ethanol (15 mL) and water (1.5 mL), and reacted under stirring at room temperature for 3 hours. To the system, water (50 mL) was added with thorough stirring, a solid precipitated out and was filtered to obtain the solid, which was dried to obtain the product **13i** (450 mg, yield 79%).
LC-MS: m/z (ESI): 494.1 [M+ H]$^+$

Step 8: Preparation of **13j**.

**13i** (100 mg, 0.2 mmol) and a zinc powder (66 mg, 1.02 mmol) were added to glacial acetic acid (5 mL), reacted under stirring at room temperature for 6 hours, and then the reaction was filtered over celite. The filtrate was concentrated and spin-dried, to obtain 2j acetate, which was extracted with dichloromethane (50 mL × 3). The organic phase was washed with a saturated aqueous potassium carbonate solution, and the organic phase was dried over anhydrous sodium sulphate, then filtered and the filtrate was concentrated to obtain the product **13j** (50 mg, yield 51%).

Step 9: Preparation of **13k**.

**13j** (50 mg, 0.1 mmol) and 12f (19 mg, 0.1 mmol) were added to N,N-dimethylacetamide (4 mL) and HATU (38 mg, 0.1 mmol) and DIPEA (26 mg, 0.2 mmol) were added, and reacted under stirring at room temperature for 1 hour. Water (20 mL) was added to the system, extracted with ethyl acetate (50 mL × 3). The organic phase was dried over anhydrous sodium sulphate, filtered, and the filtrate was concentrated. The residue was separated and purified by silica gel column to obtain the product **13k** (50 mg, yield 73%).

Step 10: Preparation of **compound 13.**

**13k** (50 mg, 0.076 mmol) was added to a mixed solvent of dichloromethane (3 mL) and trifluoroacetic acid (0.6 mL), reacted under stirring at room temperature for 24 hours, and concentrated. The residue was separated and purified by preparative thin layer chromatography on silica gel to obtain **compound 13** (6 mg, yield 17%).

LC-MS: m/z (ESI): 450.0 [M-H]$^-$
$^1$H NMR (400 MHz, $CD_3OD$): δ 8.97(d, 1H),7.92 - 7.85(m, 1H), 7.73(d, 1H), 7.33 - 7.26 (m, 1H), 7.00 (s, 1H), 6.85(s, 1H), 6.15(s, 1H), 4.65 (s, 2H), 3.79 (s, 2H), 2.82 (s, 2H), 1.88 (d, 6H).

Preparation of trifluoroacetate of compound 13: Compound 13 (4 mg, 0.00887 mmol) was added to DCM (1 mL) and then trifluoroacetic acid (0.01 mL) was added, stirred at room temperature for 5 minutes, and concentrated to dryness under reduced pressure to obtain the trifluoroacetate of compound 13 (5 mg, yield 99%).

**Example 14:** Preparation of compound 14

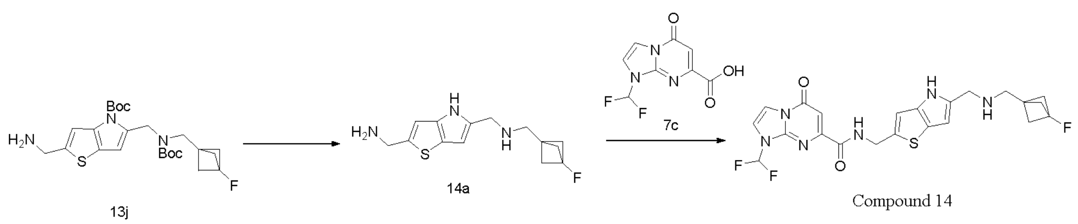

Step 1: Preparation of **14a**

**13j** (0.5 g, 1.04 mmol) was added to dichloromethane (10 mL), and a 4 M hydrogen chloride 1,4-dioxane solution (10 mL) was slowly added dropwise to the system and reacted under stirring at room temperature for 12 hours. The

reaction liquid was concentrated, and the residue was separated and purified by silica gel column chromatography to obtain **14a** (195 mg, 60.4%).

Step 2: Preparation of **compound 14**

Substrate **7c** (23 mg, 0.1 mmol) and carbonyldiimidazole (18 mg, 0.11 mmol) were added to DMF (1 mL) and reacted stirring at 70°C for 4 hours. Then the reaction was brought to room temperature, and to the system 14a (28 mg, 0.1 mmol) was added, and reacted under stirring at room temperature overnight. The reaction was concentrated and subjected to prep-HPLC to obtain the **trifluoroacetate** of **compound 14** (10 mg, yield 20%).

Prep-HPLC conditions: instrument: Waters 2767 preparative liquid phase chromatographic instrument; chromatographic column: XBridge@Prep C18 (19 mm × 250 mm). The sample was dissolved in DMF and filtered with a 0.45 uM filter head to prepare a sample solution. Preparative chromatography conditions: a. mobile phase A: acetonitrile; b. water (containing 0.1% trifluoroacetic acid); mobile phase A, gradient elution, 10% to 50%; flow rate: 12 mL/minute; elution time: 20 minutes.

LC-MS: m/z (ESI): 489.5 $[M- H]^-$

$^1H$ NMR (400 MHz, DMSO-d6): δ 11.10 (s, 1H), 9.37 (t, 1H), 8.82 (br, 2H), 8.17 (t, 1H), 8.10 (d, 1H), 7.91 (d, 1H), 7.02 (s, 1H), 6.60 (s, 1H),6.48 (s, 1H), 4.65 (d, 2H), 4.20 (br, 2H), 3.25 (br, 2H), 2.10 (d, 6H).

**Example 15:** Preparation of compound 15

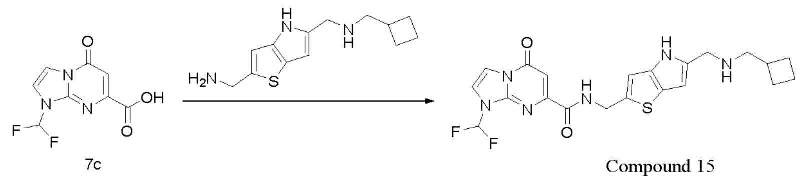

7c

Compound 15

Reference can be made to example 12 for the reaction conditions and operations to obtain **compound 15** (110.0 mg).

LC-MS: m/z (ESI): 376.0 $[M+ H]^+$.

$^1H$ NMR (400 MHz, DMSO-d6) δ 11.16 (s, 1H), 9.38 (t, 1H), 8.34 - 8.03 (m, 2H), 7.92 (d, 1H), 7.02 (s, 1H), 6.60 (s, 1H), 6.48 - 6.45 (m, 1H), 4.65 (d, 2H), 4.14 (s, 2H), 2.91 (d, 2H), 2.09 - 1.98 (m, 2H), 1.90 - 1.71 (m, 4H).

**Example 16:** Preparation of compound 16

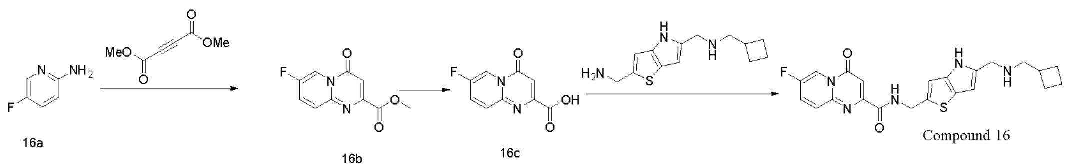

16a

16b

16c

Compound 16

Reference can be made to example 1 for the reaction conditions and operations to obtain the **compound 16 trifluoroacetate** (26.0 mg).

Prep-HPLC conditions for the final product: instrument: waters 2767 preparative chromatographic column: SunFire@ Prep C18 (19 mm × 150 mm); composition of mobile phases: mobile phase A: acetonitrile, and mobile phase B: water (containing 0.1% TFA).

LC-MS: m/z (ESI): $440.1[M+H]^+$

$^1H$ NMR (400 MHz, DMSO-d6): δ11.11-11.06 (m, 1H), 9.37-9.30 (m, 1H),9.04-8.99 (m, 1H), 8.68-8.53 (m, 2H),8.23-8.16 (m, 1H), 7.87-7.80 (m, 1H), 7.03 (s, 1H),6.91 (s, 1H), 6.49-6.45 (m, 1H), 4.68-4.62 (m, 2H), 4.19-4.11 (m, 2H),2.97-2.89 (m, 2H), 2.63-2.53(m, 1H), 2.08-1.99 (m, 2H), 1.90-1.68 (m, 4H).

**Example 17:** Preparation of compound 17

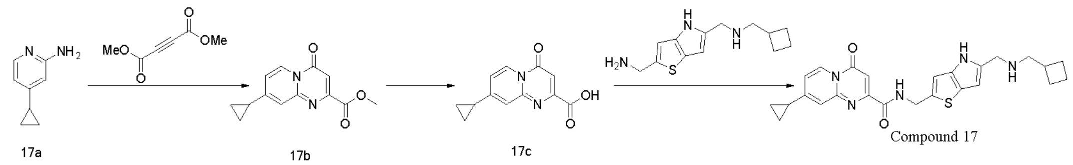

17a 17b 17c Compound 17

Reference can be made to example 1 for the reaction conditions and operations to obtain the **compound 17 trifluoroacetate** (17.0 mg).

Prep-HPLC conditions for the final product: instrument: waters 2767 preparative chromatographic column: SunFire@ Prep C18 (19 mm × 150 mm); composition of mobile phases: mobile phase A: acetonitrile, and mobile phase B: water (containing 0.1% TFA).

LC-MS: m/z (ESI): 462.2[M+H]$^+$
$^1$H NMR (400 MHz, DMSO-d6): δ11.09 (s, 1H), 9.25-9.14 (m, 1H),8.90-8.83 (m, 1H), 8.71-8.50 (m, 2H), 7.48-7.43 (m, 1H), 7.20-7.13 (m, 1H), 7.03 (s, 1H),6.77 (s, 1H), 6.47 (s, 1H), 4.68-4.59 (m, 2H), 4.19-4.12 (m, 2H),2.98-2.88 (m, 2H), 2.64-2.53(m, 1H), 2.26-2.15(m, 1H), 2.09-1.98 (m, 2H), 1.92-1.68 (m, 4H), 1.28-1.19 (m, 2H), 1.03-0.95 (m, 2H).

**Example 18:** Preparation of compound 18

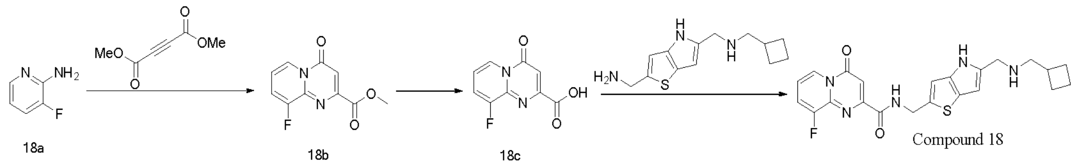

18a 18b 18c Compound 18

Reference can be made to example 1 for the reaction conditions and operations to obtain **compound 18** (15.0 mg).

LC-MS: m/z (ESI): 440.1[M+H]$^+$
$^1$H NMR (400 MHz, DMSO-d6): δ10.85 (s, 1H),9.15-9.07 (m, 1H), 8.84-8.77 (m, 1H),8.01-7.92 (m, 1H), 7.44-7.34 (m, 1H), 6.96 (s, 1H),6.92 (s, 1H), 6.10 (s, 1H), 4.67-4.59 (m, 2H), 3.67 (s, 2H),2.48(s, 2H), 2.41-2.30(m, 1H), 2.00-1.90 (m, 2H), 1.87-1.71 (m, 2H), 1.66-1.54 (m, 2H).

**Example 19:** Preparation of compound 19

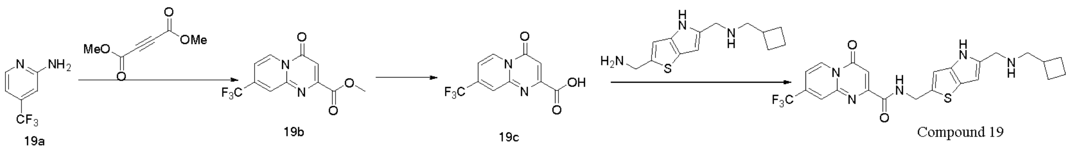

19a 19b 19c Compound 19

Reference can be made to example 1 for the reaction conditions and operations to obtain **compound 19** (5.0 mg).

LC-MS: m/z (ESI): 490.2[M+H]$^+$
$^1$H NMR (400 MHz, DMSO-d6): δ10.89 (s, 1H),9.88-9.81 (m, 1H), 8.45-8.37 (m, 1H),7.62 (s, 1H), 7.24-7.18 (m, 1H), 6.96 (s, 1H),6.60 (s, 1H), 6.14 (s, 1H), 4.67-4.62 (m, 2H), 3.69 (s, 2H),2.47(s, 2H), 2.42-2.35(m, 1H), 2.02-1.90 (m, 2H), 1.86-1.71 (m, 2H), 1.68-1.55 (m, 2H).

**Example 20:** Preparation of compound 20

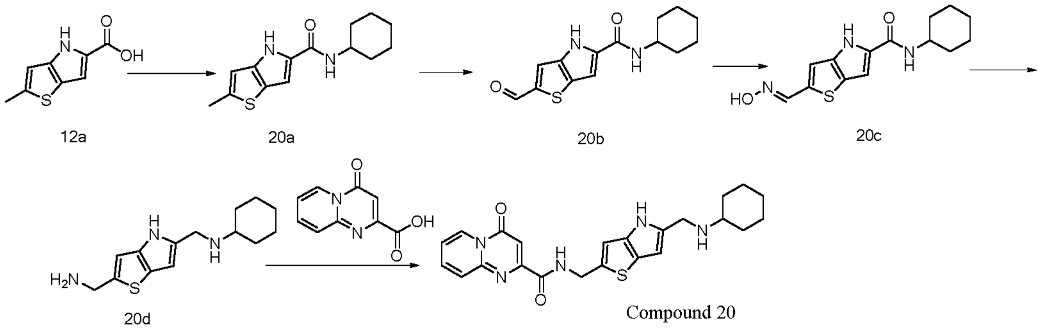

12a 20a 20b 20c 20d Compound 20

Reference can be made to example 12 for the reaction conditions and operations to obtain **compound 20** (30.0 mg).

LC-MS: m/z (ESI): 436.1[M+H]$^+$

$^1$H NMR (400 MHz, DMSO-d6): δ10.83 (s, 1H), 9.24-9.17 (m, 1H), 9.03-8.97 (m, 1H), 8.09-8.02(m, 1H),7.80-7.74 (m, 1H), 7.48-7.40 (m, 1H), 6.92 (s, 1H),6.89 (s, 1H), 6.10 (s, 1H), 4.65-4.58 (m, 2H), 3.73 (s, 2H), 2.39-2.29 (m, 1H), 1.84-1.75(m, 2H), 1.68-1.58 (m, 2H), 1.55-1.46 (m, 1H), 1.25-1.07(m,3H), 1.06-0.94(m, 2H).

**Example 21:** Preparation of compound 21

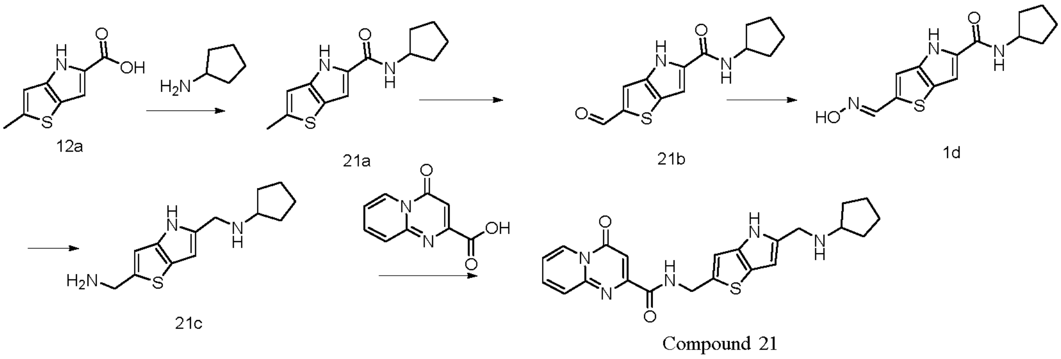

12a 21a 21b 1d 21c Compound 21

Reference can be made to example 12 for the reaction conditions and operations to obtain **compound 21** (179.0 mg):

LC-MS: m/z (ESI): 337.3 [M+ H]$^+$.

$^1$H NMR (400 MHz, DMSO-d6): δ 10.86 (s, 1H), 9.23 (t, 1H), 9.01 (d, 1H), 8.10 - 8.02 (m, 1H), 7.77 (d, 1H), 7.48 - 7.40 (m, 1H), 6.92 (s, 1H), 6.89 (s, 1H), 6.12 (s, 1H), 4.63 (d, 2H), 3.69 (s, 2H), 3.02 - 2.92 (m, 1H), 1.74 - 1.56 (m, 4H), 1.49 - 1.37 (m, 2H), 1.35 - 1.25 (m, 2H).

**Example 22:** Preparation of compound 22

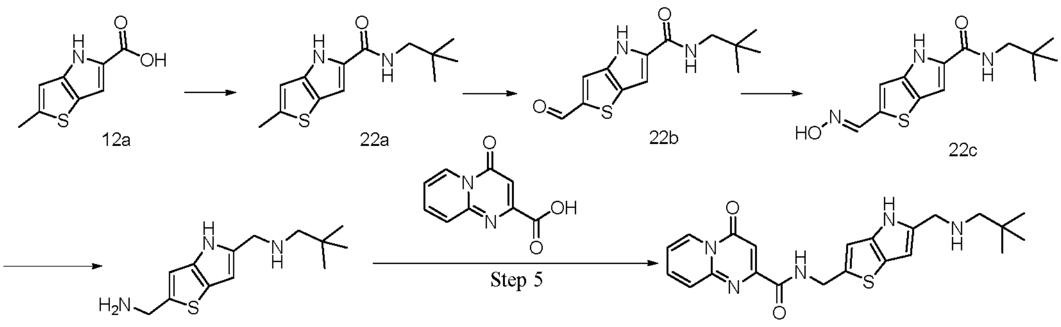


Compound 22

Reference can be made to example 12 for the reaction conditions and operations to obtain **compound 22** (110.0 mg).

LC-MS: m/z (ESI): 337.3 $[M+H]^+$.

$^1H$ NMR (400 MHz, DMSO-d6): δ 10.84 (s, 1H), 9.22 (t, 1H), 9.01 (d, 1H), 8.09 - 8.02 (m, 1H), 7.77 (d, 1H), 7.47 - 7.42 (m, 1H), 6.94 (s, 1H), 6.89 (s, 1H), 6.13 (s, 1H), 4.63 (d, 2H), 3.73 (s, 1H), 2.24 (s, 2H), 1.24 (s, 2H), 0.85 (s, 9H).

**Biological test example 1:**

Cell: MOLM-13 Cell Line
Cell culture medium: RPMI-1640 + 10% FBS + 1% P/S
Frozen stock solution: 50% RPMI-1640, 40% FBS, 10% (V/V) DMSO
Experimental procedure
Cell culture: Cells were cultured with RPMI-1640 + 10% FBS + 1% P/S in a $CO_2$ incubator at 37°C and passaged once 3-4 days.
Cell plating: Cells were plated when they grow to a logarithmic growth phase. The cells were collected and the cell density was adjusted to $6 \times 10^4$ cells/mL; then the cell suspension is then poured into a loading slot and added into a 96-well cell culture plate at 50 μL/well (3000 cells/well viable cells). Note: the cells in the loading slot were well mixed while plating; cell information and plating date were labelled on the 96-well plates. NC wells (control wells) and BG wells (background wells) were provided.
Cell administration: Compounds were diluted with RPMI-1640 + 10% FBS + 1% P/S + 0.2% DMSO + 100 ng/mL IFN-r gradient at a maximum detection concentration of 10 μM. 50 μL/well of the compounds were added to the 96-well plate in which the cells were already plated. Incubation was carried out at 37°C, 5% $CO_2$ for a period of time.
Cell proliferation test: After incubation in the incubator for a period of time, 50 μL of Cellriter-Glo Reagent per well was added.

The plate was shaken at room temperature (500 rpm), incubated for 10 min in the dark, and then the absorbance was measured with a microplate reader.

Data processing

The mean background value was calculated.

Calculated inhibition ratio = [(control well absorbance - experimental well absorbance)/(control well absorbance - background well absorbance)] × 100%

Statistic analysis

For all results mean and standard error (Mean ± SEM) were recorded, and statistical analysis was performed and the $IC_{50}$ values of the samples were calculated using the Graphpad Prism software. Specific data were presented in the form of graphs.

The biological test results are as follows in Table 1:

**Table 1 $IC_{50}$ of compounds on MOLM-13 cells**

| Compound | $IC_{50}$ |
|---|---|
| Trifluoroacetate of compound 1 | A |
| Trifluoroacetate of compound 2 | B |
| Compound 3 | A |
| Compound 4 | A |
| Trifluoroacetate of compound 5 | B |
| Trifluoroacetate of compound 6 | B |
| Compound 7 | A |
| Trifluoroacetate of compound 8 | A |
| Trifluoroacetate of compound 9 | B |
| Trifluoroacetate of compound 10 | A |
| Trifluoroacetate of compound 11 | B |
| Trifluoroacetate of compound 12 | A |
| Trifluoroacetate of compound 13 | A |
| Trifluoroacetate of compound 14 | B |
| Trifluoroacetate of compound 16 | B |
| Trifluoroacetate of compound 17 | B |
| Compound 18 | B |
| Compound 20 | B |
| Compound 22 | B |
| Notes: A < 2 μM, 2 μM ≤ B < 10 μM<br>Conclusion: The compounds of the present invention have a good inhibitory effect on MOLM-13 cells, for example, the trifluoroacetates of Compound 3, Compound 4, Compound 7 and compound 13 have $IC_{50}$ of 0.23 μM, 0.16 μM, 0.61 μM, and 0.56 μM, respectively. | |

**Biological test example 2:**

**Pharmacokinetic test in mice**

**2.1 Experimental animals:** male C57 mice, 18-20 g, 6 mice/compound or 12 mice/compound.

**2.2 Experimental design:** the C57 mice were randomly grouped according to their body weights. The animals were fasted with water available for 12 to 14 h one day before the administration, and were fed 4 h after the administration.

**Table 2-1 Administration information**

| Group | Quantity | Administration information | | | | | |
|---|---|---|---|---|---|---|---|
| | Male | Test compound | Administration dosage (mg/kg) | Administ ration concentra tion (mg/mL) | Administration volume (mL/kg) | Collected samples | Mode of administration |
| G1 | 6 | Compound 7 | 2.5 | 0.5 | 5 | Plasma | Intravenously |
| G2 | 6 | Compound 7 | 10 | 1 | 10 | Plasma | Intragastrically |

(continued)

| Group | Quantity | Administration information | | | | | |
|---|---|---|---|---|---|---|---|
| | Male | Test compound | Administration dosage (mg/kg) | Administ ration concentra tion (mg/mL) | Administration volume (mL/kg) | Collected samples | Mode of administration |
| G3 | 6 | Compound 23 in WO 2021111124 | 2.5 | 0.5 | 5 | Plasma | Intravenously |
| G4 | 6 | Compound 23 in WO 2021111124 | 10 | 1 | 10 | Plasma | Intragastrically |
| G5 | 3 | Compound 13 | 2.5 | 0.5 | 5 | Plasma | Intravenously |
| G6 | 3 | Compound 13 | 10 | 1 | 10 | Plasma | Intragastrically |

Vehicle for intravenous administration: 5% DMA + 5% Solutol + 90% Saline; vehicle for intragastric administration: 0.5% MC;

(DMA: dimethylacetamide; Solutol: polyethylene glycol-15-hydroxystearate; Saline: physiological saline; MC: Methyl cellulose solution;)

Before and after the administration, 0.06 mL of blood was taken from the orbit of the animals under isoflurane anesthesia and placed in an EDTAK2 centrifuge tube. The blood was centrifuged at 5000 rpm and 4°C for 10 min to collect the plasma. The blood collection time points for the intravenous administration group and intragastric administration group were 0 min, 5 min, 15 min, 30 min, 1 h, 2 h, 4 h, 7 h and 24 h. Before analysis and detection, all samples were stored at -80°C.

**Table 2-2 Pharmacokinetic parameters of test compounds in plasma of mice**

| Test compounds | Mode of administration | CL (mL/min/kg) | $Vd_{ss}$ (L/kg) | $AUC_{0-t}$ (hr*ng/mL) | F (%) |
|---|---|---|---|---|---|
| Compound 7 | i.v. (2.5 mg/kg) | 24.1 | 1.48 | 1718 | - |
| Compound 7 | i.g. (10 mg/kg) | - | - | 4476 | 65.1 |
| Compound 13 | i.g. (10 mg/kg) | - | - | 2881 | 92.7 |
| Compound 23 in WO 2021111124 | i.v. (2.5 mg/kg) | 57.0 | 2.21 | 730 | - |
| Compound 23 in WO 2021111124 | i.g. (10 mg/kg) | - | - | 923 | 31.6 |
| -: not applicable. | | | | | |

Control compound: Compound 23 in WO 2021111124, having a structure of:

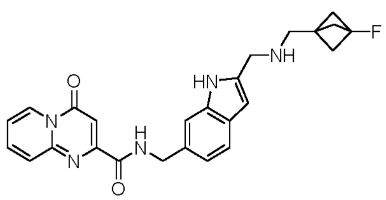

Conclusion: The compounds of the present invention have better pharmacokinetic properties in mice, such as $AUC_{0-t}$ and bioavailability (F), both of which are superior to those of the control compound.

**Biological test example 3: Test for hERG potassium ion channel**

**Experimental platform:** electrophysiological manual patch-clamp system

**Cell line:** Chinese hamster ovary (CHO) cell lines stably expressing hERG potassium ion channel

**Experimental method:** in CHO (Chinese Hamster Ovary) cells stably expressing hERG potassium channel, whole cell patch-clamp technique was used to record hERG potassium channel current at room temperature. The glass microelectrode was made of a glass electrode blank (BF150-86-10, Sutter) by a puller. The tip resistance after filling the liquid in the electrode was about 2-5 MΩ. The glass microelectrode can be connected to the patch-clamp amplifier by inserting the glass microelectrode into an amplifier probe. The clamping voltage and data recording were controlled and recorded by the pClamp 10 software through a computer. The sampling frequency was 10 kHz, and the filtering frequency was 2 kHz. After the whole cell records were obtained, the cells were clamped at -80 mV, and the step voltage that induced the hERG potassium current ($I_{hERG}$) was depolarised from -80 mV to +20 mV for 2 s, then repolarised to -50 mV, and returned to -80 mV after 1 s. This voltage stimulation was given every 10 s, and the administration process was started after the hERG potassium current was confirmed to be stable (at least 1 minute). The compound was administered for at least 1 minute at each test concentration, and at least 2 cells ($n \geq 2$) were tested at each concentration.

**Data processing:** Data analysis processing was carried out by using pClamp 10, GraphPad Prism 5 and Excel software. The inhibition degree of hERG potassium current (peak value of hERG tail current induced at -50 mV) at different compound concentrations was calculated by the following formula:

$$\text{Inhibition\%} = [1 - (I / Io)] \times 100\%$$

where Inhibition% represents the percentage of inhibition of hERG potassium current by the compound, and I and Io represent the amplitude of hERG potassium current after and before the administration, respectively.

Compound $IC_{50}$ was calculated using GraphPad Prism 5 software by fitting according to the following equation:

$$Y = \text{Bottom} + (\text{Top-Bottom})/(1 + 10 \wedge ((\text{LogIC}_{50} - X) * \text{HillSlope}))$$

Among the equation, X represents the Log value of the tested concentration of the test sample, Y represents the inhibition percentage at the corresponding concentration, and Bottom and Top represent the minimum and maximum inhibition percentage, respectively.

**Experimental results:** The $IC_{50}$ values for the inhibitory effect of the test compounds on hERG potassium channel current are shown in Table 4 below:

**Table 4**

| Test compounds | $IC_{50}$ (μM) |
|---|---|
| Compound 12 | > 30 |
| Compound 13 | > 30 |

Conclusion: The compounds of the present invention have weaker hERG inhibitory effects.

**Biological test example 4: CYP450 enzyme inhibition test**

The purpose of this study was to evaluate the effect of the test compound on the activity of five isoenzymes (CYP1A2, CYP2C9, CYP2C19, CYP2D6 and CYP3A4) of human liver microsomal cytochrome P450 (CYP) by using an *in vitro* testing system. The specific probe substrates of CYP450 isoenzymes were incubated with human liver microsomes and test compounds of different concentrations, and reduced nicotinamide adenine dinucleotide phosphate (NADPH) was added to initiate the reaction. After the completion of the reaction, the sample was treated and liquid chromatography-tandem mass spectrometry (LC-MS/MS) was used to quantitatively detect metabolites produced by specific substrates, changes in CYP enzyme activity were determined, and IC50 value was calculated to evaluate the inhibitory potential of the test compound on each CYP enzyme subtype.

Experimental conclusion: the compounds of the present invention have weaker inhibitory effects on CYP enzymes.

## Claims

1. A compound or a stereoisomer, tautomer, deuterate, solvate, prodrug, metabolite, pharmaceutically acceptable salt, or co-crystal thereof, **characterised in that** the compound is selected from a compound of general formula (I),

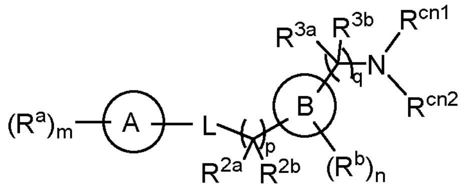

(I)

ring A is selected from

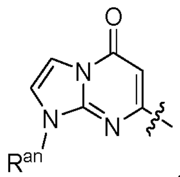
,
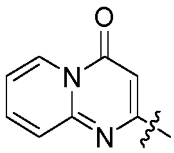
,
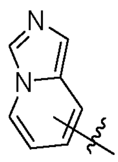
,
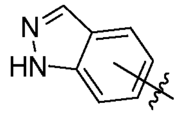
,

or

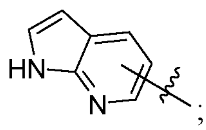
;

L is selected from

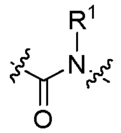

or 5- to 6-membered heteroaryl, L is connected with ring A at the left side, the heteroaryl is optionally further substituted with 0 to 3 substituents selected from H, halogen, OH, cyano, $NH_2$, $C_{1-6}$ alkyl, halogen-substituted-$C_{1-6}$ alkyl, hydroxyl-substituted-$C_{1-6}$ alkyl, cyano-substituted-$C_{1-6}$ alkyl, or $C_{1-6}$ alkoxy, the heteroaryl contains 1 to 3 heteroatoms selected from O, S or N;

$R^{an}$ is selected from H, $C_{1-6}$ alkyl, $C_{3-12}$ carbocyclyl or 3- to 12-membered heterocyclyl, the alkyl, carbocyclyl or heterocyclyl is optionally further substituted with 0 to 4 substituents selected from H, halogen, OH, =O, cyano, $NH_2$, $C_{1-6}$ alkyl, $C_{2-4}$ alkenyl, $C_{2-4}$ alkynyl, -$C_{2-4}$ alkynyl-$C_{3-6}$ cycloalkyl, halogen-substituted-$C_{1-6}$ alkyl, hydroxyl-substituted-$C_{1-6}$ alkyl, cyano-substituted-$C_{1-6}$ alkyl, $C_{1-6}$ alkoxy, $C_{3-12}$ cycloalkyl, phenyl, 3- to 12-membered heterocycloalkyl or 5- to 12-membered heteroaryl, the heterocyclyl, heterocycloalkyl or heteroaryl contains 1 to 3 heteroatoms selected from O, S or N;

ring B is selected from 5- to 10-membered heterocyclyl, wherein the heterocyclyl contains 1 to 5 heteroatoms selected from O, S or N;

$R^a$ or $R^b$ is each independently selected from H, halogen, cyano, OH, =O, $C_{1-6}$ alkyl, $C_{1-6}$ alkoxy, or $C_{3-6}$ cycloalkyl, the alkyl, alkoxy or cycloalkyl is optionally further substituted with 0 to 4 substituents selected from H, halogen, OH, =O, cyano, $NH_2$, $C_{1-6}$ alkyl, halogen-substituted-$C_{1-6}$ alkyl, hydroxyl-substituted-$C_{1-6}$ alkyl, cyano-substituted-$C_{1-6}$ alkyl, $C_{1-6}$ alkoxy or $C_{3-6}$ cycloalkyl;

alternatively, $R^{an}$ and $R^a$ together with the atom to which they are attached form a 5- to 6-membered heterocycle, the heterocycle is optionally further substituted with 0 to 4 substituents selected from H, halogen, OH, =O, cyano, $NH_2$, $C_{1-6}$ alkyl, halogen-substituted-$C_{1-6}$ alkyl, hydroxyl-substituted-$C_{1-6}$ alkyl, cyano-substituted-$C_{1-6}$ alkyl, $C_{1-6}$ alkoxy or $C_{3-6}$ cycloalkyl, the heterocycle contains 1 to 3 heteroatoms selected from O, S or N;

$R^1$ is selected from H, $C_{1-6}$ alkyl or $C_{3-6}$ cycloalkyl, the alkyl or cycloalkyl is optionally further substituted with 0 to 4

substituents selected from H, halogen, OH, =O, cyano, $NH_2$, $C_{1-6}$ alkyl, halogen-substituted-$C_{1-6}$ alkyl, hydroxyl-substituted-$C_{1-6}$ alkyl, cyano-substituted-$C_{1-6}$ alkyl, $C_{1-6}$ alkoxy or $C_{3-6}$ cycloalkyl;

$R^{2a}$, $R^{2b}$, $R^{3a}$ and $R^{3b}$ are each independently selected from H, halogen, cyano, OH, =O, $C_{1-6}$ alkyl, $C_{1-6}$ alkoxy or $C_{3-6}$ cycloalkyl, the alkyl, alkoxy or cycloalkyl is optionally further substituted with 0 to 4 substituents selected from H, halogen, OH, =O, cyano, $NH_2$, $C_{1-6}$ alkyl, halogen-substituted-$C_{1-6}$ alkyl, hydroxyl-substituted-$C_{1-6}$ alkyl, cyano-substituted-$C_{1-6}$ alkyl, $C_{1-6}$ alkoxy or $C_{3-6}$ cycloalkyl;

alternatively, $R^{2a}$ and $R^{2b}$, $R^{3a}$ and $R^{3b}$ together with the atom to which they are attached form $C_{3-6}$ carbocyclyl or 3- to 6-membered heterocyclyl, respectively, the carbocyclyl or heterocyclyl is optionally further substituted with 0 to 4 substituents selected from H, halogen, OH, =O, cyano, $NH_2$, $C_{1-6}$ alkyl, halogen-substituted-$C_{1-6}$ alkyl, hydroxyl-substituted-$C_{1-6}$ alkyl, cyano-substituted-$C_{1-6}$ alkyl, $C_{1-6}$ alkoxy or $C_{3-6}$ cycloalkyl, the heterocyclyl contains 1 to 3 heteroatoms selected from O, S or N;

$R^{cn1}$ and $R^{cn2}$ are each independently selected from H, halogen, cyano, OH, =O, $C_{1-6}$ alkyl, $C_{1-6}$ alkoxy, $C_{3-10}$ carbocyclyl, 3- to 10-membered heterocyclyl or

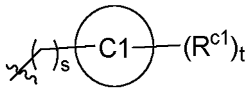
,

the alkyl, alkoxy, carbocyclyl or heterocyclyl is optionally further substituted with 0 to 4 substituents selected from H, halogen, OH, =O, cyano, $NH_2$, $C_{1-6}$ alkyl, halogen-substituted-$C_{1-6}$ alkyl, hydroxyl-substituted-$C_{1-6}$ alkyl, cyano-substituted-$C_{1-6}$ alkyl, $C_{1-6}$ alkoxy or $C_{3-6}$ cycloalkyl, the heterocyclyl contains 1 to 3 heteroatoms selected from O, S or N;

ring C1 is selected from $C_{3-12}$ carbocyclyl or 3- to 12-membered heterocyclyl, and the heterocyclyl contains 1 to 3 heteroatoms selected from O, S or N;

$R^{c1}$ is selected from H, halogen, cyano, OH, =O, $C_{1-6}$ alkyl, $C_{1-6}$ alkoxy, $C_{3-12}$ carbocyclyl or 3- to 12-membered heterocyclyl, and the alkyl, alkoxy, carbocyclyl or heterocyclyl is optionally further substituted with 0 to 4 substituents selected from H, halogen, OH, =O, cyano, $NH_2$, $C_{1-6}$ alkyl, halogen-substituted-$C_{1-6}$ alkyl, hydroxyl-substituted-$C_{1-6}$ alkyl, cyano-substituted-$C_{1-6}$ alkyl, $C_{1-6}$ alkoxy or $C_{3-6}$ cycloalky, the heterocyclyl contains 1 to 3 heteroatoms selected from O, S or N;

alternatively, $R^{cn1}$ and $R^{cn2}$ together with the atom to which they are attached form 3- to 12-membered heterocyclyl, and the heterocyclyl is optionally further substituted with 0 to 4 substituents selected from H, halogen, OH, =O, cyano, $NH_2$, $C_{1-6}$ alkyl, halogen-substituted-$C_{1-6}$ alkyl, hydroxyl-substituted-$C_{1-6}$ alkyl, cyano-substituted-$C_{1-6}$ alkyl, $C_{1-6}$ alkoxy or $C_{3-6}$ cycloalkyl, the heterocyclyl contains 1 to 3 heteroatoms selected from O, S or N;

m is selected from 0, 1, 2 or 3;
n is selected from 0, 1, 2, 3, 4, 5, 6 or 7;
p is selected from 0, 1, 2 or 3;
q is selected from 0, 1, 2 or 3;
s is selected from 0, 1, 2 or 3;
t is selected from 0, 1, 2, 3, 4, 5, 6 or 7.

2. The compound or the stereoisomer, tautomer, deuterate, solvate, prodrug, metabolite, pharmaceutically acceptable salt or co-crystal thereof according to claim 1, **characterised in that** the compound is selected from a compound of general formula (II),

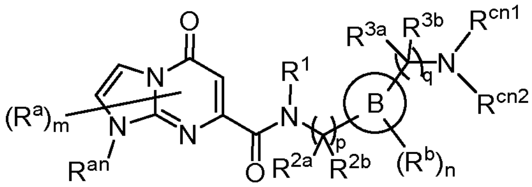
(II).

3. The compound or the stereoisomer, tautomer, deuterate, solvate, prodrug, metabolite, pharmaceutically acceptable salt or co-crystal thereof according to claim 2, **characterised in that**

$R^{an}$ is selected from H, $C_{1-4}$ alkyl, $C_{3-6}$ cycloalkyl, or 3- to 6-membered heterocycloalkyl, the alkyl, cycloalkyl, cycloalkyl or heterocycloalkyl is optionally further substituted with 0 to 4 substituents selected from H, D, halogen,

OH, =O, cyano, $NH_2$, $C_{1-4}$ alkyl, $C_{2-4}$ alkenyl, $C_{2-4}$ alkynyl, -$C_{2-4}$ alkynyl-$C_{3-6}$ cycloalkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy, $C_{3-6}$ cycloalkyl, phenyl, 3- to 6-membered heterocycloalkyl or 5- to 6-membered heteroaryl, and the heterocycloalkyl or heteroaryl contains 1 to 3 heteroatoms selected from O, S or N;

$R^a$ or $R^b$ is each independently selected from H, halogen, cyano, OH, =O, $C_{1-4}$ alkyl, $C_{1-4}$ alkoxy, or $C_{3-6}$ cycloalkyl, and the alkyl, alkoxy or cycloalkyl is optionally further substituted with 0 to 4 substituents selected from H, halogen, OH, =O, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy or $C_{3-6}$ cycloalkyl;

alternatively, $R^{an}$ and $R^a$ together with the atom to which they are attached form a 5- to 6-membered heteroaromatic ring, the heteroaromatic ring is optionally further substituted with 0 to 4 substituents selected from H, halogen, OH, =O, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy or $C_{3-6}$ cycloalkyl, and the heteroaryl contains 1 to 3 heteroatoms selected from O, S or N;

$R^1$ is selected from H, $C_{1-4}$ alkyl or $C_{3-6}$ cycloalkyl, the alkyl or cycloalkyl is optionally further substituted with 0 to 4 substituents selected from H, halogen, OH, =O, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy or $C_{3-6}$ cycloalkyl;

$R^{2a}$, $R^{2b}$, $R^{3a}$ and $R^{3b}$ are each independently selected from H, halogen, cyano, OH, =O, $C_{1-4}$ alkyl, $C_{1-4}$ alkoxy or $C_{3-6}$ cycloalkyl, and the alkyl, alkoxy or cycloalkyl is optionally further substituted with 0 to 4 substituents selected from H, halogen, OH, =O, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy or $C_{3-6}$ cycloalkyl;

ring B is selected from 5-fused-5-membered bisheteroaryl, 5-fused-6-membered bisheteroaryl or 6-fused-6-membered bisheteroaryl, and the heteroaryl contains 1 to 5 heteroatoms selected from O, S or N;

m is selected from 0, 1 or 2;

n is selected from 0, 1, 2, 3 or 4;

p is selected from 0, 1 or 2;

q is selected from 0, 1 or 2.

**4.** The compound or the stereoisomer, tautomer, deuterate, solvate, prodrug, metabolite, pharmaceutically acceptable salt or co-crystal thereof according to claim 3, **characterised in that**

$R^{cn1}$ and $R^{cn2}$ are each independently selected from H, halogen, cyano, OH, =O, $C_{1-4}$ alkyl, $C_{1-4}$ alkoxy, $C_{3-10}$ cycloalkyl, 3- to 10-membered heterocycloalkyl or

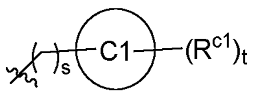

,

the alkyl, alkoxy, cycloalkyl or heterocycloalkyl is optionally further substituted with 0 to 4 substituents selected from H, halogen, OH, =O, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy or $C_{3-6}$ cycloalkyl, and the heterocycloalkyl contains 1 to 3 heteroatoms selected from O, S or N;

ring C1 is selected from $C_{3-7}$ monocycloalkyl, $C_{4-11}$ fused cycloalkyl, $C_{5-11}$ spirocycloalkyl, $C_{5-12}$bridged cycloalkyl, 4- to 7-membered monoheterocycloalkyl, 4- to 11-membered fused heterocycloalkyl, 5- to 11-membered spiroheterocycloalkyl or 5- to 12-membered bridged heterocycloalkyl, and the heterocycloalkyl contains 1 to 3 heteroatoms selected from O, S or N;

$R^{c1}$ is selected from H, halogen, cyano, OH, =O, $C_{1-4}$ alkyl, $C_{1-4}$ alkoxy, $C_{3-6}$ cycloalkyl or 3- to 6-membered heterocycloalkyl, the alkyl, alkoxy, cycloalkyl or heterocycloalkyl is optionally further substituted with 0 to 4 substituents selected from H, halogen, OH, =O, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy or $C_{3-6}$ cycloalkyl, and the heterocycloalkyl contains 1 to 3 heteroatoms selected from O, S or N;

alternatively, $R^{cn1}$ and $R^{cn2}$ together with the atom to which they are attached form 4- to 7-membered monoheterocycloalkyl, 4- to 11-membered fused heterocycloalkyl, 5- to 11-membered spiroheterocycloalkyl or 5- to 12-membered bridged heterocycloalkyl, the heterocycloalkyl is optionally further substituted with 0 to 4 substituents selected from H, halogen, OH, =O, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy or $C_{3-6}$ cycloalkyl, and the heterocycloalkyl contains 1 to 3 heteroatoms selected from O, S or N;

s is selected from 0, 1 or 2;

t is selected from 0, 0, 1, 2, 3 or 4.

**5.** The compound or the stereoisomer, tautomer, deuterate, solvate, prodrug, metabolite, pharmaceutically acceptable salt or co-crystal thereof according to claim 4, **characterised in that**

$R^{an}$ is selected from H, methyl, ethyl, propyl, isopropyl, cyclopropyl, cyclohexyl, cyclopentyl, cyclobutyl, oxetanyl, oxacyclopentyl, oxacyclohexyl, azetidinyl, azacyclopentyl, piperidyl or piperazinyl, the methyl, ethyl, propyl, isopropyl, cyclopropyl, cyclohexyl, cyclopentyl, cyclobutyl, oxetanyl, oxacyclopentyl, oxacyclohexyl, azetidinyl, azacyclopentyl, piperidyl or piperazinyl is optionally further substituted with 0 to 4 substituents selected from H, D, F, Cl, Br, I, OH, =O, cyano, $NH_2$, $C_{1-4}$ alkyl, $C_{2-4}$ alkenyl, $C_{2-4}$ alkynyl, -$C_{2-4}$ alkynyl-$C_{3-6}$ cycloalkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy, $C_{3-6}$ cycloalkyl, phenyl, 3- to 6-membered heterocycloalkyl or 5- to 6-membered heteroaryl, and the heterocycloalkyl or heteroaryl contains 1 to 3 heteroatoms selected from O, S or N;

$R^a$ or $R^b$ is each independently selected from H, F, Cl, Br, I, cyano, OH, =O, methyl, ethyl, propyl, isopropyl, cyclopropyl, cyclohexyl, cyclopentyl, cyclobutyl, azetidinyl, azacyclopentyl, azacyclohexyl, piperidyl, methoxy or ethoxy, and the methyl, ethyl, propyl, isopropyl, cyclopropyl, cyclohexyl, cyclopentyl, cyclobutyl, azetidinyl, azacyclopentyl, azacyclohexyl, piperidyl, methoxy or ethoxy is optionally further substituted with 0 to 4 substituents selected from H, F, Cl, Br, I, OH, =O, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy or $C_{3-6}$ cycloalkyl;

alternatively, $R^{an}$ and $R^a$ together with the atom to which they are attached form a pyridine ring, pyrimidine ring, pyridazine ring or pyrazine ring, and the pyridine ring, pyrimidine ring or pyrazine ring is optionally further substituted with 0 to 4 substituents selected from H, F, Cl, Br, I, OH, =O, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy or $C_{3-6}$ cycloalkyl;

$R^1$ is selected from H, methyl, ethyl, propyl, isopropyl or cyclopropyl, and the methyl, ethyl, propyl, isopropyl or cyclopropyl is optionally further substituted with 0 to 4 substituents selected from H, F, Cl, Br, I, OH, =O, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy or $C_{3-6}$ cycloalkyl;

$R^{2a}$, $R^{2b}$, $R^{3a}$ and $R^{3b}$ are each independently selected from H, F, Cl, Br, I, cyano, OH, =O, methyl, ethyl, propyl, isopropyl, methoxy, ethoxy or cyclopropyl, and the methyl, ethyl, propyl, isopropyl, methoxy, ethoxy or cyclopropyl is optionally further substituted with 0 to 4 substituents selected from H, F, Cl, Br, I, OH, =O, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy or $C_{3-6}$ cycloalkyl;

ring B is selected from pyrrolopyrrolyl, pyrrolopyrazolyl, pyrroloimidazolyl, pyrazolopyrazolyl, pyrazoloimidazolyl, imidazoimidazolyl, benzopyrrolyl, benzopyrazolyl, benzoimidazolyl, pyridopyrrolyl, pyridopyrazolyl, pyridoimidazolyl, pyrimidopyrrolyl, pyrimidopyrazolyl, pyrimidoimidazolyl, pyrazinopyrrolyl, pyrazinopyrazolyl, pyrazinoimidazolyl, pyridazinopyrrolyl, pyridazinopyrazolyl, pyridazinoimidazolyl, benzopyridyl, benzopyrimidyl, benzopyrazinyl, benzopyridazinyl, pyridopyridyl, pyridopyrimidinyl, pyridopyrazinyl or pyridopyridazinyl;

$R^{cn1}$ and $R^{cn2}$ are each independently selected from H, F, Cl, Br, I, cyano, OH, =O, methyl, ethyl, propyl, isopropyl, cyclopropyl, cyclohexyl, cyclopentyl, cyclobutyl, azetidinyl, azacyclopentyl, azacyclohexyl or

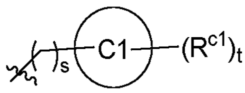
,

and the methyl, ethyl, propyl, isopropyl, cyclopropyl, cyclohexyl, cyclopentyl, cyclobutyl, azetidinyl, azacyclopentyl, or azacyclohexyl is optionally further substituted with 0 to 4 substituents selected from H, F, Cl, Br, I, OH, =O, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy or $C_{3-6}$ cycloalkyl;

ring C1 is selected from cyclopropyl, cyclobutyl, cyclopentyl, cyclohexyl, oxetanyl, oxacyclopentyl, oxacyclohexyl, azetidinyl, azacyclopentyl, piperidine, morpholine, piperazine, 1,4-diazepanyl, cyclopropyl fused cyclopentyl, cyclobutyl fused cyclopentyl, cyclopentyl fused cyclopentyl, cyclopentyl fused cyclohexyl, cyclopropyl spirocyclopentyl, cyclobutyl spirocyclobutyl, cyclobutyl spirocyclopentyl, cyclopentyl spirocyclopentyl, cyclopentyl spirocyclohexyl, cyclohexyl spirocyclohexyl, cyclopropyl fused azetidinyl, cyclopropyl fused azacyclopentyl, cyclopropyl fused azacyclohexyl, cyclobutyl fused azetidinyl, cyclobutyl fused azacyclopentyl, cyclobutyl fused azacyclohexyl, cyclopentyl fused azetidinyl, cyclopentyl fused azacyclopentyl, cyclopentyl fused azacyclohexyl, cyclohexyl fused azetidinyl, cyclohexyl fused azacyclopentyl, cyclohexyl fused azacyclohexyl, azetidinyl fused azetidinyl, azetidinyl fused azacyclopentyl, azetidinyl fused azacyclohexyl, azacyclopentyl fused azetidinyl,

azacyclopentyl fused azacyclopentyl, azacyclopentyl fused azacyclohexyl, azacyclohexyl fused azetidinyl, azacyclohexyl fused azacyclopentyl, azacyclohexyl fused azacyclohexyl, cyclobutyl spiroazetidinyl, cyclobutyl spiroazacyclopentyl, cyclobutyl spiroazacyclohexyl, cyclopentyl spiroazetidinyl, cyclopentyl spiroazacyclopentyl, cyclopentyl spiroazacyclohexyl, cyclohexyl spiroazetidinyl, cyclohexyl spiroazacyclopentyl, cyclohexyl spiroazacyclohexyl, azetidinyl spiroazetidinyl, azetidinyl spiroazacyclopentyl, azetidinyl spiroazacyclohexyl, azacyclopentyl spiroazetidinyl, azacyclopentyl spiroazacyclopentyl, azacyclopentyl spiroazacyclohexyl, azacyclohexyl spiroazetidinyl, azacyclohexyl spiroazacyclopentyl, azacyclohexyl spiroazacyclohexyl, oxetanyl, oxacyclopentyl, oxacyclohexyl, cyclopropyl fused oxetanyl, cyclopropyl fused oxacyclopentyl, cyclopropyl fused oxacyclohexyl, cyclobutyl fused oxetanyl, cyclobutyl fused oxacyclopentyl, cyclobutyl fused oxacyclohexyl, cyclopentyl fused oxetanyl, cyclopentyl fused oxacyclopentyl, cyclopentyl fused oxacyclohexyl, cyclohexyl fused oxetanyl, cyclohexyl fused oxacyclopentyl, cyclohexyl fused oxacyclohexyl, azetidinyl fused oxetanyl, azetidinyl fused oxacyclopentyl, azetidinyl fused oxacyclohexyl, azacyclopentyl fused oxetanyl, azacyclopentyl fused oxacyclopentyl, azacyclopentyl fused oxacyclohexyl, azacyclohexyl fused oxetanyl, azacyclohexyl fused oxacyclopentyl, azacyclohexyl fused oxacyclohexyl, cyclobutyl spirooxetanyl, cyclobutyl spirooxacyclopentyl, cyclobutyl spirooxacyclohexyl, cyclopentyl spirooxetanyl, cyclopentyl spirooxacyclopentyl, cyclopentyl spirooxacyclohexyl, cyclohexyl spirooxetanyl, cyclohexyl spirooxacyclopentyl, cyclohexyl spirooxacyclohexyl, azetidinyl spirooxetanyl, azetidinyl spirooxacyclopentyl, azetidinyl spirooxacyclohexyl, azacyclopentyl spirooxetanyl, azacyclopentyl spirooxacyclopentyl, azacyclopentyl spirooxacyclohexyl, azacyclohexyl spirooxetanyl, azacyclohexyl spirooxacyclopentyl, azacyclohexyl spirooxacyclohexyl, bicyclo[2.1.1]hexyl, bicyclo[2.2.1]heptyl, bicyclo[3.3.2]decanyl, bicyclo[2.2.2]octanyl, bicyclo[3.2.1]octanyl, bicyclo[3.3.3]undecyl, adamantyl,

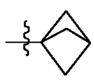, 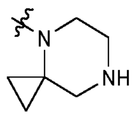,

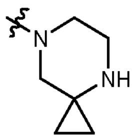, 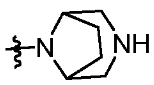, 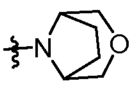, 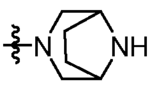, 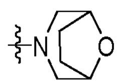, 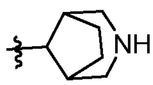, 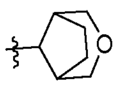,

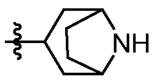, 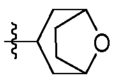, 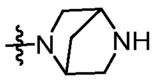, 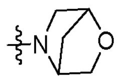, 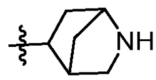, 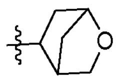,

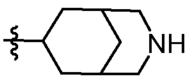, 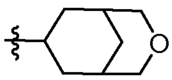, 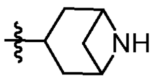, 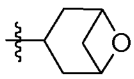, 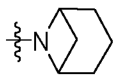, 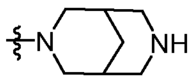,

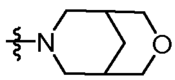, or 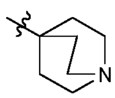;

each $R^{c1}$ is independently selected from H, F, Cl, Br, I, cyano, OH, =O, methyl, ethyl, propyl, isopropyl, cyclopropyl, cyclohexyl, cyclopentyl, cyclobutyl, azetidinyl, azacyclopentyl, piperidyl, piperazinyl, methoxy or ethoxy, and the methyl, ethyl, propyl, isopropyl, cyclopropyl, cyclohexyl, cyclopentyl, cyclobutyl, azetidinyl, azacyclopentyl, piperidyl, piperazinyl, methoxy or ethoxy is optionally further substituted with 0 to 4 substituents selected from H, F, Cl, Br, I, OH, =O, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy or $C_{3-6}$ cycloalkyl;

alternatively, $R^{cn1}$ and $R^{cn2}$ together with the atom to which they are attached form substituted or unsubstituted azetidinyl, azacyclopentyl, piperidine, piperazine, azetidinyl fused cyclopropyl, azetidinyl fused cyclobutyl, azetidinyl fused cyclopentyl, azetidinyl fused cyclohexyl, azacyclopentyl fused cyclopropyl, azacyclopentyl fused cyclobutyl, azacyclopentyl fused cyclopentyl, azacyclopentyl fused cyclohexyl, azacyclohexyl fused cyclopropyl, azacyclohexyl fused cyclobutyl, azacyclohexyl fused cyclopentyl, azacyclohexyl fused cyclohexyl, azetidinyl fused azetidinyl, azetidinyl fused azacyclopentyl, azetidinyl fused azacyclohexyl, azacyclopentyl fused azetidinyl, azacyclopentyl fused azacyclopentyl, azacyclopentyl fused azacyclohexyl, azacyclohexyl fused azetidinyl,

azacyclohexyl fused azacyclopentyl, azacyclohexyl fused azacyclohexyl, azetidinyl spiroazetidinyl, azetidinyl spiroazacyclopentyl, azetidinyl spiroazacyclohexyl, azacyclopentyl spiroazetidinyl, azacyclopentyl spiroazacyclopentyl, azacyclopentyl spiroazacyclohexyl, azacyclohexyl spiroazetidinyl, azacyclohexyl spiroazacyclopentyl, azacyclohexyl spiroazacyclohexyl,

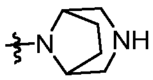 , 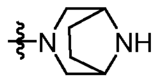 ,

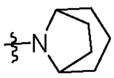 , 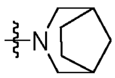 , 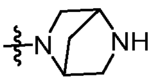 , 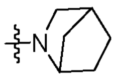 , 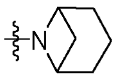 , 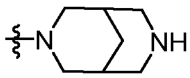 ,

or

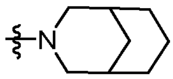 ,

which, when substituted, is optionally further substituted with 0 to 4 substituents selected from H, halogen, OH, =O, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy or $C_{3-6}$ cycloalkyl.

**6.** The compound or the stereoisomer, tautomer, deuterate, solvate, prodrug, metabolite, pharmaceutically acceptable salt or co-crystal thereof according to claim 5, **characterised in that**

$R^{an}$ is selected from H, methyl, ethyl, propyl, isopropyl, cyclopropyl, cyclobutyl, cyclopentyl, oxetanyl, oxacyclopentyl or oxacyclohexyl, and the methyl, ethyl, propyl, isopropyl, cyclopropyl, oxetanyl, oxacyclopentyl or oxacyclohexyl is optionally further substituted with 0 to 4 substituents selected from H, D, F, Cl, Br, I, OH, =O, cyano, $NH_2$, $C_{1-4}$ alkyl, halogen-substituted-$C_{1-4}$ alkyl, $C_{2-4}$ alkenyl, $C_{2-4}$ alkynyl, -$C_{2-4}$ alkynyl-$C_{3-6}$ cycloalkyl, hydroxyl-substituted-$C_{1-4}$ alkyl, cyano-substituted-$C_{1-4}$ alkyl, $C_{1-4}$ alkoxy, $C_{3-6}$ cycloalkyl, phenyl, 3- to 6-membered heterocycloalkyl or 5- to 6-membered heteroaryl, the heterocycloalkyl or heteroaryl contains 1 to 3 heteroatoms selected from O, S or N;
$R^a$ or $R^b$ is each independently selected from H, F, Cl, Br, I, cyano, OH, =O, methyl, ethyl, propyl, isopropyl, or cyclopropyl, and the methyl, ethyl, propyl, isopropyl or cyclopropyl is optionally further substituted with 0 to 4 substituents selected from H, F, Cl, Br, I, OH, =O, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy or $C_{3-6}$ cycloalkyl;
alternatively, $R^{an}$ and $R^a$ together with the atom to which they are attached form a pyridine ring, and the pyridine ring is optionally further substituted with 0 to 4 substituents selected from H, F, Cl, Br, I, OH, =O, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy or $C_{3-6}$ cycloalkyl;
ring B is selected from benzopyrrolyl, benzopyrazolyl, benzoimidazolyl, pyridopyrrolyl, pyridopyrazolyl, pyridoimidazolyl, pyrimidopyrrolyl, pyrimidopyrazolyl, pyrimidoimidazolyl, pyrazinopyrrolyl, pyrazinopyrazolyl, pyrazinoimidazolyl, pyridazinopyrrolyl, pyridazinopyrazolyl, or pyridazinoimidazolyl;
$R^{cn1}$ and $R^{cn2}$ are each independently selected from H, F, Cl, Br, I, cyano, OH, =O, methyl, ethyl, propyl, isopropyl, cyclopropyl, cyclohexyl, cyclopentyl, cyclobutyl or

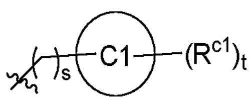 ,

and the methyl, ethyl, propyl, isopropyl, cyclopropyl, cyclohexyl, cyclopentyl, or cyclobutyl is optionally further substituted with 0 to 4 substituents selected from H, F, Cl, Br, I, OH, =O, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy or $C_{3-6}$ cycloalkyl;
ring C1 is selected from cyclopropyl, cyclobutyl, cyclopentyl, cyclohexyl, oxetanyl, oxacyclopentyl, oxacyclohexyl, azetidinyl, azacyclopentyl, piperidine, morpholine, piperazine, 1,4-diazepanyl,

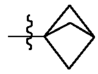

,

bicyclo[2.1.1]hexyl, bicyclo[2.2.1]heptyl, bicyclo[3.3.2]decanyl, bicyclo[2.2.2]octanyl, bicyclo[3.2.1]octanyl, bicyclo[3.3.3]undecyl, or adamantyl;

each $R^{c1}$ is independently selected from H, F, Cl, Br, I, cyano, OH, =O, methyl, ethyl, propyl, isopropyl, cyclopropyl, methoxy or ethoxy, and the methyl, ethyl, propyl, isopropyl, cyclopropyl, methoxy or ethoxy is optionally further substituted with 0 to 4 substituents selected from H, F, Cl, Br, I, OH, =O, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy or $C_{3-6}$ cycloalkyl;

alternatively, $R^{cn1}$ and $R^{cn2}$ together with the atom to which they are attached form substituted or unsubstituted azetidinyl, azacyclopentyl, piperidine or piperazine, which, when substituted, is optionally further substituted with 0 to 4 substituents selected from H, F, Cl, Br, I, OH, =O, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy or $C_{3-6}$ cycloalkyl.

**7.** The compound or the stereoisomer, tautomer, deuterate, solvate, prodrug, metabolite, pharmaceutically acceptable salt or co-crystal thereof according to claim 6, **characterised in that** the compound is selected from a compound of general formula (II-1) or general formula (II-2),

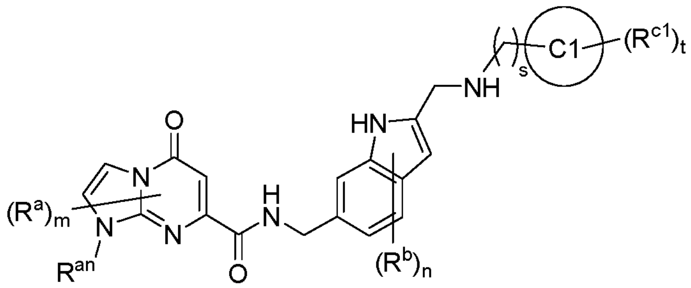

(II-1)

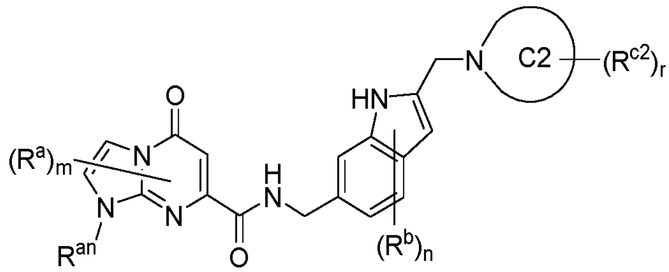

(II-2)

wherein $R^{an}$ is selected from H, methyl, $CD_3$, ethyl, propyl, isopropyl, cyclopropyl, oxetanyl, oxacyclopentyl or oxacyclohexyl, and the methyl, ethyl, propyl, isopropyl, cyclopropyl, oxetanyl, oxacyclopentyl or oxacyclohexyl is optionally further substituted with 0 to 4 substituents selected from H, F, Cl, Br, I, OH, =O, cyano, $NH_2$, methyl, ethyl, propyl, isopropyl, ethenyl, 1-propenyl, 2-propenyl, ethynyl, 1-propynyl, 2-propynyl,

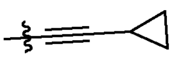

,

cyclopropyl, methoxy, ethoxy, or phenyl;

$R^a$ or $R^b$ is each independently selected from H, F, Cl, Br, I, cyano, OH, =O, methyl, ethyl, propyl, isopropyl, or cyclopropyl;

alternatively, $R^{an}$ and $R^a$ together with the atom to which they are attached form a pyridine ring;

ring C1 is selected from cyclopropyl, cyclobutyl, cyclopentyl, cyclohexyl or

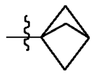

;

each $R^{c1}$ is independently selected from H, F, Cl, Br, I, cyano, OH, =O, methyl, ethyl, propyl, isopropyl or cyclopropyl;
ring C2 is selected from azetidinyl, azacyclopentyl, piperidine or piperazine; each $R^{c2}$ is independently selected from H, F, Cl, Br, I, OH, =O, cyano, $NH_2$, methyl, ethyl, propyl, isopropyl, cyclopropyl, methoxy, or ethoxy;
m is selected from 0 or 1;
n is selected from 0 or 1;
r is selected from 0, 1, 2 or 3;
s is selected from 0 or 1;
t is selected from 0, 1, 2 or 3.

**8.** The compound or the stereoisomer, tautomer, deuterate, solvate, prodrug, metabolite, pharmaceutically acceptable salt or co-crystal thereof according to claim 1, **characterised in that** the compound is selected from a compound of general formula (III),

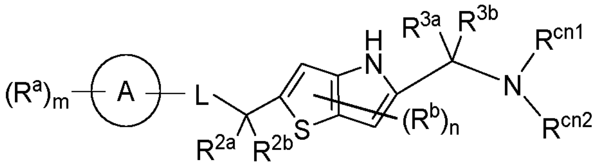

(III).

**9.** The compound or the stereoisomer, tautomer, deuterate, solvate, prodrug, metabolite, pharmaceutically acceptable salt or co-crystal thereof according to claim 8, **characterised in that**

$R^{an}$ is selected from H, $C_{1-4}$ alkyl, $C_{3-6}$ cycloalkyl, or 3- to 6-membered heterocycloalkyl, the alkyl, cycloalkyl or heterocycloalkyl is optionally further substituted with 0 to 4 substituents selected from H, halogen, OH, =O, cyano, $NH_2$, $C_{1-4}$ alkyl, $C_{2-4}$ alkenyl, $C_{2-4}$ alkynyl, -$C_{2-4}$ alkynyl-$C_{3-6}$ cycloalkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy, $C_{3-6}$ cycloalkyl, phenyl, 3- to 6-membered heterocycloalkyl or 5- to 6-membered heteroaryl, and the heterocycloalkyl or heteroaryl contains 1 to 3 heteroatoms selected from O, S or N;
$R^a$ or $R^b$ is each independently selected from H, halogen, cyano, OH, =O, $C_{1-4}$ alkyl, $C_{1-4}$ alkoxy, or $C_{3-6}$ cycloalkyl, and the alkyl, alkoxy or cycloalkyl is optionally further substituted with 0 to 4 substituents selected from H, halogen, OH, =O, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy or $C_{3-6}$ cycloalkyl;
$R^1$ is selected from H, $C_{1-4}$ alkyl or $C_{3-6}$ cycloalkyl, the alkyl or cycloalkyl is optionally further substituted with 0 to 4 substituents selected from H, halogen, OH, =O, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy or $C_{3-6}$ cycloalkyl;
$R^{2a}$, $R^{2b}$, $R^{3a}$ and $R^{3b}$ are each independently selected from H, halogen, cyano, OH, =O, $C_{1-4}$ alkyl, $C_{1-4}$ alkoxy or $C_{3-6}$ cycloalkyl, and the alkyl, alkoxy or cycloalkyl is optionally further substituted with 0 to 4 substituents selected from H, halogen, OH, =O, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy or $C_{3-6}$ cycloalkyl;
m is selected from 0, 1 or 2;
n is selected from 0, 1 or 2.

**10.** The compound or the stereoisomer, tautomer, deuterate, solvate, prodrug, metabolite, pharmaceutically acceptable salt or co-crystal thereof according to claim 9, **characterised in that**

L is selected from

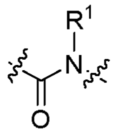

or 5-membered heteroaryl, L is connected with ring A at the left side, the heteroaryl is optionally further substituted with 0 to 2 substituents selected from H, halogen, OH, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, or $C_{1-4}$ alkoxy, and the heteroaryl contains 1 to 3 heteroatoms selected from O, S or N;

$R^1$ is selected from H, methyl, ethyl, propyl, isopropyl or cyclopropyl, and the methyl, ethyl, propyl, isopropyl or cyclopropyl is optionally further substituted with 0 to 4 substituents selected from H, F, Cl, Br, I, OH, =O, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy or $C_{3-6}$ cycloalkyl;

$R^{cn1}$ and $R^{cn2}$ are each independently selected from H, halogen, cyano, OH, =O, $C_{1-4}$ alkyl, $C_{1-4}$ alkoxy, $C_{3-7}$ monocycloalkyl, $C_{4-10}$ fused cycloalkyl, $C_{5-10}$-membered spirocycloalkyl, $C_{5-10}$ bridged cycloalkyl, 4- to 7-membered monoheterocycloalkyl, 4- to 10-membered fused heterocycloalkyl, 5- to 10-membered spiroheterocycloalkyl, 5- to 10-membered bridged heterocycloalkyl or

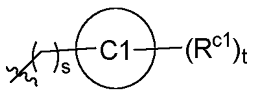

,

the alkyl, alkoxy, cycloalkyl or heterocycloalkyl is optionally further substituted with 0 to 4 substituents selected from H, halogen, OH, =O, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy or $C_{3-6}$ cycloalkyl, and the heterocycloalkyl contains 1 to 3 heteroatoms selected from O, S or N;

ring C1 is selected from $C_{3-7}$ monocycloalkyl, $C_{4-11}$ fused cycloalkyl, $C_{5-11}$ spirocycloalkyl, $C_{5-12}$ bridged cycloalkyl, 4- to 7-membered monoheterocycloalkyl, 4- to 11-membered fused heterocycloalkyl, 5- to 11-membered spiroheterocycloalkyl or 5- to 12-membered bridged heterocycloalkyl, and the heterocycloalkyl contains 1 to 3 heteroatoms selected from O, S or N;

each $R^{c1}$ is independently selected from H, halogen, cyano, OH, =O, $C_{1-4}$ alkyl, $C_{1-4}$ alkoxy, $C_{3-6}$ cycloalkyl or 3- to 6-membered heterocycloalkyl, the alkyl, alkoxy, cycloalkyl or heterocycloalkyl is optionally further substituted with 0 to 4 substituents selected from H, halogen, OH, =O, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy or $C_{3-6}$ cycloalkyl, and the heterocycloalkyl contains 1 to 3 heteroatoms selected from O, S or N;

alternatively, $R^{cn1}$ and $R^{cn2}$ together with the atom to which they are attached form 4- to 7-membered monoheterocycloalkyl, 4- to 11-membered fused heterocycloalkyl, 5- to 11-membered spiroheterocycloalkyl or 5- to 12-membered bridged heterocycloalkyl, the heterocycloalkyl is optionally further substituted with 0 to 4 substituents selected from H, halogen, OH, =O, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy or $C_{3-6}$ cycloalkyl, and the heterocycloalkyl contains 1 to 3 heteroatoms selected from O, S or N;

p is selected from 0, 1, or 2;

t is selected from 0, 1, 2, 3 or 4.

**11.** The compound or the stereoisomer, tautomer, deuterate, solvate, prodrug, metabolite, pharmaceutically acceptable salt or co-crystal thereof according to claim 10, **characterised in that**

$R^{an}$ is selected from H, methyl, ethyl, propyl, isopropyl, cyclopropyl, cyclohexyl, cyclopentyl, cyclobutyl, oxetanyl, oxacyclopentyl, oxacyclohexyl, azetidinyl, azacyclopentyl, piperidyl or piperazinyl, the methyl, ethyl, propyl, isopropyl, cyclopropyl, cyclohexyl, cyclopentyl, cyclobutyl, oxetanyl, oxacyclopentyl, oxacyclohexyl, azetidinyl, azacyclopentyl, piperidyl or piperazinyl is optionally further substituted with 0 to 4 substituents selected from H, F, Cl, Br, I, OH, =O, cyano, $NH_2$, $C_{1-4}$ alkyl, $C_{2-4}$ alkenyl, $C_{2-4}$ alkynyl, -$C_{2-4}$ alkynyl-$C_{3-6}$ cycloalkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy, $C_{3-6}$ cycloalkyl, phenyl, 3- to 6-membered heterocycloalkyl or 5- to 6-membered heteroaryl, and the heterocycloalkyl or heteroaryl contains 1 to 3 heteroatoms selected from O, S or N;

$R^a$ or $R^b$ is each independently selected from H, F, Cl, Br, I, cyano, OH, =O, methyl, ethyl, propyl, isopropyl, cyclopropyl, cyclohexyl, cyclopentyl, cyclobutyl, azetidinyl, azacyclopentyl, azacyclohexyl, piperidyl, methoxy or ethoxy, and the methyl, ethyl, propyl, isopropyl, cyclopropyl, cyclohexyl, cyclopentyl, cyclobutyl, azetidinyl, azacyclopentyl, azacyclohexyl, piperidyl, methoxy or ethoxy is optionally further substituted with 0 to 4 substituents selected from H, F, Cl, Br, I, OH, =O, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy or $C_{3-6}$ cycloalkyl;

L is selected from

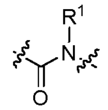

oxazolyl, thiazolyl, thienyl, furyl, pyrrolyl, pyrazolyl, triazolyl or imidazolyl, L is connected with ring A at the left side, and the oxazolyl, thiazolyl, thienyl, furyl, pyrrolyl, pyrazolyl, triazolyl or imidazolyl is optionally further substituted with 0 to 2 substituents selected from H, F, Cl, Br, I, OH, cyano, $NH_2$, methyl, ethyl, propyl, isopropyl, methoxy, or ethoxy;

$R^1$ is selected from H, methyl, ethyl, propyl, or isopropyl;

$R^{2a}$, $R^{2b}$, $R^{3a}$ and $R^{3b}$ are each independently selected from H, F, Cl, Br, I, cyano, OH, =O, methyl, ethyl, propyl, isopropyl, methoxy, ethoxy or cyclopropyl, and the methyl, ethyl, propyl, isopropyl, methoxy, ethoxy or cyclopropyl is optionally further substituted with 0 to 4 substituents selected from H, F, Cl, Br, I, OH, =O, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy or $C_{3-6}$ cycloalkyl;

$R^{cn1}$ and $R^{cn2}$ are each independently selected from H, F, Cl, Br, I, cyano, OH, =O, methyl, ethyl, propyl, isopropyl, cyclopropyl, cyclohexyl, cyclopentyl, cyclobutyl, azetidinyl, azacyclopentyl, azacyclohexyl or

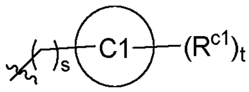

and the methyl, ethyl, propyl, isopropyl, cyclopropyl, cyclohexyl, cyclopentyl, cyclobutyl, azetidinyl, azacyclopentyl, or azacyclohexyl is optionally further substituted with 0 to 4 substituents selected from H, F, Cl, Br, I, OH, =O, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy or $C_{3-6}$ cycloalkyl;

ring C1 is selected from cyclopropyl, cyclobutyl, cyclopentyl, cyclohexyl, oxetanyl, oxacyclopentyl, oxacyclohexyl, azetidinyl, azacyclopentyl, piperidine, morpholine, piperazine, 1,4-diazepanyl, cyclopropyl fused cyclopentyl, cyclopentyl fused cyclobutyl, cyclopentyl fused cyclopentyl, cyclopentyl fused cyclohexyl, cyclopropyl spirocyclopentyl, cyclobutyl spirocyclobutyl, cyclobutyl spirocyclopentyl, cyclopentyl spirocyclopentyl, cyclopentyl spirocyclohexyl, cyclohexyl spirocyclohexyl, cyclopropyl fused azetidinyl, cyclopropyl fused azacyclopentyl, cyclopropyl fused azacyclohexyl, cyclobutyl fused azetidinyl, cyclobutyl fused azacyclopentyl, cyclobutyl fused azacyclohexyl, cyclopentyl fused azetidinyl, cyclopentyl fused azacyclopentyl, cyclopentyl fused azacyclohexyl, cyclohexyl fused azetidinyl, cyclohexyl fused azacyclopentyl, cyclohexyl fused azacyclohexyl, azetidinyl fused azetidinyl, azetidinyl fused azacyclopentyl, azetidinyl fused azacyclohexyl, azacyclopentyl fused azetidinyl, azacyclopentyl fused azacyclopentyl, azacyclopentyl fused azacyclohexyl, azacyclohexyl fused azetidinyl, azacyclohexyl fused azacyclopentyl, azacyclohexyl fused azacyclohexyl, cyclobutyl spiroazetidinyl, cyclobutyl spiroazacyclopentyl, cyclobutyl spiroazacyclohexyl, cyclopentyl spiroazetidinyl, cyclopentyl spiroazacyclopentyl, cyclopentyl spiroazacyclohexyl, cyclohexyl spiroazetidinyl, cyclohexyl spiroazacyclopentyl, cyclohexyl spiroazacyclohexyl, azetidinyl spiroazetidinyl, azetidinyl spiroazacyclopentyl, azetidinyl spiroazacyclohexyl, azacyclopentyl spiroazetidinyl, azacyclopentyl spiroazacyclopentyl, azacyclopentyl spiroazacyclohexyl, azacyclohexyl spiroazetidinyl, azacyclohexyl spiroazacyclopentyl, azacyclohexyl spiroazacyclohexyl, oxetanyl, oxacyclopentyl, oxacyclohexyl, cyclopropyl fused oxetanyl, cyclopropyl fused oxacyclopentyl, cyclopropyl fused oxacyclohexyl, cyclobutyl fused oxetanyl, cyclobutyl fused oxacyclopentyl, cyclobutyl fused oxacyclohexyl, cyclopentyl fused oxetanyl, cyclopentyl fused oxacyclopentyl, cyclopentyl fused oxacyclohexyl, cyclohexyl fused oxetanyl, cyclohexyl fused oxacyclopentyl, cyclohexyl fused oxacyclohexyl, azetidinyl fused oxetanyl, azetidinyl fused oxacyclopentyl, azetidinyl fused oxacyclohexyl, azacyclopentyl fused oxetanyl, azacyclopentyl fused oxacyclopentyl, azacyclopentyl fused oxacyclohexyl, azacyclohexyl fused oxetanyl, azacyclohexyl fused oxacyclopentyl, azacyclohexyl fused oxacyclohexyl, cyclobutyl spirooxetanyl, cyclobutyl spirooxacyclopentyl, cyclobutyl spirooxacyclohexyl, cyclopentyl spirooxetanyl, cyclopentyl spirooxacyclopentyl, cyclopentyl spirooxacyclohexyl, cyclohexyl spirooxetanyl, cyclohexyl spirooxacyclopentyl, cyclohexyl spirooxacyclohexyl, azetidinyl spirooxetanyl, azetidinyl spirooxacyclopentyl, azetidinyl spirooxacyclohexyl, azacyclopentyl spirooxetanyl, azacyclopentyl spirooxacyclopentyl, azacyclopentyl spirooxacyclohexyl, azacyclohexyl spirooxetanyl, azacyclohexyl spirooxacyclopentyl, azacyclohexyl spirooxacyclohexyl, bicyclo[2.1.1]hexyl, bicyclo[2.2.1]heptyl, bicyclo[3.3.2]decanyl, bicyclo[2.2.2]octanyl, bicyclo[3.2.1]octanyl, bicyclo[3.3.3]undecyl, adamantyl,

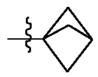, 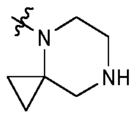,

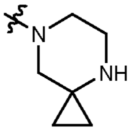, 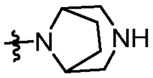, 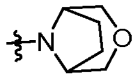, 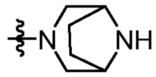, 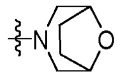, 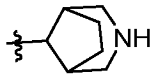, 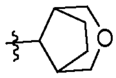,

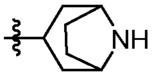, 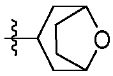, 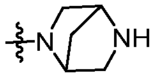, 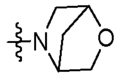, 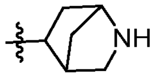, 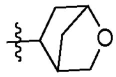,

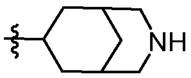, 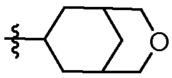, 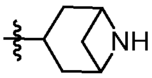, 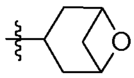, 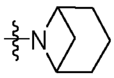, 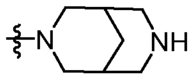,

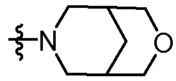. or 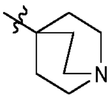

each $R^{c1}$ is independently selected from H, F, Cl, Br, I, cyano, OH, =O, methyl, ethyl, propyl, isopropyl, cyclopropyl, cyclohexyl, cyclopentyl, cyclobutyl, azetidinyl, azacyclopentyl, piperidyl, piperazinyl, methoxy or ethoxy, and the methyl, ethyl, propyl, isopropyl, cyclopropyl, cyclohexyl, cyclopentyl, cyclobutyl, azetidinyl, azacyclopentyl, piperidyl, piperazinyl, methoxy or ethoxy is optionally further substituted with 0 to 4 substituents selected from H, F, Cl, Br, I, OH, =O, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy or $C_{3-6}$ cycloalkyl;

alternatively, $R^{cn1}$ and $R^{cn2}$ together with the atom to which they are attached form substituted or unsubstituted azetidinyl, azacyclopentyl, piperidine, piperazine, azetidinyl fused cyclopropyl, azetidinyl fused cyclobutyl, azetidinyl fused cyclopentyl, azetidinyl fused cyclohexyl, azacyclopentyl fused cyclopropyl, azacyclopentyl fused cyclobutyl, azacyclopentyl fused cyclopentyl, azacyclopentyl fused cyclohexyl, azacyclohexyl fused cyclopropyl, azacyclohexyl fused cyclobutyl, azacyclohexyl fused cyclopentyl, azacyclohexyl fused cyclohexyl, azetidinyl fused azetidinyl, azetidinyl fused azacyclopentyl, azetidinyl fused azacyclohexyl, azacyclopentyl fused azetidinyl, azacyclopentyl fused azacyclopentyl, azacyclopentyl fused azacyclohexyl, azacyclohexyl fused azetidinyl, azacyclohexyl fused azacyclopentyl, azacyclohexyl fused azacyclohexyl, azetidinyl spiroazetidinyl, azetidinyl spiroazacyclopentyl, azetidinyl spiroazacyclohexyl, azacyclopentyl spiroazetidinyl, azacyclopentyl spiroazacyclopentyl, azacyclopentyl spiroazacyclohexyl, azacyclohexyl spiroazetidinyl, azacyclohexyl spiroazacyclopentyl, azacyclohexyl spiroazacyclohexyl, azacyclopentyl spirocyclobutyl, azacyclopentyl spirocyclopentyl, azacyclohexyl spirocyclobutyl, azacyclohexyl spirocyclopentyl, azacyclohexyl spirocyclohexyl,

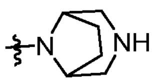,

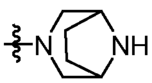, 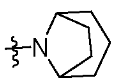, 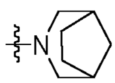, 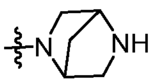, 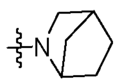, 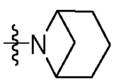, 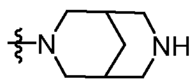,

or

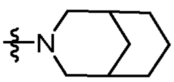,

which, when substituted, is optionally further substituted with 0 to 4 substituents selected from H, halogen, OH,

=O, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy or $C_{3-6}$ cycloalkyl.

**12.** The compound or the stereoisomer, tautomer, deuterate, solvate, prodrug, metabolite, pharmaceutically acceptable salt or co-crystal thereof according to claim 11, **characterised in that**

$R^{an}$ is selected from H, methyl, ethyl, propyl, isopropyl, or cyclopropyl, and the methyl, ethyl, propyl, isopropyl, or cyclopropyl is optionally further substituted with 0 to 4 substituents selected from H, F, Cl, Br, I, OH, =O, cyano, $NH_2$, methyl, ethyl, propyl, isopropyl, ethenyl, 1-propenyl, 2-propenyl, ethynyl, 1-propynyl, 2-propynyl,

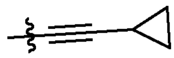

, cyclopropyl, methoxy, ethoxy, or phenyl;
$R^a$ or $R^b$ is each independently selected from H, F, Cl, Br, I, cyano, OH, =O, methyl, ethyl, propyl, isopropyl, or cyclopropyl, and the methyl, ethyl, propyl, isopropyl or cyclopropyl is optionally further substituted with 0 to 4 substituents selected from H, F, Cl, Br, I, OH, =O, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy or $C_{3-6}$ cycloalkyl;
$R^{cn1}$ and $R^{cn2}$ are each independently selected from H, F, Cl, Br, I, cyano, OH, =O, methyl, ethyl, propyl, isopropyl, cyclopropyl, cyclohexyl, cyclopentyl, cyclobutyl or

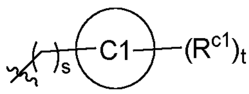

,

and the methyl, ethyl, propyl, isopropyl, cyclopropyl, cyclohexyl, cyclopentyl, or cyclobutyl is optionally further substituted with 0 to 4 substituents selected from H, F, Cl, Br, I, OH, =O, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy or $C_{3-6}$ cycloalkyl;
ring C1 is selected from cyclopropyl, cyclobutyl, cyclopentyl, cyclohexyl, oxetanyl, oxacyclopentyl, oxacyclohexyl, azetidinyl, azacyclopentyl, piperidine, morpholine, piperazine, 1,4-diazepanyl, cyclopropyl fused cyclopentyl, cyclopentyl fused cyclobutyl, cyclopentyl fused cyclopentyl, cyclopentyl fused cyclohexyl,

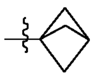

,

bicyclo[2.1.1]hexyl, bicyclo[2.2.1]heptyl, bicyclo[3.3.2]decanyl, bicyclo[2.2.2]octanyl, bicyclo[3.2.1]octanyl, bicyclo[3.3.3]undecyl, or adamantyl;
each $R^{c1}$ is independently selected from H, methyl, ethyl, propyl, isopropyl, cyclopropyl, methoxy or ethoxy, and the methyl, ethyl, propyl, isopropyl, cyclopropyl, methoxy or ethoxy is optionally further substituted with 0 to 4 substituents selected from H, F, Cl, Br, I, OH, =O, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy or $C_{3-6}$ cycloalkyl;
alternatively, $R^{cn1}$ and $R^{cn2}$ together with the atom to which they are attached form substituted or unsubstituted azetidinyl, azacyclopentyl, piperidine, piperazine, azacyclopentyl spirocyclobutyl, azacyclopentyl spirocyclopentyl, azacyclohexyl spirocyclobutyl, azacyclohexyl spirocyclopentyl, or azacyclohexyl spirocyclohexyl, which, when substituted, is optionally further substituted with 0 to 4 substituents selected from H, F, Cl, Br, I, OH, =O, cyano, $NH_2$, $C_{1-4}$ alkyl, halogen-substituted-$C_{1-4}$ alkyl, hydroxyl-substituted-$C_{1-4}$ alkyl, cyano-substituted-$C_{1-4}$ alkyl, $C_{1-4}$ alkoxy or $C_{3-6}$ cycloalkyl.

**13.** The compound or the stereoisomer, tautomer, deuterate, solvate, prodrug, metabolite, pharmaceutically acceptable salt or co-crystal thereof according to claim 12, **characterised in that**

ring A is selected from

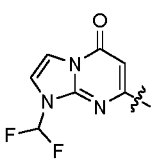, 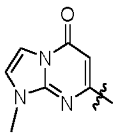, 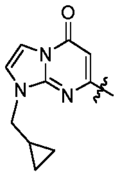, 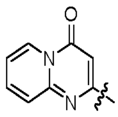, 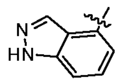,

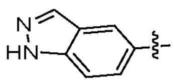, 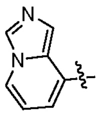, 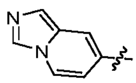, 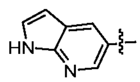, or 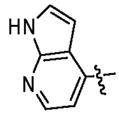;

L is selected from

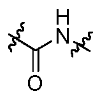, 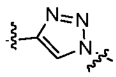, 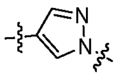, or 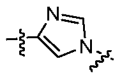,

and is connected with ring A at the left side;

$R^a$ or $R^b$ is each independently selected from H, F, Cl, Br, I, cyano, OH, =O, $CF_3$, methyl, ethyl, propyl, isopropyl, or cyclopropyl;

$R^{2a}$, $R^{2b}$, $R^{3a}$ and $R^{3b}$ are each independently selected from H;

$R^{cn1}$ and $R^{cn2}$ are each independently selected from H, methyl, ethyl, propyl, isopropyl, cyclopropyl, cyclohexyl, cyclopentyl, cyclobutyl or

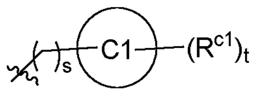,

and the methyl, ethyl, propyl, isopropyl, cyclopropyl, cyclohexyl, cyclopentyl, or cyclobutyl is optionally further substituted with 0 to 4 substituents selected from H, F, Cl, Br, I, OH, =O, cyano, $NH_2$, $CF_3$, methyl, ethyl, propyl, isopropyl, cyclopropyl, methoxy or ethoxy;

ring C1 is selected from cyclopropyl, cyclobutyl, cyclopentyl, cyclohexyl, cyclopentyl fused cyclobutyl or

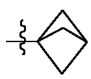;

each $R^{c1}$ is independently selected from H, F, or Cl;

alternatively, $R^{cn1}$ and $R^{cn2}$ together with the atom to which they are attached form substituted or unsubstituted azetidinyl, azacyclopentyl, piperidine, piperazine, azacyclopentyl spirocyclobutyl, azacyclopentyl spirocyclopentyl, azacyclohexyl spirocyclobutyl, azacyclohexyl spirocyclopentyl, or azacyclohexyl spirocyclohexyl, which, when substituted, is optionally further substituted with 0 to 4 substituents selected from H, F, Cl, Br, I, OH, =O, cyano, $NH_2$, $CF_3$, methyl, ethyl, propyl, isopropyl, cyclopropyl, methoxy or ethoxy;

m is selected from 0 or 1;

n is selected from 0 or 1;

s is selected from 0 or 1;

t is selected from 0, 1 or 2.

**14.** The compound or the stereoisomer, tautomer, deuterate, solvate, prodrug, metabolite, pharmaceutically acceptable salt or co-crystal thereof according to claim 1, **characterised in that** the compound is selected from one of the structures shown in Table S-1 or Table S-2.

**15.** A pharmaceutical composition, **characterised by** comprising the compound or the stereoisomer, tautomer, deuterate, solvate, prodrug, metabolite, pharmaceutically acceptable salt or co-crystal thereof according to any one of claims 1-14, and a pharmaceutically acceptable carrier, **characterised in that** preferably, the pharmaceutical composition comprises 1-1500 mg of the compound or the stereoisomer, deuterate, solvate, prodrug, metabolite,

pharmaceutically acceptable salt or co-crystal thereof according to any one of claims 1-14.

**16.** Use of the compound or the stereoisomer, tautomer, deuterate, solvate, prodrug, metabolite, pharmaceutically acceptable salt or co-crystal thereof according to any one of claims 1 to 15 in the preparation of a drug for treating a disease related to the inhibition of METTL3 enzyme.

**17.** The use according to claim 16, **characterised in that** the disease is selected from cancer.

**18.** A method for treating a disease in a mammal, the method comprising administering to a subject a therapeutically effective amount of the compound or the stereoisomer, deuterate, solvate, prodrug, metabolite, pharmaceutically acceptable salt, or co-crystal thereof according to any one of claims 1-14, **characterised in that** the therapeutically effective amount is preferably 1-1500 mg, and the disease is preferably cancer.

| INTERNATIONAL SEARCH REPORT | International application No. PCT/CN2023/076002 |
|---|---|

**A. CLASSIFICATION OF SUBJECT MATTER**

C07D487/04(2006.01)i;C07D495/04(2006.01)i;C07D403/12(2006.01)i;C07D417/12(2006.01)i;A61K31/519(2006.01)i; A61P35/02(2006.01)i;A61P35/00(2006.01)i

According to International Patent Classification (IPC) or to both national classification and IPC

**B. FIELDS SEARCHED**

Minimum documentation searched (classification system followed by classification symbols)

C07D,A61K,A61P

Documentation searched other than minimum documentation to the extent that such documents are included in the fields searched

Electronic data base consulted during the international search (name of data base and, where practicable, search terms used)

CNABS, DWPI, CNKI, 万方数据库, WANFANG DATABASE, STN(REGISTRY, MARPAT, CAPLUS): METTL3, 白血病, 检索结构式, search structural formula

**C. DOCUMENTS CONSIDERED TO BE RELEVANT**

| Category* | Citation of document, with indication, where appropriate, of the relevant passages | Relevant to claim No. |
|---|---|---|
| A | WO 2021111124 A1 (STORM THERAPEUTICS LTD.) 10 June 2021 (2021-06-10) abstract, claims 1-26, embodiment 1 | 1-18 |
| A | CN 113905787 A (STORM THERAPEUTICS LTD.) 07 January 2022 (2022-01-07) abstract, claims 1-56, compound 270 | 1-18 |
| A | WO 2021079196 A2 (ACCENT THERAPEUTICS INC. et al.) 29 April 2021 (2021-04-29) abstract, claims 1-55 | 1-18 |
| A | WO 2021081211 A1 (ACCENT THERAPEUTICS INC.) 29 April 2021 (2021-04-29) abstract, claims 1-58, description pages 39-136 | 1-18 |

☐ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **20 April 2023** | **27 April 2023** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2023/076002**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **18**
because they relate to subject matter not required to be searched by this Authority, namely:

Claim 18 relates to a method for treatment of diseases. The present search report is provided on the basis of the corresponding pharmaceutical use thereof.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2023/076002**

| Patent document cited in search report | | | Publication date (day/month/year) | Patent family member(s) | | | Publication date (day/month/year) |
|---|---|---|---|---|---|---|---|
| WO | 2021111124 | A1 | 10 June 2021 | AU | 2020394867 | A1 | 26 May 2022 |
| | | | | CA | 3162166 | A1 | 10 June 2021 |
| | | | | JP | 2023506147 | A | 15 February 2023 |
| | | | | US | 2023002378 | A1 | 05 January 2023 |
| | | | | IL | 293340 | A | 01 July 2022 |
| | | | | KR | 20220113431 | A | 12 August 2022 |
| | | | | EP | 4069694 | A1 | 12 October 2022 |
| | | | | BR | 112022010561 | A2 | 16 November 2022 |
| CN | 113905787 | A | 07 January 2022 | US | 2023085408 | A1 | 16 March 2023 |
| | | | | WO | 2020201773 | A1 | 08 October 2020 |
| | | | | EP | 3946618 | A1 | 09 February 2022 |
| | | | | JP | 2022528562 | A | 14 June 2022 |
| WO | 2021079196 | A2 | 29 April 2021 | AU | 2020372002 | A1 | 26 May 2022 |
| | | | | JP | 2022553077 | A | 21 December 2022 |
| | | | | EP | 4048674 | A2 | 31 August 2022 |
| | | | | US | 2023027361 | A1 | 26 January 2023 |
| | | | | WO | 2021079196 | A3 | 01 July 2021 |
| | | | | WO | 2021079196 | A9 | 14 July 2022 |
| WO | 2021081211 | A1 | 29 April 2021 | | None | | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2021111124 A